(19) **Europäisches Patentamt / European Patent Office / Office européen des brevets**

(11) **EP 1 390 741 B1**

(12) **EUROPEAN PATENT SPECIFICATION**

(45) Date of publication and mention
of the grant of the patent:
**31.10.2012  Bulletin 2012/44**

(51) Int Cl.:
**G01N 31/00** (2006.01)     **G06F 19/00** (2011.01)

(21) Application number: **02762144.0**

(86) International application number:
**PCT/US2002/012202**

(22) Date of filing: **17.04.2002**

(87) International publication number:
**WO 2002/084277 (24.10.2002 Gazette 2002/43)**

(54) **STRUCTURE-BASED CONSTRUCTION OF HUMAN ANTIBODY LIBRARY**

AUF STRUKTUREN BERUHENDE KONSTRUKTION EINER BIBLIOTHEK MENSCHLICHER ANTIKÖRPER

CONSTRUCTION STRUCTURELLE DE BIBLIOTHEQUES D'ANTICORPS HUMAINS

(84) Designated Contracting States:
**AT BE CH CY DE DK ES FI FR GB GR IE IT LI LU MC NL PT SE TR**

(30) Priority: **17.04.2001  US 284407 P**

(43) Date of publication of application:
**25.02.2004  Bulletin 2004/09**

(73) Proprietor: **Abmaxis, Inc.**
**Whitehouse Station, NJ 08889-0100 (US)**

(72) Inventor: **LUO, Peizhi**
**Sunnyvale, CA 94086 (US)**

(74) Representative: **Harding, Charles Thomas et al**
**D Young & Co LLP**
**120 Holborn**
**London**
**EC1N 2DY (GB)**

(56) References cited:
**WO-A-97/08320**

- **MARTIN, C.R.M. ET AL.: "Modeling antibody hypervariable loops: a combined algorithm" P.N.A.S., vol. 86, no. 23, December 1989 (1989-12), pages 9268-9272, XP000165667**
- **GRIFFITHS A.D. ET AL.: "High affinity antibodies from large repertoires" EMBO J., vol. 13, no. 14, 15 July 1994 (1994-07-15), pages 3245-3260, XP000455240**
- **KNAPPIK ET AL.: 'Fully synthetic human combinatorial antibody libraries (HuCAL) based on modular consensus frameworks and CDRs randomized with trinucleotides' JOURNAL OF MOLECULAR BIOLOGY vol. 296, 11 February 2000, pages 57 - 86, XP002941688**
- **IBA ET AL.: 'Changes in the specificity of antibodies against steroid antigens by introduction of mutations into complementarity-determining regions of the VH domain' PROTEIN ENGINEERING vol. 11, no. 5, May 1998, pages 361 - 370, XP002953909**
- **CHOTHIA ET AL.: 'Structural determinants in the sequences of immunoglobulin variable domain' JOURNAL OF MOLECULAR BIOLOGY vol. 278, 01 May 1998, pages 457 - 479, XP002953910**

**Description**

**BACKGROUND OF THE INVENTION**

Field of the Invention

The present invention relates generally to computer-aided design of human antibody sequence libraries, and, more particularly, relates to methods and systems for selecting and constructing fully human or human-derived antibody library based on three-dimensional structural frameworks of vertebrate antibody repertoire.

Description of Related Art

Antibodies are made by vertebrates in response to various internal and external stimuli (antigens). Synthesized exclusively by B cells, antibodies are produced in millions of forms, each with different amino acid sequence and a different binding site for antigen. Collectively called immunoglobulins (abbreviated as Ig), they are among the most abundant protein components in the blood, constituting about 20% of the total plama protein by weight.

A naturally occurring antibody molecule consists of two identical "light" (L) protein chains and two identically "heavy" (H) protein chains, all held together covalently by precisely located disulfide linkages. Chothia et al. (1985) J. Mol. Biol. 186:651-663; and Novotny and Haber (1985) Proc. Natl. Acad. Sci. USA 82:4592-4596. The N-terminal regions of the L and H chains together form the antigen recognition site of each antibody.

The mammalian immune system has evolved unique genetic mechanisms that enable it to generate an almost unlimited number of different light and heavy chains in a remarkably economical way by joining separate gene segments together before they are transcribed. For each type of Ig chain-$\kappa$ light chains, $\lambda$ light chains, and heavy chain-there is a ssseparate pool of gene segments from which a single peptide chain is eventually synthesized. Each pool is on a different chromosome and usually contains a large number of gene segments encoding the V region of an Ig chain and a smaller number of gene segments encoding the C region. During B cell development a complete coding sequence for each of the two Ig chains to be synthesized is assembled by site-specific genetic recombination, bringing together the entire coding sequences for a V region and the coding sequence for a C region. In addition, the V region of a light chain is encoded by a DNA sequence assembled from two gene segments- a V gene segment and short joining or J gene segment. The V region of a heavy chain is encoded by a DNA sequence assembled from three gene segments- a V gene segment, a J gene segment and a diversity or D segment.

The large number of inherited V, J and D gene segments available for encoding Ig chains makes a substantial contribution on its own to antibody diversity, but the combinatorial joining of these segments greatly increases this contribution. Further, imprecise joining of gene segments and somatic mutations introduced during the V-D-J segment joining at the pre-B cell stage greatly increases the diversity of the V regions.

After immunization against an antigen, a mammal goes through a process known as affinity maturation to produce antibodies with higher affinity toward the antigen. Such antigen-driven somatic hypermutation fine-tunes antibody responses to a given antigen, presumably due to the accumulation of point mutations specifically in both heavy-and light-chain V region coding sequences and a selected expansion of high-affinity antibody-bearing B cell clones.

Structurally, various functions of an antibody are confined to discrete protein domains (regions). The sites that recognize and bind antigen consist of three complementarity-determining regions (CDRs) that lie within the variable ($V_H$ and $V_L$) regions at the N-terminal ends of the two H and two L chains. The constant domains are not involved directly in binding the antibody to an antigen, but are involved in various effector functions, such as participation of the antibody in antibody-dependent cellular cytotoxicity.

The domains of natural light and heavy chains have the same general structures, and each domain comprises four framework regions, whose sequences are somewhat conserved, connected by three hyper-variable or CDRs. The four framework regions largely adopt a $\beta$-sheet conformation and the CDRs form loops connecting, and in some cases forming part of, the $\beta$-sheet structure. The CDRs in each chain are held in close proximity by the framework regions and, with the CDRs from the other chain, contribute to the formation of the antigen binding site.

Generally all antibodies adopt a characteristic "immunoglobulin fold". Specifically, both the variable and constant domains of an antigen binding fragment (Fab, consisting of $V_L$ and $C_L$ of the light chain and $V_H$ and $C_H1$ of the heavy chain) consist of two twisted antiparallel $\beta$-sheets which form a $\beta$-sandwich structure. The constant regions have three- and four-stranded $\beta$-sheets arranged in a Greek key-like motif, while variable regions have a further two short $\beta$ strands producing a five-stranded $\beta$-sheet.

The $V_L$ and $V_H$ domains interact via the five-stranded $\beta$ sheets to form a nine-stranded $\beta$ barrel of about 8.4 Å radius, with the strands at the domain interface inclined at approximately 50° to one another. The domain pairing brings the CDR loops into close proximity. The CDRs themselves form some 25% of the $V_L/V_H$ domain interface.

The six CDRs, (CDR-L1, -L2 and -L3 for the light chain, and CDR-H1, -H2 and -H3 for the heavy chain), are supported on the β barrel framework, forming the antigen binding site. While their sequence is hypervariable in comparison with the rest of the immunoglobulin structure, some of the loops show a relatively high degree of both sequence and structural conservation. In particular, CDR-L2 and CDR-H1 are highly conserved in conformation.

Chothia and co-workers have shown that five of the six CDR loops (all except CDR-H3) adopt a discrete, limited number of main-chain conformations (termed canonical structures of the CDRs) by analysis of conserved key residues. Chothia and Lesk (1987) J. Mol. Biol. 196:901-917; Chothia et al. (1989) Nature (London) 342:877; and Chothia et al. (1998) J. Mol. Biol. 278:457-479. The adopted structure depends on both the CDR length and the identity of certain key amino acid residues, both in the CDR and in the contacting framework, involved in its packing. The canonical conformations were determined by specific packing, hydrogen bonding interactions, and stereochemical constraints of only these key residues which serve as structural determinants.

Various methods have been developed for modeling the three dimensional structures of the antigen binding site of an antibody. Other than x-ray crystallography, nuclear magnetic resonance (NMR) spectroscopy has been used in combination with computer modeling building to study the atomic details of antibody-ligand interactions. Dwek et al. (1975) Eur. J. Biochem. 53:25-39. Dwek and coworkers used spin-labeled hapten to deduce the combining site of the MoPC 315 myeloma protein for dinitrophenyl. Similar analysis has also been done anti-spin label monoclonal antibodies (Anglister et al. (1987) Biochem. 26: 6958-6064) and on the anti-2-phenyloxazolone Fv fragments (McManus and Riechmann (1991) Biochem. 30:5851-5857).

Computer-implemented analysis and modeling of antibody combining site (or antigen binding site) is based on homology analysis comparing the target antibody sequence with those of antibodies with known structures or structural motifs in existing data bases (e.g. the Brookhaven Protein Data Bank). By using such homology modeling methods approximate three-dimensional structure of the target antibody is constructed. Early antibody modeling was based on the conjecture that CDR loops with identical length and different sequence may adopt similar conformations. Kabat and Wu (1972) Proc. Natl. Acad. Sci. USA 69: 960-964. A typical segment match algorithm is as follows: given a loop sequence, the Protein Data Bank can be searched for short, homologous backbone fragments (e.g. tripeptides) which are then assembled and computationally refined into a new combining site model.

More recently, the canonical loop concept has been incorporated into computer-implemented structural modeling of antibody combining site. In its most general form, the canonical structure concept assumes that (1) sequence variation at other than canonical positions is irrelevant for loop conformation, (2) canonical loop conformations are essentially independent of loop-loop interactions, and (3) only a limited number of canonical motifs exist and these are well represented in the database of currently known antibody crystal structures. Based on this concept, Chothia predicted all six CDR loop conformations in the lysozyme-binding antibody D 1.3 and five canonical loop conformations in four other antibodies. Chothia (1989), *supra.* It is also possible to improve modeling of CDRs of antibody structures by combining the homology modeling with conformational search procedures. Martin, A.C.R. (1989) PNAS 86, 9268-72.

Besides modeling a specific antibody structure, efforts have been made in generating artificial (or synthetic) libraries of antibodies which are screened against one or more specific, target antigens. Various artificial sequences are generated by site-specific or random mutagenesis on the antibody sequence, especially into the CDR regions of the variable domains. For example, Iba et al. used computer-driven model building system to change the specificity of antibodies against steroid antigens by introducing mutations into the CDR regions. Iba et al. (1998) Protein Eng. 11: 361-370. A phage-display library of Abs in which 16 residues of 17-α- hydroxyprogesterone (17-OHP) were mutated in three CDR regions of the heavy chain that appeared to form the steroid-binding pocket. The phage display library were screened against 17-OHP and cortisol that had been conjugated with bovine serum albumin. Many clones were isolated that had retained 17-OHP-binding ability as well as clones with the newly developed ability to bind cortisol in addition to 17-OHP.

Phage display technology has been used extensively to generate large libraries of antibody fragments by exploiting the capability of bacteriophage to express and display biologically functional protein molecule on its surface. Combinatorial libraries of antibodies have been generated in bacteriophage lambda expression systems which may be screened as bacteriophage plaques or as colonies of lysogens (Huse et al. (1989) Science 246: 1275; Caton and Koprowski (1990) Proc. Natl. Acad. Sci. (U.S.A.) 87: 6450; Mullinax et al (1990) Proc. Natl. Acad. Sci. (U.S.A.) 87: 8095; Persson et al. (1991) Proc. Natl. Acad. Sci. (U.S.A. 88: 2432). Various embodiments of bacteriophage antibody display libraries and lambda phage expression libraries have been described (Kang et al. (1991) Proc. Natl. Acad. Sci. (U.S.A.) 88: 4363; Clackson et al. (1991) Nature 352: 624; McCafferty et al. (1990) Nature 348: 552; Burton et al. (1991) Proc. Natl. Acad. Sci. (U.S.A.) 88: 10134; Hoogenboom et al. (1991) Nucleic Acids Res. 19: 4133; Chang et al. (1991) J. Immunol. 147: 3610; Breitling et al. (1991) Gene 104: 147; Marks et al. (1991) J. Mol. Biol. 222: 581; Barbas et al. (1992) Proc. Natl. Acad. Sci. (U.S.A.) 89: 4457; Hawkins and Winter (1992) J. Immunol. 22: 867; Marks et al. (1992) Biotechnology 10: 779; Marks et al. (1992) J. Biol. Chem. 267: 16007; Lowman et al (1991) Biochemistry 30: 10832; Lerner et al. (1992) Science 258: 1313). Also see review by Rader, C. and Barbas, C. F. (1997) "Phage display of combinatorial antibody libraries" Curr. Opin. Biotechnol. 8:503-508.

Generally, a phage library is created by inserting a library of a random oligonucleotide or a cDNA library encoding antibody fragment such as $V_L$ and $V_H$ into gene 3 of M 13 or fd phage. Each inserted gene is expressed at the N-terminal of the gene 3 product, a minor coat protein of the phage. As a result, peptide libraries that contain diverse peptides can be constructed. The phage library is then affinity screened against immobilized target molecule of interest, such as an antigen, and specifically bound phages are recovered and amplified by infection into *Escherichia coli* host cells. Typically, the target molecule of interest such as a receptor (e.g., polypeptide, carbohydrate, glycoprotein, nucleic acid) is immobilized by covalent linkage to a chromatography resin to enrich for reactive phage by affinity chromatography) and/or labeled for screen plaques or colony lifts. This procedure is called biopanning. Finally, amplified phages can be sequenced for deduction of the specific peptide sequences.

The sequences of the antibodies in these phage display libraries are from natural sources. For example, cDNA of antibody gene pools have been generated from immunized or naive human or rodents. Barbas and Burton (1996) Trends Biotech. 14:230-234 (immunized donors); De Haard et al. (1999) J. Biol. Chem. 274:18218-18230 (naive B-cell Ig repertoires). The antibody cDNA library can be constructed by reverse transcription of RNA encoding the gene pool from total RNA samples isolated from B cells contained in peripheral blood supplied by human or animal. First strand cDNA synthesis is usually performed using the method of Marks et al. in which a set of heavy and light chain cDNA primers are designed to anneal to the constant regions for priming the systhesis of cDNA of heavy chain and light chains (both κ and λ) antibody genes in separate tubes. Marks et al. (1991) Eur. J. Immunol. 21:985-991.

Synthetic or artificial libraries of antibody sequences were constructed *in vitro* from human germline sequences. Griffiths et al. (1994) EMBO J. 13:3245-3260. Highly diverse repertoires of heavy and light chains were created entirely in vitro from a bank of human V gene segments and then, by recombination of the repertoire in bacteria, an even larger (close to $6.5 \times 10^{10}$) synthetic library of Fab fragments were generated in bacteria and displayed on filamentous phage.

Highly diverse synthetic libraries of antibody sequences were also constructed based on consensus sequences of each germline family of human antibody repertoire. For example, a fully synthetic combinatorial antibody library was constructed based on modular consensus frameworks and CDR3 regions in heavy and light chains randomized with trinucleotides. Knappik et al. (2000) J. Mol. Biol. 296:57-86. Knappik et al. analysed the human antibody repertoire in terms of structure, amino acid sequence diversity and germline usage. Modular consensus framework sequences with seven $V_H$ and seven $V_L$ were derived to cover 95% of variable germline families and optimized for expression in E. coli. A consensus sequence was derived for each highly used germline family and optimized for expression in *E. coli*. Molecular modeling of their CDR loops of the consensus sequences verified that all canonical classes were present. Diversity of the antibody library was created by replacing the CDR3 regions of seven $V_H$ and seven $V_L$ frameworks of the master genes by CDR3 library cassettte. A synthetic library of combinatorial antibody was generated from mixed trinucleotides and biased towards natural human antibody CDR3 sequences. This library was cloned into phagemid and expressed as soluble proteins in the periplasm of E. coli.

Griffiths et al EMBO J. Vol. 13, No. 14, 15 July 1994 pp3245-3260 teaches the isolation of high affinity human antibodies directly from large synthetic repertoires. WO97/08320 relates to synthetic DNA sequences which encode one or more collections of homologous proteins, and methods for generating and applying libraries of these DNA sequences.

Marti-Renom et al Annu. Rev. BioPhys. Biomol. Struct. 2000, Vol 29, p291-325 considers comparative modelling to predict the three-dimensional structure of a given protein sequence (target) based primarily on its alignment to one or more proteins of known structure (templates).

MacCullum et al "Computational Analysis of Protein Sequence and Structure - Thesis 1997 p 1-162 - particularly Ch 2 - p36-53 and Appendix A, p133-136 thereof - teach antibody-antigen interactions: contact analysis and binding site topography.

Mirny et al (J. Mol. Biol. 199, Vol 283 p507-526) looks at protein structure prediction by threading and considers why it works and why it does not work.

## SUMMARY OF THE INVENTION

The present invention provides a comprehensive methodology to map the functional space of proteins by exploiting the fundamental structure-sequence relationship within protein families. The methodology of the present invention provides for efficient *in silico* selection and construction of a library of antibodies with diverse sequences. By using the methodology libraries of antibodies can be constructed with diverse sequences in the CDR regions, and humanized frameworks of the variable regions having fully human, human-derived antibody, or antibody of human origin (collectively referred to herein as "human antibody") based on three-dimensional structures of antibodies generated by all species of vertebrates including human.

In one aspect of the invention, a method is provided for constructing a library of artificial antibodies *in silico* based on ensembles of 3D structures of existing antibodies of human origin, optional also including those of other vertebrate origins. By using the method, a master library of human antibody sequences can be selected to better represent all antibody structural repertoire in the vertebrate antibody repertoire that are functionally important for high affinity binding to antigens and eliciting antibody-dependent cellular responses.

Such a functionally representative library provides a structurally diverse and yet functionally more relevant source of antibody candidates which can then be screened for binding to a wide variety of target molecules, including but not limited to biomacromolecules such as protein, peptide, and nucleic acids, and small molecules.

In one embodiment, the method comprises the steps of:

clustering variable regions of a collection of antibodies having known 3D structures into at least two families of structural ensembles, each family of structural ensemble comprising at least two different antibody sequences but with substantially identical main chain conformations;

selecting a representative structural template from each family of structural ensemble;

profiling a tester polypeptide sequence onto the representative structural template within each family of structural ensemble; and

selecting the tester antibody sequence that is compatible to the structural constraints of the representative structural template.

According to the method, examples of the collection of antibodies include, but are not limited to, antibodies or immunoglobulins collected in a protein database such as the protein data bank of Brookhaven National Laboratory, genbank at the National Institute of Health, and Swiss-PROT protein sequence database.

The collection of antibodies having known 3D structures include any antibody having resolved X-ray crystal structure, NMR structure or a 3D structure based on structural modeling such as homology modeling.

The variable regions of a collection of antibodies may be the full length of the heavy chain or light chain variable region or a specific portion of the heavy chain or light chain variable region, such as a CDR (e.g., $V_H$ or $V_L$ CDR1, CDR2, and CDR3), a framework region (FR, e.g., $V_H$ or $V_L$ FR1, FR2, FR3, and FR4), and a combination thereof.

Also according to the method, the clustering step includes clustering the collection of antibodies such that the root mean square difference of the main chain conformations of antibody sequences in each family of the structural ensemble is less than 2 Å.

Optionally, the clustering step includes clustering the collection of antibodies such that the Z-score of the main chain conformations of antibody sequences in each family of the structural ensemble is more than 4.

The clustering step may be implemented by an algorithm selected from the group consisting of CE, Monte Carlo and 3D clustering algorithms.

Also according to the method, the profiling step includes reverse threading the tester polypeptide sequence onto the representative structural template within each family of structural ensemble.

Optionally, the profiling step is implemented by a multiple sequence alignment algorithm such as the profile HMM algorithm and PSI-BLAST (Position-Specific Iterated BLAST).

When the representative structural template is adopted by a CDR region, the profiling step includes profiling the tester polypeptide sequence that is a human or non-human antibody onto the representative structural template within each family of structural ensemble.

When the representative structural template is adopted by a FR region, the profiling step includes profiling the tester polypeptide sequence that is a human or non-human antibody, preferably a human germline antibody sequence, onto the representative structural template within each family of structural ensemble.

In another aspect of the invention, a method is provided for in silico selection of antibody sequences based on structural alignment with a target structural template. Diverse sequences which still retain the same functionally relevant structure as the target structural template can be selected by using reverse threading, the profile HMM algorithm and PSI-BLAST. By using the method, a library of diverse antibody sequences can be constructed and screened experimentally in vitro or in vivo for antibody mutants with improved or desired function(s).

In one embodiment, the method comprises the steps of:

providing a target structural template of a variable region of one or more antibodies;

profiling a tester polypeptide sequence onto the target structural template; and

selecting the tester polypeptide sequence that is structurally compatible with the target structural template.

According to the method, the target structural template may be a 3D structure of a heavy chain or light chain variable region of an antibody (e.g., CDR, FR and a combination thereof), or a structural ensemble of heavy chain or light chain variable regions of at least two different antibodies.

Also according to the method, the profiling step includes reverse threading the tester polypeptide sequence onto the target structural template.

Optionally, the profiling step is implemented by a multiple sequence alignment algorithm such as the profile HMM algorithm and PSI-BLAST.

Also optionally, when the target structural template is adopted by a CDR region of the target antibody, the profiling step includes profiling a heavy chain or light chain variable region of the tester polypeptide sequence that is either a human antibody or a non-human antibody.

Also optionally, when the target structural template is adopted by a FR region of the target antibody, the profiling step includes profiling a heavy chain or light chain variable region of the tester polypeptide sequence that is a human antibody, preferably a human germline antibody, onto the target structural template.

According to any of the above method, the tester polypeptide sequence may be the sequence or a segment sequence of an expressed protein, preferably an antibody, more preferably a human antibody, and most preferably a human germline antibody sequence.

According to any of the above method, the selecting step includes selecting the tester polypeptide sequence by using an energy scoring function selected from the group consisting of electrostatic interactions, van der Waals interactions, electrostatic solvation energy, solvent-accessible surface solvation energy, and conformational entropy.

Optionally, the selecting step includes selecting the tester polypeptide sequence by using a scoring function incorporating a forcefield selected from the group consisting of the Amber forcefield, Charmm forcefield, the Discover cvff forcefields, the ECEPP forcefields, the GROMOS forcefields, the OPLS forcefields, the MMFF94 forcefield, the Tripose forcefield, the MM3 forcefield, the Dreiding forcefield, and UNRES forcefield, and other knowledge-based statistical forcefield (mean field) and structure-based thermodynamic potential functions.

In yet another aspect of the invention, a method is provided for in silico selection of antibody sequences based on homology alignment with a target sequence template. Remote homologues with diverse sequences but retaining the same functionally relevant structure can be selected by using profile hidden Markov Model (HMM) and PSI-BLAST. By using the method, a library of diverse antibody sequences can be constructed with a relatively smaller size than that constructed by complete randomization of the target sequence. This library can then be filtered using certain cutoff value based on, for example, the occurrence frequency of variants in each amino acid residue position, and screened experimentally in vitro or in vivo for antibody mutants with improved or desired function(s).

In one embodiment, the method comprises the steps of:

> providing a target sequence of a heavy chain or light chain variable region of an antibody;
> aligning the target sequence with a tester polypeptide sequence; and
> selecting the tester polypeptide sequence that has at least 15% sequence homology with the target sequence.

According to the method, the target sequence may be the full length of the heavy chain or light chain variable region, or a specific portion of the variable region, such as a CDR, a framework (FR) region and a combination thereof.

Also according to the method, the aligning step includes aligning the target sequence with the polypeptide segment of the tester protein by using a sequence alignment algorithm selected from the group consisting of BLAST, PSI-BLAST, profile HMM, and COBLATH.

Also according to the method, when the target sequence is a CDR region of the target antibody, the alignment step includes aligning any protein sequences that is of either human or non-human origin with the target sequence.

Also according to the method, when the target sequence is a CDR region of the target antibody, the tester polypeptide sequence is a heavy chain or light chain variable region of a human or non-human antibody.

Also according to the method, when the target sequence is a FR region of the target antibody, the tester polypeptide sequence is a heavy chain or light chain variable region of a human antibody, preferably a human germline antibody sequence.

Also according to the method, the selecting step includes selecting the polypeptide segment of the tester protein that has preferably at least 25%, preferably at least 35%, and most preferably at least 45% sequence homology with the target sequence.

According to any of the above methods, the method further comprises:

> introducing the DNA segment encoding the selected tester polypeptide into cells of a host organism;
> expressing the DNA segment in the host cells such that a recombinant antibody containing the selected polypeptide or antibody sequence is produced in the cells of the host organism; and
> selecting the recombinant antibody that binds to a target antigen with affinity higher than $10^6$ $M^{-1}$.

The recombinant antibody may be a fully assembled antibody, a Fab fragment, an Fv fragment, or a single chain antibody.

The host organism includes any organism or its cell line that is capable of expressing transferred foreign genetic sequence, including but not limited to bacteria, yeast, plant, insect, and mammals.

The target antigen to be screened against includes small molecules and macromolecules such as proteins, peptides, nucleic acids and polycarbohydates.

**BRIEF DESCRIPTION OF FIGURES**

**Figure 1** illustrates a flow chart of a computer-implemented process that can be used in the present invention to construct an antibody library in silico.

**Figure 2** shows 7 $V_H$ and 7 $V_L$ consensus sequences for 7 $V_H$ and 7 $V_L$ framework of Hucal library in fasta format by Knappik et al., supra.

**Figure 3** shows the structures of the seven $V_H$ sequences superimposed on each other. The structures are aligned by superimposing the Ca atoms using the CE with RMSD < 2Å and Z-score > 4.

**Figure 4** shows **(A)** the Ca trace of the superimposed structures of these 3 $V_H$ sequences (1DHA in green, 1DHO in cyan, and 1DHW in yellow); **(B)** the superimposed structures with a ribbon representation of the β-sheets of the VH frameworks. As shown in both **Figures 4A** and **4B,** the 3 $V_H$ sequences (1DHA, 1DHO, and 1DHW) collapse into one structural family with RMSD <0.7 Å and Z-score >6 using 1DHA as standard, even though their sequence identity ranges widely from 72% to 87% relative to 1DHA.

**Figure 5** shows the structures of the seven $V_L$ sequences retrieved from the PDB and superimposed on each other. The structures are aligned by using 1DGX as the reference structure with RMSD< 1.6Å and Z-score >6. The seven $V_L$ sequences have a wide range of conformational variability, especially in the CDR regions highlighted (The structural flexibility at N- and C-termini are discarded here).

**Figure 6** shows the superpositioned 1DGX(green), 1DH4 (yellow), 1DH5 (color cyan) and 1DH6 (magenta) with similar conformation but varying length in the CDR regions. By using the CE algorithm, four $V_L$ sequences (1DGX, 1DH4, 1DH5 and 1DH6) of the 7 consensus sequences families can be clustered into one structural family with RMSD < 0.6 Å and Z-score >6 and with sequence identity ranging from 67% to 80% using 1DGX as the structure reference. These four sequences also belong to the $V_L$ kappa sequence family.

**Figure 7** shows three superimposed structures of 1DH7, 1DH8, and 1DH9 in lamda variable light chain, can be clustered into 1 structure family with RMSD < 1.5Å and Z-score > 6 using 1DGX as the reference.

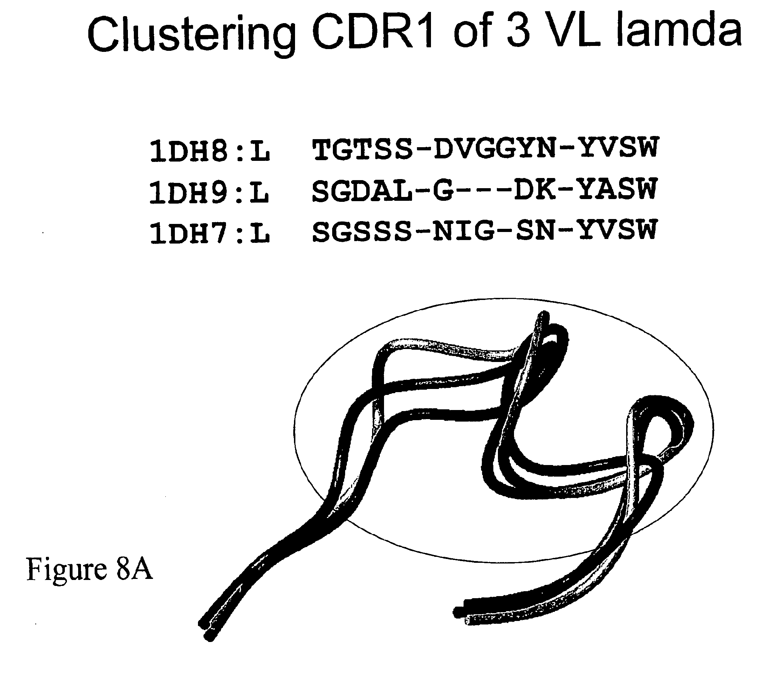

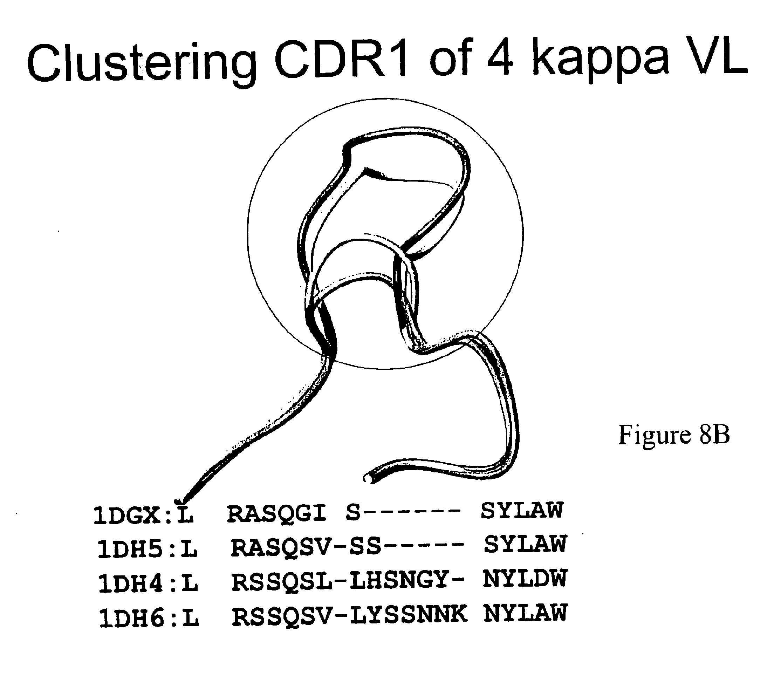

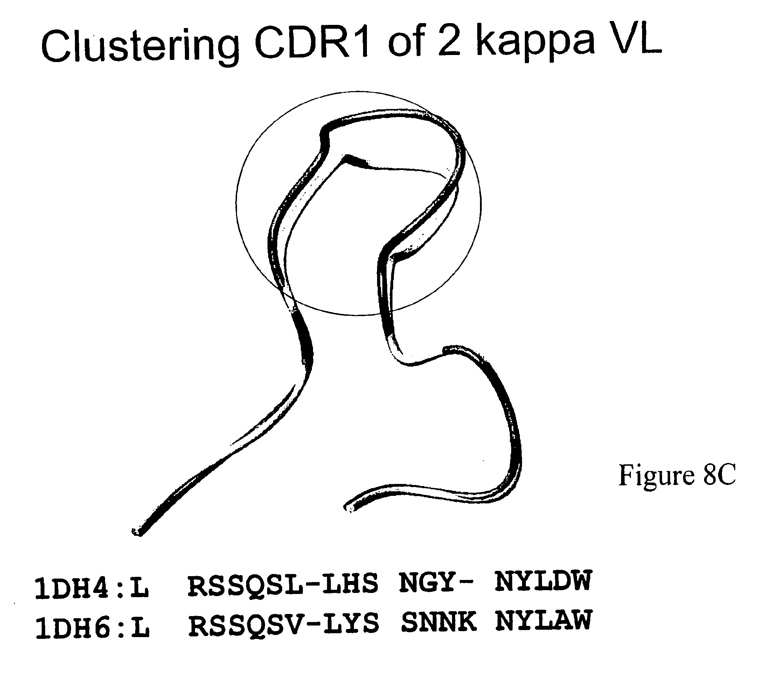

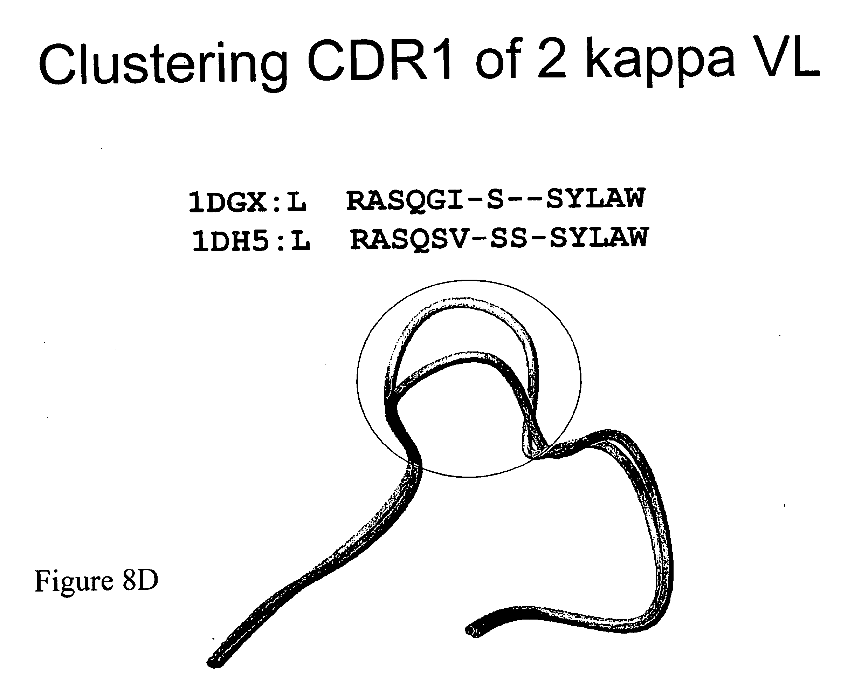

**Figure 8** shows in **(A)** that CDR1 regions of the three lamda (λ) $V_L$ sequences (1DH7, 1DH8-and 1DH9) adopt similar conformations with RMSD < 1Å. **(B)** that CDR1 regions of the 4 kappa (κ) VL sequences (1DH4, 1DH6; 1DGX and 1DH5) adopt similar conformations with RMSD < 0.6Å and gaps of 1-6 amino acids. **(C)** that CDR1 regions of the two kappa (κ) $V_L$ sequences (1DH4 and 1DH6) adopt similar conformations with RMSD < 0.6Å and 1 amino acid gap in CDR1. Thus, structures of these two kappa $V_L$ sequences are further clustered into one structural family according to the present invention. **(D)** that CDR1 regions of the two kappa (κ) $V_L$ sequences (1DGX and 1DH5) adopt similar conformations with RMSD < 0.6Å and 1 amino acid gap in CDR1.

**Figure 9** shows that clustering of the structures adopted by the seven consensus germline $V_L$ sequences based on the structural families in the CDR1 region led to two to three distinct families of antibody structures: (1DH7, 1DH8 and 1DH9) for lamda variable light chains, (1DH4 and 1DH6), and/or (1DGX and 1DH5) for kappa variable light chains. The members within each family adopt similar conformations in their CDR1 regions with varying length in amino acids.

**Figure 10** shows the PDB IDs of the consensus sequences of $V_H$ and $V_L$, residues aligned, high score, P(N) sum, smallest probability, % identity with the query sequence, the germline family to which the identified germline sequence belongs.

**Figure 11** shows the homology alignment for each of the selected **human** antibody germline sequences with the query sequence.

**Figure 12A** shows the flow chart for selecting the optimal remote homologous sequence(s) of structure-based multiple sequence alignment by using the profile Hidden Markov Model (HMM).

**Figure 12B** shows results generated by using the method diagramed in **Figure 12A** targeting $V_H$ framework regions.

**Figure 12C** shows results generated by using the method diagramed in **Figure 12A** targeting kappa $V_L$ CDR1.

**Figure 13** shows the top sequences from germline gene segments selected using the profile HMM method for various re-clustered structures.

**DEFINITION**

<u>Structural family</u>: a group of structures that are clustered into a family based on some empirically chosen cutoff values of the root mean square deviation (RMSD) (for example, their Ca atoms of the aligned residues) and statistical significance (Z-score). These values are empirically decided after an overall comparison among structures of interest. For example, for CE algorithms, the starting criteria used are RMSD < 2 Å and Z-score > 4.

<u>Structural ensemble</u>: It is well known that in the structural determination by NMR (nuclear magnetic resonance), the ensemble of structures rather than a single structure, with perhaps several members, all of which fit the NMR data and retain good stereochemistry, is deposited with the Protein Data Bank (PDB). Comparison between the models in this ensemble provides some information on how well the protein conformation was determined by the NMR constraints. In structural clustering, it is important to analyze the all members within a structural cluster to understand some consensus information about the distribution of all structural templates within a family and constraints on their sequences or sequence profiles within a structural family. It should be pointed out that all the sequences corresponding to NMR-determined ensemble structures have the same sequences (one protein with variable conformations). The structural ensemble here in the present invention refers to different proteins with variations in sequence and/or length but have similar main chain conformations.

<u>Ensemble average or representative structure</u>: if all members within a structural cluster has the same length of amino acids, the positions of atoms in main chain atoms of all structures are averaged, and the average model is then adjusted to obey normal bond distances and angles ("restrained minimization"), similar to NMR-determined average structure. If all members within a structural cluster vary in the length of amino acids, a member which is representative of the average characteristics of all other members within the cluster will be chosen as the representative structure.

<u>Canonical structures</u>: the commonly occurring main-chain conformations of the hypervariable regions.

<u>Structural repertoire</u>: the collection of all structures populated by a class of proteins such as the modular structures and canonical structures observed for antibody framework and CDR regions.

<u>Sequence repertoire</u>: collection of sequences for a protein family.

<u>Functional repertoire</u>: a collection of all functions performed by proteins, such as the antibodies' diverse functional epitopes that are capable of binding to various antigens.

<u>Germline gene segments</u>: refers to the genes from the germline (the haploid gametes and those diploid cells from which they are formed). The germline DNA contain multiple gene segments that encode a single immunoglubin heavy or light chain. These gene segments are carried in the germ cells but cannot be transcribed and translated into heavy and light chains until they are arranged into functional genes. During B-cell differentiation in the bone marrow, these gene segments are randomly shuffled by a dynamic genetic system capable of generating more than $10^8$ specificities. Most of these gene segments are published and collected by the germline database.

<u>Rearranged immunoglobulin sequences</u>: the functional immunoglobulin gene sequences in heavy and light chains that are generated by transcribing and translating the germline gene segments during B-cell differentiation and maturation process. Most of the rearranged immunoglobulin sequences used here are from Kabat-Wu database.

<u>BLAST</u>: Basic Local Alignment Search Tool for pairwise sequence analysis. BLAST uses a heuristic algorithm with position-independent scoring parameters to detect similarity between two sequences.

PSI-BLAST: The Position-Specific Iterated BLAST, or PSI-BLAST program performs an iterative search in which sequences found in one round of searching are used to build a score model for the next round of searching. In PSI-BLAST the algorithm is not tied to a specific score matrix. Traditionally, it has been implemented using an AxA substitution matrix where A is the alphabet size. PSI-BLAST instead uses a QxA matrix, where Q is the length of the query sequence; at each position the cost of a letter depends on the position with respect to the query and the letter in the subject sequence. Two PSI-BLAST parameters have been adjusted: the pseudocount constant default has been changed from 10 to 7, and the E-value threshold for including matches in the PSI-BLAST model has been changed from 0.001 to 0.002.

COBLATH: A method that combines PSI-BLAST with Threading method for fold recognition and query-template alignment. It might be used to compare the compatibility between sequences and structural templates.

<u>Profile Hidden Markov Model (profile HMMs)</u>: statistical models of the primary structure consensus of a sequence family. They use position-specific scores for amino acids and for opening and extending an insertion and deletion to detect remote sequence homologues based on the statistical description of the consensus of a multiple sequence alignment. The multiple sequence alignments are given either by the multiple sequence alignment program such as ClustalW or structure-based multiple sequence alignment given by structural clustering.

<u>Threading</u>: a process of assigning the folding of the protein by threading its sequence to a library of potential structural templates by using a scoring function that incorporates the sequence as well as the local parameters such as secondary structure and solvent exposure. The threading process starts from prediction of the secondary structure of the amino acid sequence and solvent accessibility for each residue of the query sequence. The resulting one-dimensional (1D) profile of the predicted structure is threaded into each member of a library of known 3D structures. The optimal threading for each sequence-structure pair is obtained using dynamic programming. The overall best sequence-structure pair constitutes the predicted 3D structure for the query sequence.

<u>Reverse Threading</u>: a process of searching for the optimal sequence(s) from sequence database by threading them onto a given target structure and/or structure cluster. Various scoring functions may be used to select for the optimal sequence(s) from the library comprising protein sequences with various lengths.

<u>Reverse Engineering</u>: the procedure to select and construct sequence or sequence libraries that are compatible to the structural constraints is called reverse engineering including but not limited to reverse threading.

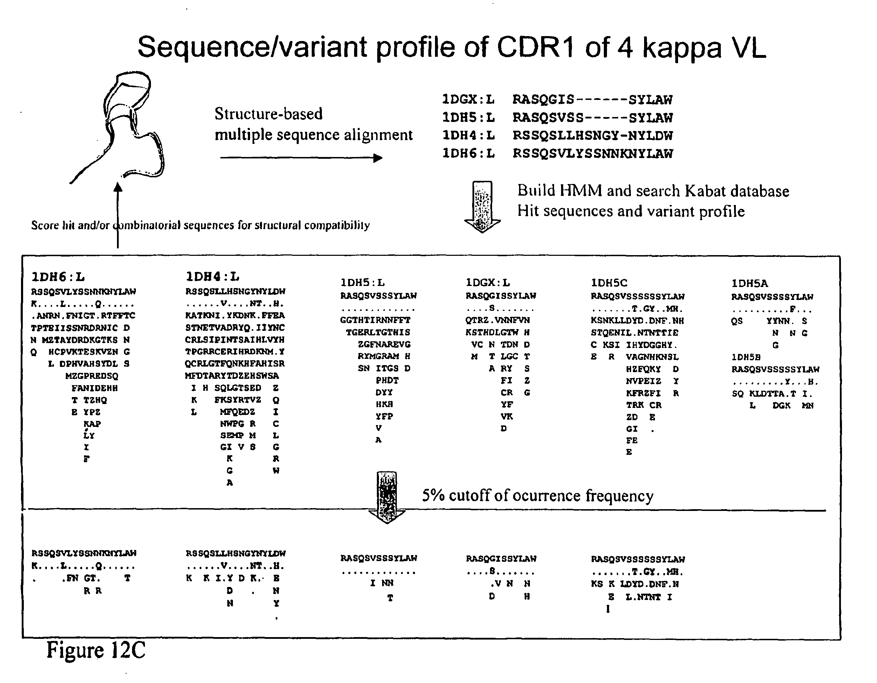

<u>Supervariable Region of Antibody</u>: regions within antibody CDRs that show diverse structure, sequence and chain length variability compared to the other regions of CDRs or CDR ensembles which are relatively constant in structure, sequence and chain length. As exemplified in **Figure 12C,** the super-variability of a region of a specific CDR family can be exploited in CDR library construction.

## DETAILED DESCRIPTION OF THE INVENTION

The present invention provides a system and method for efficient in silico selection and construction of fully human and human-derived antibody libraries. The process is carried out computationally (i.e., in *silico)* in a high throughput manner by mining the ever-expanding databases of protein sequences of all organisms, especially human. The inventive methodology is developed by combining database mining of evolutionary sequences from nature with computational design of structurally relevant variants of the nature sequences.

In one aspect of the invention, the methodology is implemented by a computer system which computationally selects those human antibody sequences based on three-dimensional structural ensemble and/ or ensemble average represented by a limited, discrete number of classes (or clusters) of antibody structures. By using the method, a master library of human antibody sequences can be constructed to better represent all antibody structures in the vertebrate antibody repertoire that are functionally important for high affinity binding to a large variety of antigens and eliciting antibody-dependent cellular responses.

In another aspect of the invention, the methodology is implemented by a computer system which computationally selects from the protein databases protein sequences, particularly antibody sequences, based on structural alignment with a target structural template. Diverse sequences which still retain the same functionally relevant structure as the target structural template can be selected by using reverse threading. By using the method, a library of diverse antibody sequences can be constructed and screened experimentally in vitro or in vivo for antibody mutants with improved or desired function(s).

In yet another aspect of the invention, the methodology is implemented by a computer system which computationally selects from the protein databases protein sequences, particularly antibody sequences, based on homology alignment with a target sequence template. Remote homologues with diverse sequences but retaining the same functionally relevant structure can be selected by using structure-based sequence alignment methods such as profile hidden Markov Model (HMM). By using the method, a library of diverse antibody sequences can be constructed with a relatively smaller size than that constructed by complete randomization of the target sequence. This library can then be thoroughly screened experimentally in vitro or in vivo for antibody mutants with improved or desired function(s).

The inventive methodology can be used to design any protein with novel function or improved function over the target protein which serves as a lead in the process. In particular, mutant antibodies can be designed to include diverse sequences in the CDR regions, and to replace non-human sequences in the frameworks of the variable regions with human ones to reduce immunogenicity of the designed antibody when used as human therapeutics.

The library constructed by using the inventive methodology provides a structurally diverse and yet functionally more relevant source of antibody candidates for further screening for novel antibody with high affinity against a wide range of antigens and having no or minimum immunogenicity to human subject treated with antibody therapeutics.

## 1. Principles of *in silico* selection and construction of a master library of functionally representative human antibody

Antibody is a unique class of proteins which play profound roles in a vertebrate's ability to defend itself against infection by neutralizing (or inactivating) viruses and bacterial toxins, and by recruiting the complement system and various types of white blood cells to kill extracellular microorganisms and larger parasites.

Like every protein of some biological significance, the biological functions of the proteins depend directly on the three-dimensional (3D) structure of the protein. The 3D structure or conformation determines the activity of an enzyme, how a receptor interacts with its ligand, and the affinity of the binding between the receptor and ligand. Thus, it would be biologically more relevant to screen a library of protein such as antibody based on the 3D structure a particular protein sequence adopts rather than the primary DNA or amino acid sequence of the protein.

In particular, as two of the most important handlers to map the functional space of proteins, the sequence and structure information of antibodies have been accumulated for more than a few decades. Extensive analysis on their patterns have provided some of the most detailed understanding of fundamental process for molecular recognitions, which has a direct impact on the combinatorial technology in chemistry and biology.

So far, major efforts in mapping the functional diversity of antibodies have been focused on capturing the complexity in antibody sequence space by either simply increasing the size of antibody sequence pool (the so-called one-pot approach) or by generating large synthetic libraries in CDR regions. Only recently has systematic analysis of antibody sequence repertoire been utilized to design highly diverse consensus sequence library based on highly used human germline sequences as observed in the rearranged human antibody sequences. Knappik et al., *supra.* These consensus sequences were further analyzed to account for the canonical structures for the CDR regions.

In the present invention a distinctly novel approach is utilized to map the functional repertoire of antibody molecules. This approach is taken by exploiting the characteristics of antibody in sequence diversity and global structural conservation.

It is recognized that although a protein may have astronomical number of possible conformations (about 1016 for a small protein of 100 residues (Dill (1985) Biochem. 24:1501-1509), all antibodies adopt a characteristic "immunoglobulin fold" globally. The natural antibody repertoire shows an amazing ability in recognizing a wide variety of molecules. To confer such diverse functions of binding ability to a vertebrate's antibodies, an extremely diverse sequence repertoire (about $10^{12}$ possible combinations between the sequences of mouse heavy chain and light chains) is created by random genomic splicing of heavy and light chains with high variability in both sequence and length in their CDRs.

The structural repertoire to accommodate the much larger sequence repertoire is, however, surprisingly small. Only a limited number of canonical backbone conformations are found to account for structures adopted by the CDRs that are docked onto highly conserved immunoglobin scaffold.

1) <u>General approach</u>

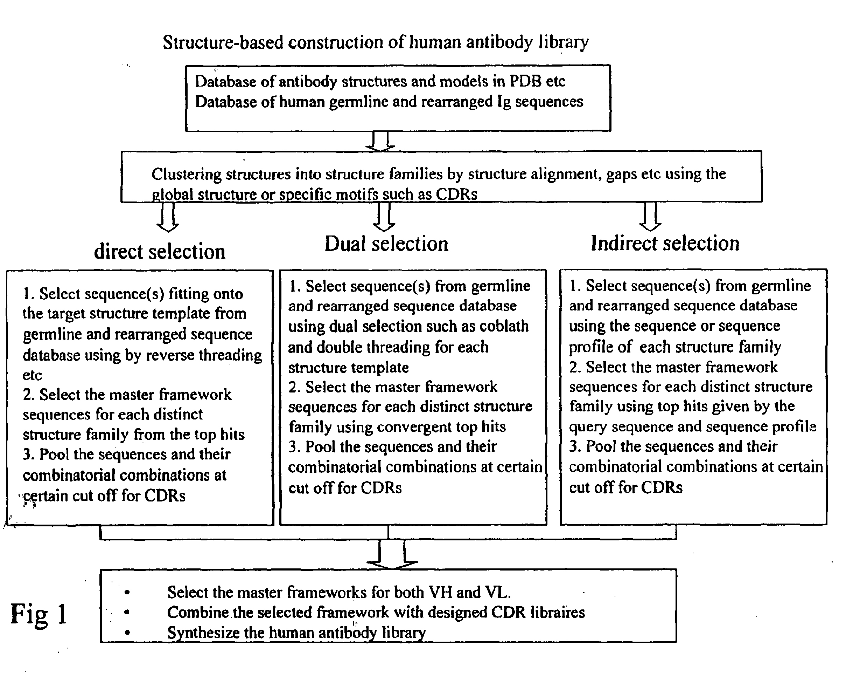

The general approach for constructing a structure-based human library is illustrated by a flow chart in **Figure 1.**

As illustrated in **Figure 1**, antibody structures and models in various protein structure databases such as the PDB are collected. The structural repertoire of these antibody molecules are mapped out in their three dimensional shape space. It is believed that conservation and variation in the shape space should make it possible to develop some general frameworks that remain constant across different species. On the other hand, variation in the shape space should make it possible to capture the functional diversity of antibody against a wide array of antigens in specific antibody regions.

Referring to **Figure 1**, the variable regions in shape space are clustered either separately (such as CDR3) or in combination (CDR1 & CDR2) into distinct families with or without certain conserved structural frameworks.

Still referring to **Figure 1**, these structural clusters, the ensemble average, and/or their corresponding sequence profiles are used to map out the corresponding sequence in human germline (or in a rearranged antibody sequence database) to find optimal sequences or sequence profiles within each family.

As diagramed in three boxes in the middle portion of **Figure 1**, at least three approaches can be taken to exploit the information generated by structure-based clustering of target antibody sequence(s). As described in the left box, one approach is to directly select sequences that fit onto the target structural template by using algorithms such as reverse threading, PSI-BLAST and profile HMM. For example, a library of recombinant antibodies can be generated by 1) selecting from a human antibody germline database sequence segments that fit onto a structural template of a target FR region of an antibody; 2) selecting from a protein database sequence segments that fit onto a structural template of a target CDR region of the antibody; and 3) combining the selected FR and CDR segments to build the library of recombinant antibodies which are then synthesized and screened against a target antigen in vitro or in vivo.

As described in the right box in the middle section of **Figure 1**, another approach is to indirectly select antibody sequences using a target sequence or sequence profile built based on a structural template of a target antibody. For example, a library of recombinant antibodies can be generated by 1) aligning the target sequence or sequence profile with tester sequences from a protein database (e.g., human germline antibody sequence database or PDB) by using BLAST or multiple sequence alignment methods such as profile HMM; 2) selecting the tester sequences with homology to the target sequence (e.g., sequence homology of at least 15%); and optionally 3) evaluating the structural compatibility of the selected tester sequence with the structure template of the target sequence or sequence profile. This process can be carried out to construct a library of recombinant antibodies by targeting a particular region of an antibody such as a CDR, FR, and combination thereof. The selected tester sequences may be profiled based on variability in each amino acid residue and those variants with low occurrence frequency (e.g., 5 times out of 100 selected tester sequences) may be filtered and discarded. The rest of the selected tester sequences may be pooled and combined by a combinatorial combination of the amino acid variants in each residue position. The tester sequences selected by targeting the CDR region and the ones targeting the FR regions may also be combined; and the combined sequences may be filtered based on their structural compatibility with the target antibody. The library of recombinant antibodies can be synthesized and screened against a target antigen in vitro or in vivo.

As described in the middle box in the middle section of **Figure 1**, yet another approach is to select antibody sequences based on a target structural template combining methods described in the left and right boxes. For example, a library of recombinant antibodies can be generated by 1) aligning the sequence or sequence profile of the target structural template with tester sequences from a protein database (e.g., human germline antibody sequence database or PDB) and reverse threading the tester sequences onto the target structural template by using a structure/ sequence dual selection algorithm such as COBLATH; and 2) selecting the tester sequences with homology to the target sequence (e.g., sequence homology of at least 15%) and structurally compatible with the target structural template. This process can be carried out to construct a library of recombinant antibodies by targeting a particular region of an antibody such as a CDR, FR, and combination thereof. The selected tester sequences may be profiled based on variability in each amino acid residue and those variants with low occurrence frequency (e.g., 5 times out of 100 selected tester sequences) may be filtered and discarded. The rest of the selected tester sequences may be pooled and combined by a combinatorial combination of the amino acid variants in each residue position. The tester sequences selected by targeting the CDR region and the ones targeting the FR regions may also be combined; and the combined sequences may be filtered based on their structural compatibility with the target antibody. The library of recombinant antibodies can be synthesized and screened against a target antigen in vitro or in vivo.

There are several advantages associated with this approach of mapping the functional space of proteins using diversity libraries that are designed by sampling the diversity in shape space rather than in sequence space.

First, protein-protein interactions between ligand and receptor, antigen and antibody are conducted in well-defined conformation in space. Therefore, antibody libraries should be designed to map the 3-dimensional space populated by antibodies in order to target antigens with different shapes.

Second, compared to the larger sequence repertoire, structure repertoire of antibodies is limited to a small number of canonical structures in its main chain conformations in the CDR regions which are docked onto a common core structure for both the variable light and heavy chains. The simplicity in structure repertoire makes it easy to map the functional diversity based on variation in its 3-dimensional space and simple to cluster seemly complicated sequence pools into distinct families for library construction.

Third, it is conceived that the conserved nature of the structural repertoire of immunoglobins across very different species (Barre et al. (1994) "Structural conservation of hypervariable regions in immunoglobins evolution" Nature Struct. Biol. 1:915-920) that clustering structure repertoire of antibodies from different species into distinct families is a viable approach to map its functional space. This approach is simple yet functionally more relevant for selecting and constructing the diversity libraries once it is applied to the sequence repertoire for a specific species. This is particularly important for constructing human antibody libraries for therapeutic application or for humanizing murine antibodies by using human-derived sequence repertoire for its counterparts. In contrast, sequence homology-based approaches would be less flexible and hard to transfer from species to species if sequence homology is relatively low.

Moreover, the structure-based construction of sequence libraries makes it possible to apply various methods developed in structural biology to filter apparent complexity in sequence spaces based on structural or physical principles, in addition to the tools used in sequence analysis that are largely relied on the principles of evolution.

Accordingly, the present invention provides a method of constructing a master library of functionally representative antibody. This master library is formed by a repertoire of antibody sequences adopting distinct classes of structures that covers, ideally, almost all of the 3D structural ensembles and/or ensemble averages of all vertebrate antibodies.

According to the present invention, a master library of functionally representative antibody is represented by a library of antibody sequences adopting distinct classes of structures that covers, ideally, almost all families of the 3D structures of all vertebrate antibodies. Although a protein may have astronomical number of possible sequence combinations (about $10^{16}$ for a small protein of 100 residues (Dill (1985) Biochem. 24:1501-1509), all antibodies adopt a characteristic "immunoglobulin fold" globally. The natural antibody repertoire shows an amazing ability in recognizing a wide variety of molecules. To confer such diverse functions of binding ability to a vertebrate's antibodies, an extremely diverse sequence repertoire (about $10^{12}$ possible combinations between the sequences of mouse heavy chain and light chains) is created by random genomic splicing of heavy and light chains with high variability in both sequence and length in their CDRs.

The structural repertoire to accommodate the much larger sequence repertoire is, however, surprisingly small. Only a limited number of canonical backbone conformations are found to account for structures adopted by the CDRs that are docked onto highly conserved immunoglobulin scaffold.

According to the present invention, it is believed that antibody achieves its functional diversity by decorating a diverse array of amino acids onto a finite number of CDR canonical structures. The present invention clusters antibodies with experimental or modeled structures into distinct families. By clustering the antibodies according to their 3D structures instead of using conventional methods of classification based on sequence homology, each family of the structure repertoire should better represent the population of antibodies with binding geometry complementary to the recognition sites of potential antigens, although the binding affinity could be further optimized by matching the shape and chemical nature of the specific amino acids. Therefore, the approach taken in the present invention tends to maximize the functional diversity of antibody in recognizing and binding to a wide array of antigens *in silico* and meanwhile to minimize the sequence space required for efficient screening *in vitro* or *in vivo*.

2) <u>Construction of antibody sequence library based on structural constraints</u>

Once structural families are identified, either the cluster containing multiple members, a representative member, or an ensemble average of the cluster if possible, can be used as structural constraints to either select for optimal sequences or to construct sequences for further constructing sequence libraries.

There are several ways to use these structural families from sampling antibody structure databases as the constraints for constructing desired sequence libraries. The main chain conformations of 3D structures within a structural family or cluster are called structure ensembles or structure templates. The ensemble average is referred to the average structure of all members within a structure cluster or family when it is physically meaningful to take average of the main chain conformations. If it is not physically meaningful or possible to take average for all members within a structural cluster or family due to the difference in length, etc. a representative structure may be used to represent the "average structural properties" of all members within a structural family or cluster. The structure ensembles or templates, ensemble average, or representative structures described above are collectively referred to herein as the "structural templates".

The difference in using these terms in describing structural constraints depends on how much structural constraint within a cluster should be included in constructing sequence libraries. For structural constraints, the most stringent and reasonable approach should be to include all ensemble structures or templates within a cluster or family. The ensemble average if done properly, may be the simplest structural constraint and easy to compute. If taking ensemble average is not physically meaningful, the representative structure may be a compromise to replace constraints by structure cluster.

Once the structural constraints are identified, there are several ways to construct sequence libraries by applying structural constraints. The procedure to select and construct sequence or sequence libraries that are compatible to the structural constraints is called reverse engineering including but not limited to reverse threading. However, an important aspect of current invention is to restrict the sequence database for library construction to specific species and/or to even the specific population of the same species. For therapeutic purpose, the human immunoglobulin sequence database are preferably used to construct human-derived antibody libraries, especially in the frameworks of the variable regions. In the CDR regions, sequences with non-human origins may optionally be used to increase the diversity of these regions so as to increase the chance of finding antibodies with novel or improved function(s). The methods in applying both the physical and evolutionary constraints to construct sequence libraries are described in detail below.

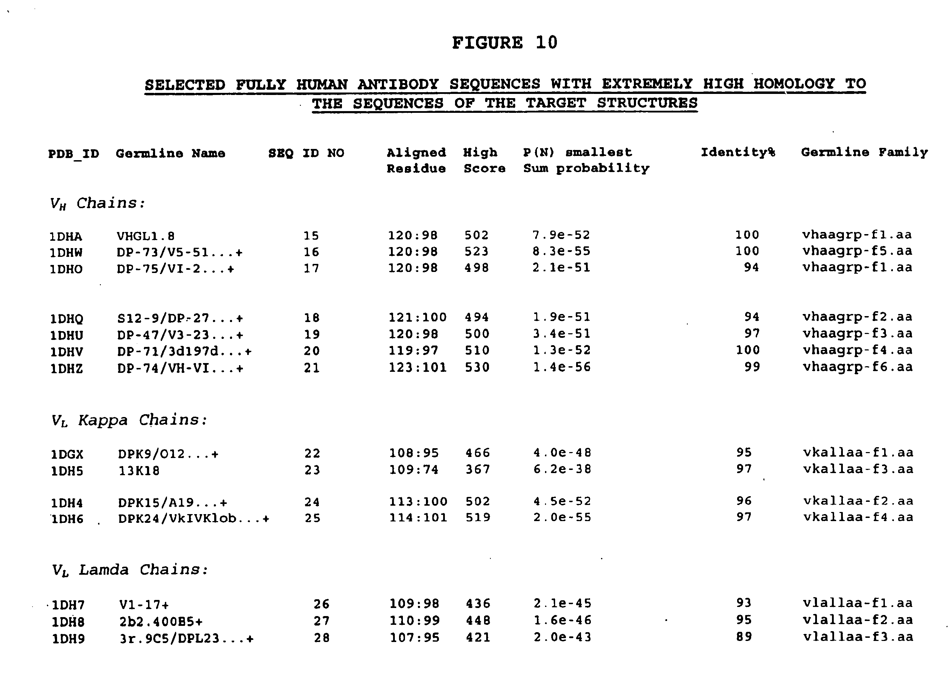

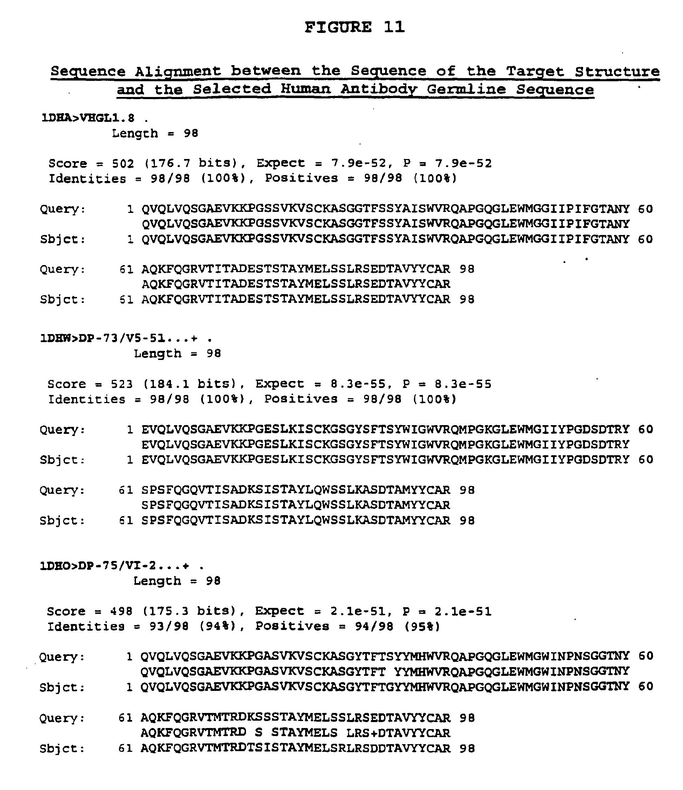

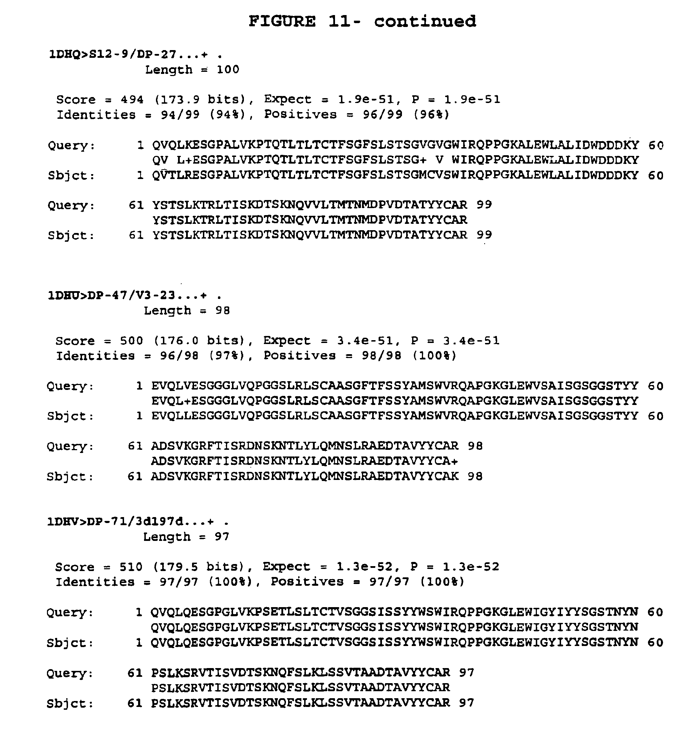

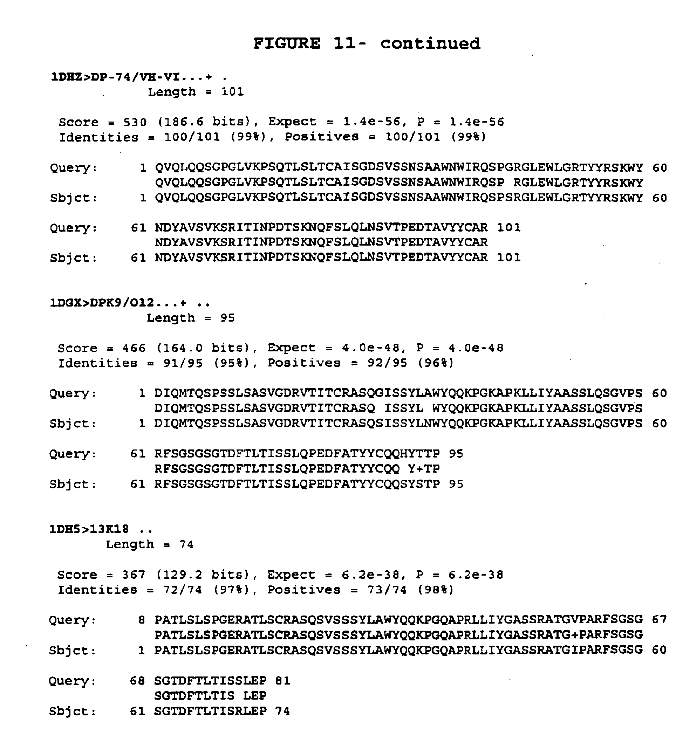

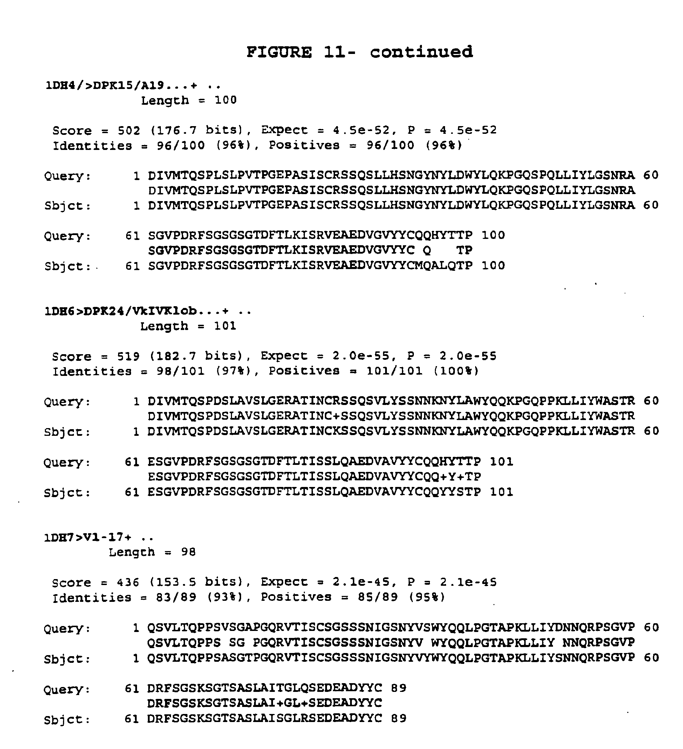

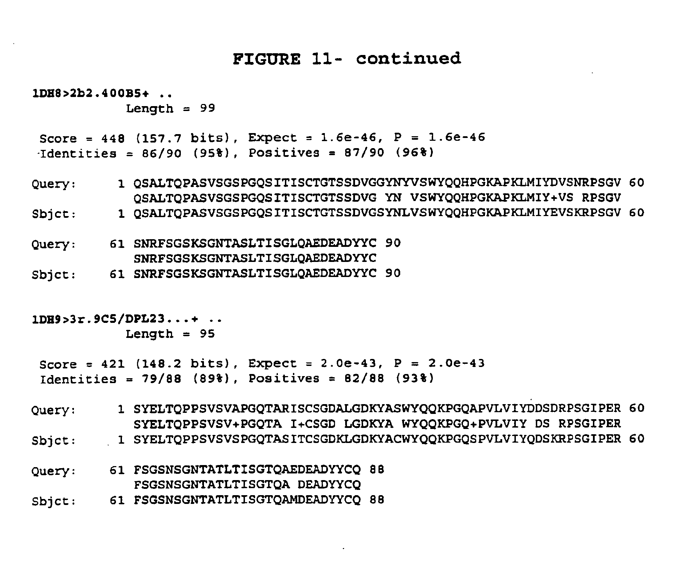

One method is to use the sequence that is compatible to the ensemble average structure or the representative member within a structure cluster to search for the optimal sequences from the germline sequence database. This will usually yield the sequence with the highest sequence identity to the query sequence using BLAST as demonstrated in Section 3 below (**Figures 10** and **11**).

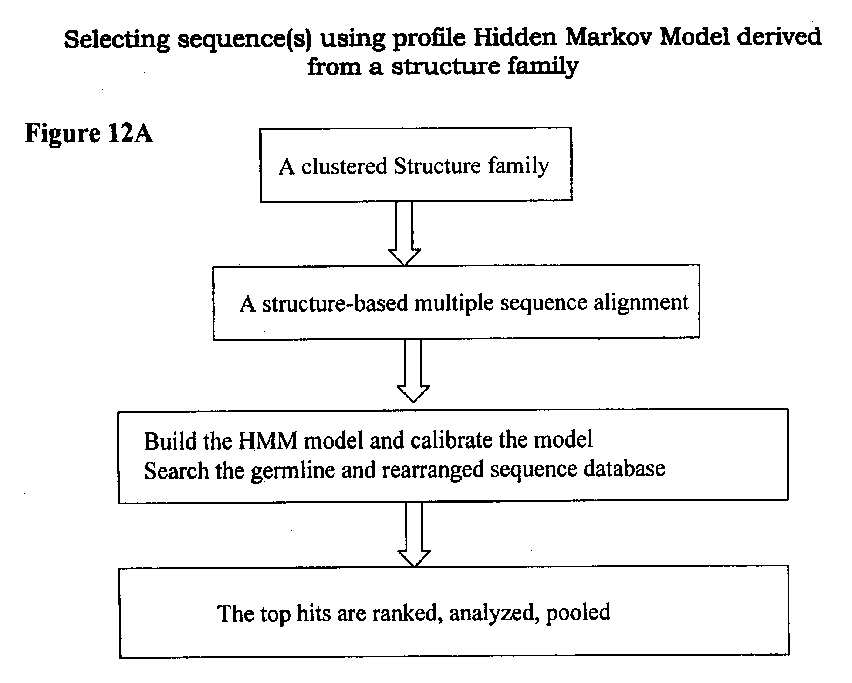

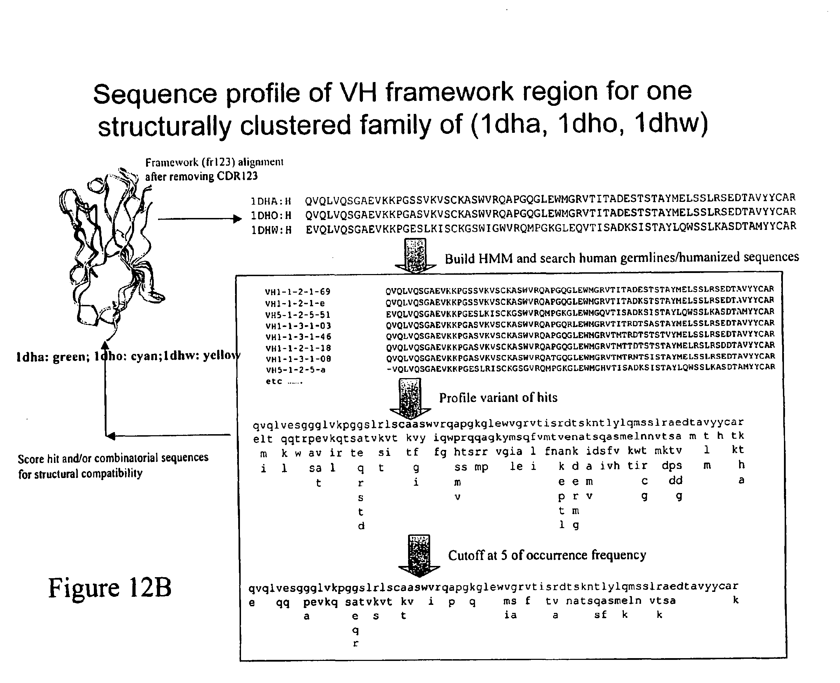

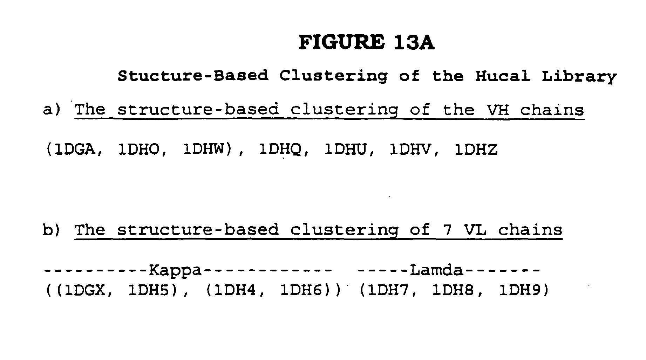

The clustered structures within a structure family can give multiple sequence alignments based on 3D structures. These aligned sequences might come from different species; they may be close or remote sequence homologues. The multiple sequence alignment can be used, however, to build a profile Hidden Markov Model (HMM); and this HMM will then be used to search for the close and/or remote human homologues from human sequence database such as the human germline and/or rearranged sequence database as demonstrated in Section 3 below (**Figures 12** and **13A-C**).

A more direct way to search for sequences compatible to structural constraints is to thread amino acid sequences from human germline and/or rearranged sequence database onto structure templates of the structural cluster and to find out the optimal scoring sequences on their target structure templates. These sequences can be then used for constructing sequence libraries for the structure cluster. This procedure is called reverse threading because it tries to find the best sequences fitting to the target structure templates, which is the opposite of threading which tries to find the best structure template from a structure library for a given sequence.

Additionally, the top hits of the sequences found for a structure cluster or queried sequences may be profiled by threading multiple amino acids at each position in a combinatorial approach to select for the best "consensus sequence" compatible with the structural ensemble and/or ensemble average. This process of searching for consensus sequence is different from the consensus sequence from the method of using simple sequence average at each position described in Knappik, et al, *supra.* The consensus sequence according to the present invention is created using the physically oriented reverse engineering approach using all possible combination of amino acids that are allowed at each position from the retrieved sequences but are optimized by scoring their compatibility with the structural constraints.

The human antibody sequences that are selected according to these criteria for the framework regions can serve as the sequence template for building a master framework for constructing the human antibody library of the present invention. These selected human sequences are then pooled together and included in the master framework. The same methods can be used to construct the sequence libraries for CDR regions if the structure templates for each canonical structure family of CDRs are used to construct the sequence libraries for these regions.

Once the master framework of human antibody is constructed, mutagenesis can be carried out to diversify specified region(s) in the master framework. For example, CDR regions, especially CDR3 of the heavy chain, of the master framework can be randomly mutagenized to mimic the natural process of antibody diversification. The mutagenized antibody sequence may be further selected *in silico* based their compatibility to the structural ensemble average. All of the antibody sequences selected in these processes are pooled to form a master library of human antibody which can be screened against a wide range of antigens *in vitro* or *in vivo.*

Since the selection and construction of the antibody library of the present invention is based on structural clustering, not simple sequence homology alignment, it is thus possible to further limit the number of antibody sequences in the library and yet not to sacrifice the functionally relevant sequences. For example, multiple human antibody sequences may be highly diverse in their sequences and yet adopt the same 3D structure when threaded onto the structural ensemble average.

Further, for those antibody sequences mutagenized randomly in the CDR3 region, not all structures of randomized CDR3 are compatible with the framework structural ensemble averages. Consequently, a fewer number of CDR3 loops that are structurally diverse will be selected, and therefore a fewer number of human sequences selected. As a result, the sequence space of antibody to be screened is reduced in size without sacrificing diversity in antibody functionality.

By using the method, a master library of human antibody sequences is selected and constructed to better represent all antibody structures in the vertebrate antibody repertoire that are functionally important for high affinity binding to antigen and eliciting antibody-dependent cellular responses. Such a functionally representative library provides a structurally diverse, and yet functionally more relevant source of antibody candidates which can then be screened for binding to a wide variety of target molecules, including but not limited to biomacromolecules such as protein, peptide, and nucleic acids, and small molecules.

The method of present invention is an efficient way of constructing a digital library of antibody which represents most of the 3D structures of antibodies that are functionally relevant. Thus, the human antibody sequences selected from the reverse engineering process such as threading are finite and yet covers most of the functionally relevant structures of antibody in human antibody gene pool.

In contrast, the current methods of construction of antibody library in vitro involve isolation of cDNA libraries from immunized human antibody gene pool, naive B-cell Ig repertoire, or particular germline sequences. Barbas and Burton (1996), supra; De Haard et al. (1999), supra; and Griffiths et al (1994), supra. These libraries are very large and extremely diverse in terms of antibody sequences.

The conventional approach is to create a library of antibody as large, and as diverse as possible to mimic immunological response to antigen in vivo. Typically, these large libraries of antibody are displayed on phage surface and screened for antibodies with high affinity binding ability to a target molecule. Such a "fishing in a large pond" or "finding a needle in a huge hey stack" approach is based on the assumption that simple increase in the size of sequence repertoire should make it more likely to fish out the antibody that can bind to a target antigen with high affinity.

There may be several problems associated with such a conventional approach. A simple increase in the size of sequence library may not necessarily correlate with an effective increase in functional diversity. Further, due to the physical limit on making an extremely large experimental library, it may be very difficult to construct a library with diversity over $10^{11}$ in vitro in the lab. The library that is actually screened experimentally probably presents only a fraction of the sequence repertoire at the theoretically predicted size. In addition, there is legitimate concern that with the difficulties and the under representation problems associated with handling and manipulation of an extremely large library in vitro, time and money may be lost in an effort trying to increase the size of the library and yet not increasing functional diversity significantly.

Another approach existing in the art is to design an artificial antibody library computationally and then construct a synthetic antibody library which is expressed in bacteria. Knappik et al., supra. The artificial antibody library was designed based the consensus sequence of each subgroup of the heavy chain and light chain sequences according to the germline families. The consensus was automatically weighted according to the frequency of usage. The most homologous rearranged sequences for each consensus sequence was identified by searching against the compilation of rearranged sequences, and all positions where the consensus differed from this nearest rearranged sequence were inspected. Furthermore, models for the seven $V_H$ and seven $V_L$ consensus sequences were built and analyzed according to their structural properties. A library of artificial antibodies were then constructed and expressed in E. coli. This library constructed can be used to screen for antibodies with high affinity binding to a target molecule.

However, there is a major problem concerning such an approach as far as therapeutic applications of the selected antibody are concerned. Although derived from human sequence pools, the consensus sequences found by using this approach, by definition, are not natural sequences. (1) Combination of sequences, albeit human sequences, at various positions may give rise to new immunogenic epitopes, thus significantly limiting therapeutic applications of the selected antibodies to human, whereas the method described here can give either fully human sequences or human derived sequences or both. (2) Consensus sequence has its own serious limitation. Moreover, the definition of consensus sequence may be too arbitrary and such artificial sequences defined may not be representative of a natural, functional structure, although experimental test and structure analysis may eliminate some unfavorable amino acid combinations. (3) Although the consensus sequences designed to cover mainly those human germline sequences that are highly used in rearranged human sequences, it might bias consensus sequence library toward a limited number of antigens exposed to human being so far, whereas sampling functional space by mapping structures of different species covers a wide range of functional epitopes of antibodies exposed to a wide array of antigens. This would be very important for designing antibody libraries to target novel antigens.

By contrast, the method of the present invention is based on structural constraints of antibodies directly or derived from natural sources. According to the present invention, a complete structural repertoire of all antibodies available including both human and other vertebrates can be analyzed for structural ensembles and/or ensemble averages within each representative 3D structural family. Based on this analysis, the structural models are clustered into distinct structure families, each of which includes one or more representative members. These structure families ideally should represent evenly the structure space which all antibodies, including those from humans and other animals, would adopt. Thus, by collecting and building structural models for each structural ensembles and/or their ensemble averages for these antibodies. a relatively comprehensive survey of functional repertoire of antibodies across the species may be achieved.

Further, the method of the present invention involves using selection of native human antibody sequence which fits the best onto the structural ensemble or ensemble average in each of the structural family. By selecting and pooling the native human sequences based on the 3D structural templates in each family, a more focused human antibody library is created. The library may be smaller than the native antibody gene pool and yet representative of the functional repertoire of antibody in all vertebrates.

Moreover, the sequences of the antibody library constructed using the method of the present invention are closely related to human sequences. The antibody selected from this library against a target molecule should be more desirable than an artificial, non-human antibody for therapeutic applications and humanization of non-human antibodies. This approach can minimize the potential of creating new immunogenic epitopes associated with using synthetic antibody sequences derived from randomization of the consensus sequences.

In addition, the library generated according to the method of the present invention should encompass a broader spectrum of the basic function of an antibody: antigen recognition and neutralization. Since the family of the structural ensemble averages are clustered based on not only the structures adopted by known human sequences, but also structures collected from other vertebrates. In particular, monoclonal antibody produced by mice is a rich source of structures to be included in the process of clustering. Since these monoclonal antibodies are generated from immunization against a vast number of antigens, including these antibodies in the clustering process should tend to enlarge the functional repertoire, although a few special features specific to mice should be taken into account or avoided when applied to human. This approach may effectively avoid the problem associated with known human antibody sequences that are restricted to those isolated against a limited number of antigens.

**2. Process of clustering antibodies based on their structural ensemble or ensemble averages.**

According to the present invention, a master library of human antibody sequence can be constructed based on 3D structural clusters of antibodies from human and other vertebrates. The 3D structural ensembles and/or the ensemble averages serve as master frameworks upon which human antibody sequences are mapped onto by threading etc and those best compatible are selected to form the master library of human antibody.

The structural ensemble or ensemble averages of antibody from various species may be modeled *in silico* by using various structural alignment methods for comparing antibodies with known 3D structures. By "known 3D structures" is meant x-ray crystal structures, NMR structures, and 3D structures of antibody modeled *in silico*. Currently, there are about 360 antibody 3D structures deposited in the Protein Data Bank (PDB) which include 306 X-ray structures, 17 NMR structures, and 32 modeled structures.

For example, antibody structural cluster can be generated by pairwise structural alignment for $V_H$ or $V_L$ of two or more antibodies with known 3D structures from the PDB. Various algorithms have been developed for protein structure alignment, including those attempting global optimization of the alignment path for some similarity measure using dynamic programming (Orengo et al. (1992) Proteins 14:139-167), Monte Carlo (Holm and Sander (1993) J. Mol. Biol. 233: 123-138), 3D clustering (Fischer et al. (1992) J. Biomol. Struct. Dyn. 9: 769-789; and Vriend and Sander (1991) Proteins 11:52-58) and graph theory (Alexandrov (1996) Protein Eng. 9: 727-732), and algorithm using incremental combinatorial extension (CE) of the optimal path (Shindyalov and Bourne (1998) Protein Eng. 9:739-747; and Shindyalov and Bourne (2001) Nucleic Acid Res. 29:228-229).

In an embodiment of the present invention, the antibody structural families are clustered by structural alignment using the CE algorithm. Compared to Monte Carlo and 3D clustering algorithms, the CE algorithm significantly reduces the search space and empirically establishes a reasonable target function. The CE target function assumes that alignment path is continuous when including gaps and there is an optimal match between the pair. Various protein properties can also be used with CE algorithm, for example, 1) structure superposition as rigid bodies; 2) inter-residue distance, 3) environmental properties (e.g., exposure, secondary structure); 4) conformational properties (e.g., bond angles, dihedral angles, and orientation with respect to the protein center of mass).

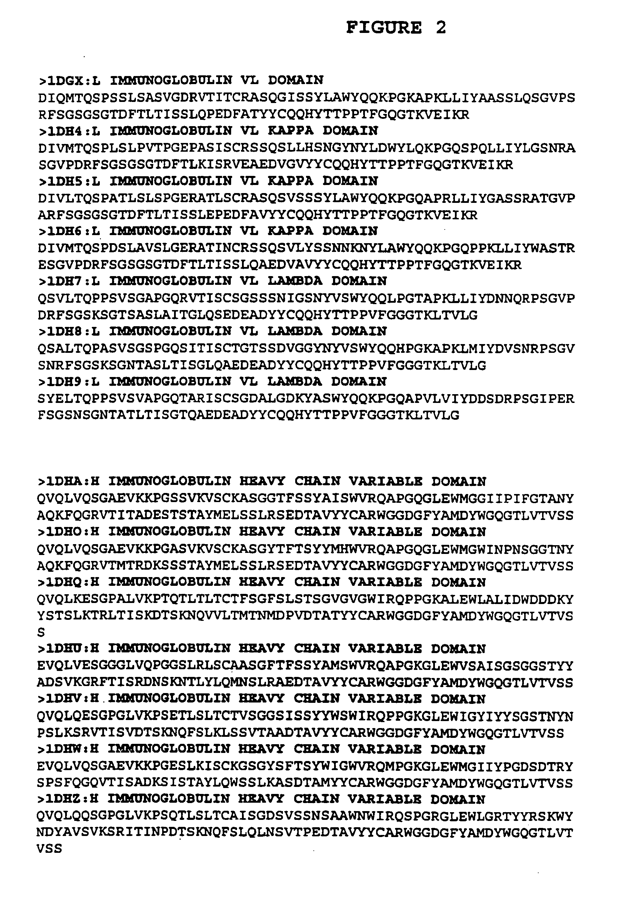

As a proof of principle, 3D structures of a series of artificial antibody sequences were compared by using the CE algorithm and classified into a smaller number of clusters based on their 3D structural alignments. These artificial sequences tested are the consensus sequences of the subgroups of the heavy and light chain sequences according to the germline families. Knappik et al., supra. These sequences shown in **Figure 2** consist of the following 7 VH and 7 VL consensus sequences:

| $V_H$ | $V_L$ |
|---|---|
| 1DHA [SEQ ID NO: 1] | 1DGX [SEQ ID NO: 8] |
| 1DHO [SEQ ID NO: 2] | 1DH4 [SEQ ID NO: 9] |
| 1DHQ [SEQ ID NO: 3] | 1DH5 [SEQ ID NO: 10] |
| 1DHU [SEQ ID NO: 4] | 1DH6 [SEQ ID NO: 11] |
| 1DHV [SEQ ID NO: 5] | 1DH7 [SEQ ID NO: 12] |
| 1DHW [SEQ ID NO: 6] | 1DH8 [SEQ ID NO: 13] |
| 1DHZ [SEQ ID NO: 7] | 1DH9 [SEQ ID NO: 14] |

The seven $V_H$ consensus sequences stored in the PDB, 1DHA, 1DHO, 1DHQ, 1DHU, 1DHV, 1DGW, and 1DHZ, correspond to VH1A, VH1B, VH2, VH3, VH4, and VH6, respectively, as described in Knappik et al., supra. The seven $V_L$ consensus sequences stored in the PDB, 1DGX, 1DH4, IDH5, 1DH6, 1DH7, 1DH8, and 1DH9, correspond to VLκ1, VLκ2, VLκ3, VLκ4, VLλ1, VLλ2, and VLλ3, respectively, as described in Knappik et al., supra.

The 3D structural models of these VH and VL consensus sequences built by Knappik et al. were retrieved from the PDB and compared by using the CE algorithm. It should be noticed that CDR3 of the heavy and light chains were the same for all frameworks in the modeled structures. The CE program compares pairs of protein structures of polypeptide chain or their segments based on the root mean square difference (RMSD), their statistical significance (Z-score), length difference, allowable gaps (given as a percentage of the total number of residues without a matching partner relative to the complete alignment) and sequence identity.

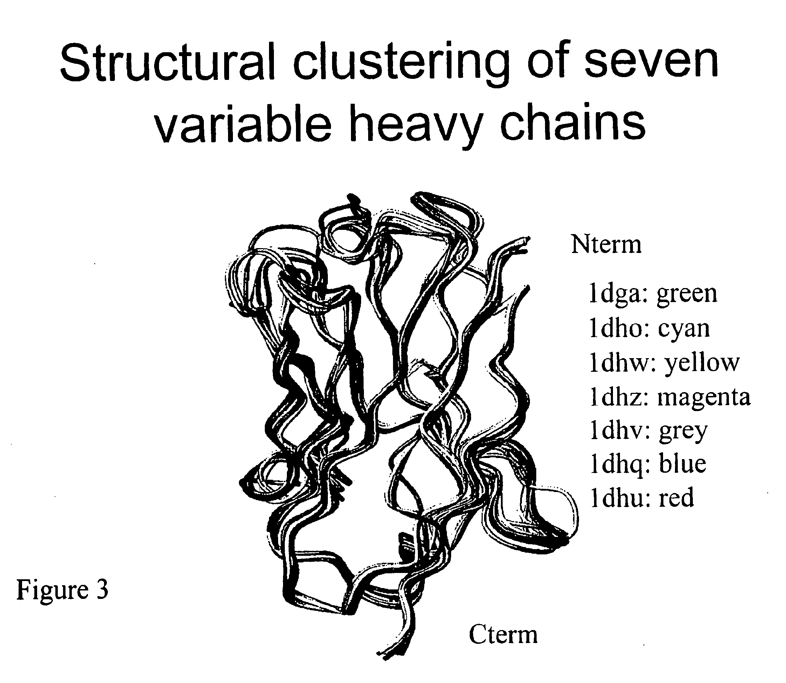

**Figure 3** shows the structures of the seven VH sequences superimposed on each other. The structures are aligned by superimposing the Ca atoms using the CE with RMSD < 2Å and Z-score > 4. As shown in **Figure 3**, the seven VH sequences have a range of conformational variability, especially in the CDR regions. According to Knappik et al., these seven structures cover all canonical classes of the CDRs of the VH structures.

However, by using the method of the present invention, a closer look into the seven structures reveals a striking conformational similarity between at least three of the seven VH sequences. By using the CE algorithm, five VH sequences (1DHA, 1DHO, 1DHW, 1DHZ, 1DHV) of the 7 consensus sequences families can be clustered into one structural family with RMSD < 1.5Å and Z-score > 4 and with sequence identity ranging from 48% to 87% using 1dha as standard. Further clustering of the 5 VH sequences (1DHA, 1DHO, 1DHW, 1DHZ, 1DHV) reveals that the 3 VH sequences (1DHA, 1DHO, and 1DHW) collapse into one structural family with RMSD <0.7 Å and Z-score >6 using 1DHA as standard, even though their sequence identity ranges widely **from** 72% to 87% relative to 1DHA.

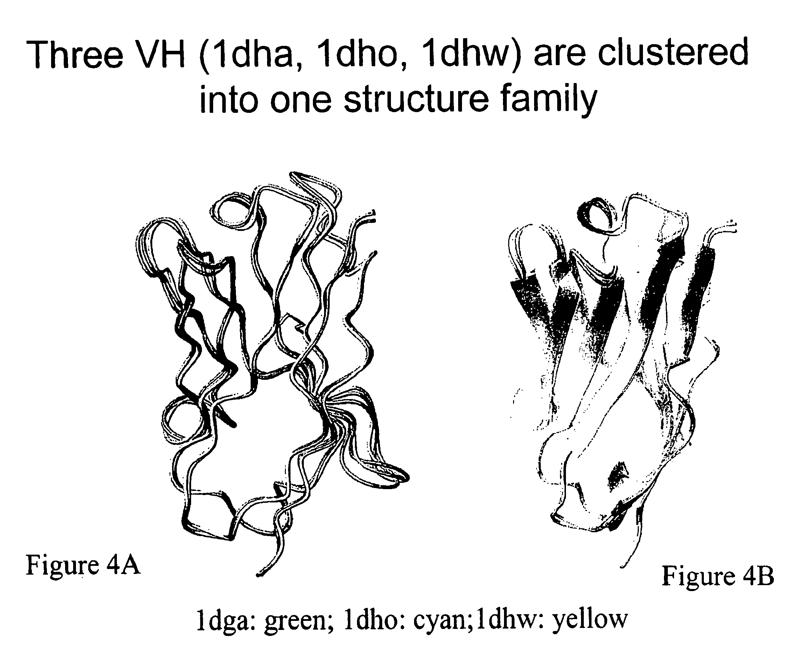

**Figure 4A** shows the Ca trace of the superimposed structures of these 3 VH sequences (1DHA in green, 1DHO in cyan, and 1DHW in yellow). **Figure 4B** shows the superimposed structures with a ribbon representation of the β-sheets of the VH frameworks. As shown in both **Figures 4A** and **4B**, these three structures have an almost perfect superposition (RMSD <0.7 Å) even in the CDR regions. According to the present invention, these three structures are clustered into one VH structure family based on the structural clustering criteria of the present invention. The rest of the 7 VH sequences: 1DHQ, 1DHU, 1DHV, and 1DHZ, have distinctly different structures and thus clustered into 4 distinct structural families with only one member within each family according to the present invention. Thus, by using the method of the present invention, the 7 consensus germlines VH sequences of human antibody designated by Knappik et al. can be presented by 5 distinctly different structural families. The preferred criteria are RMSD < 1 Å for each structural family and Z-score > 6.

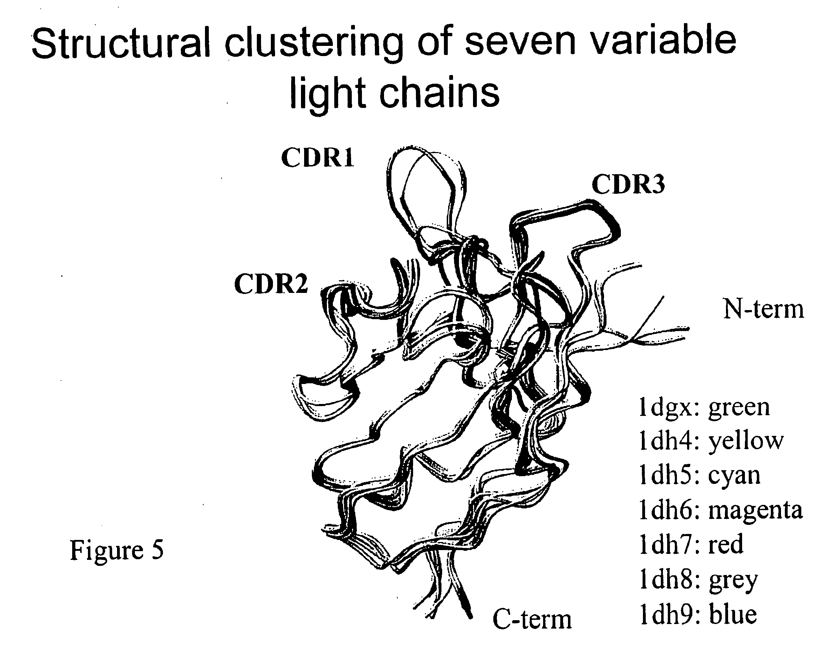

**Figure 5** shows the structures of the seven VL sequences retrieved from the PDB and superimposed on each other. The structures are aligned by using 1DGX as the reference structure with RMSD< 1.6Å and Z-score >6. As shown in **Figure 5**, the seven VL sequences have a wide range of conformational variability, especially in the CDR regions (The structural flexibility at N- and C-termini are discarded here). According to Knappik et al., these seven structures cover all canonical classes of the CDRs of the VL structures.

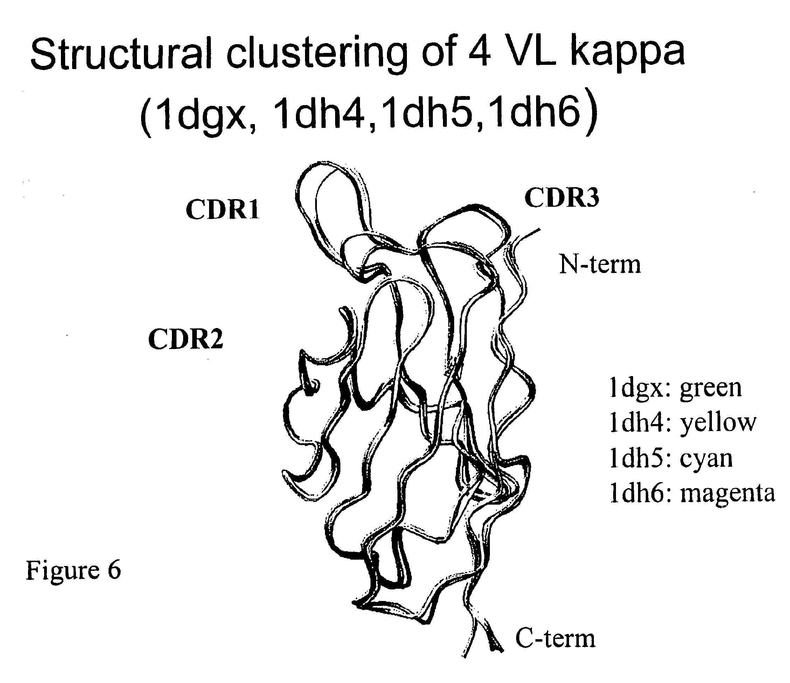

However, by using the method of the present invention, the seven VL sequences can be re-clustered into smaller number of families. By using the CE algorithm, four VL sequences (1DGX, 1DH4, 1DH5 and 1DH6) of the 7 consensus sequences families can be clustered into one structural family with RMSD < 0.6 Å and Z-score >6 and with sequence identity ranging from 67% to 80% using 1DGX as the structure reference. **Figure 6** shows the superimposed 1DGX(green),1DH4 (yellow), 1DH5 (color cyan) and 1DH6 (magenta) with similar conformation but varying length in the CDR regions. These four sequences also belong to the VL kappa sequence family.

Further clustering of the 4 VL sequences (1DGX, 1DH4, IDH5 and 1DH6) reveals that the 2 VL sequences (1DH4 and 1DH6) collapse into a structural family with RMSD <0.6 Å and Z-score > 6 with length of CDR1 loop closer to each other, using 1DGX as the reference, while two VL sequences (1DGX and 1DH5) can be clustered into another structural family (data not shown).

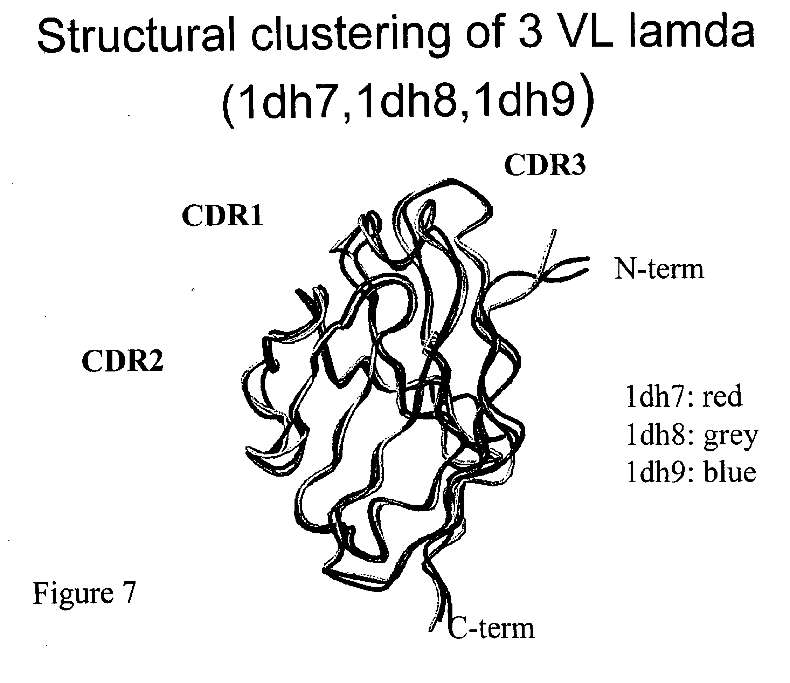

**Figure 7** shows three superimposed structures of 1DH7, 1DH8, and 1DH9 in lamda variable light chain, can be clustered into 1 structure family with RMSD < 1.5Å and Z-score > 6 using 1DGX as the reference according to the present invention.

Thus, by using the method of the present invention, the 7 consensus germlines VL sequences of human antibody designated by Knappik et al. can be represented by 2 to 3 distinctly different structural families. Combined with the clustering of the 7 consensus germlines VH sequences into a 5 structural families, the total structural family for human antibody germline can be represented by 5 x (2 to 3) = 10 to 15 distinct families, a much reduced structural repertoire than the germline sequence repertoire of Knappik et al.: 7x7=49.

The structures of the consensus germline VH and VL sequences can also be clustered based on the conformation ensemble adopted by a specific region of the VH or VL, such as a particular CDR region.

**Figure 8A** shows that CDR1 regions of the three lamda ($\lambda$) VL sequences (1DH7, 1DH8 and 1DH9) adopt similar conformations with RMSD < 1Å. Thus, structures of these three lamda VL sequences are clustered into one structural family according to the present invention.

**Figure 8B** shows that CDR1 regions of the 4 kappa ($\kappa$) VL sequences (1DH4, 1DH6, 1DGX and 1DH5) adopt similar conformations with RMSD < 0.6Å and gaps of 1-6 amino acids. Thus, structures of these four kappa VL sequences are clustered into one structural family according to the present invention.

**Figure 8C** shows that CDR1 regions of the two kappa ($\kappa$) VL sequences (1DH4 and 1DH6) adopt similar conformations with RMSD < 0.6Å and 1 amino acid gap in CDR1. Thus, structures of these two kappa VL sequences are further clustered into one structural family according to the present invention.

**Figure 8D** shows that CDR1 regions of the two kappa ($\kappa$) VL sequences (1DGX and 1DH5) adopt similar conformations with RMSD < 0.6Å and 1 amino acid gap in CDR1. Thus, structures of these two kappa VL sequences are further clustered into one structural family according to the present invention.

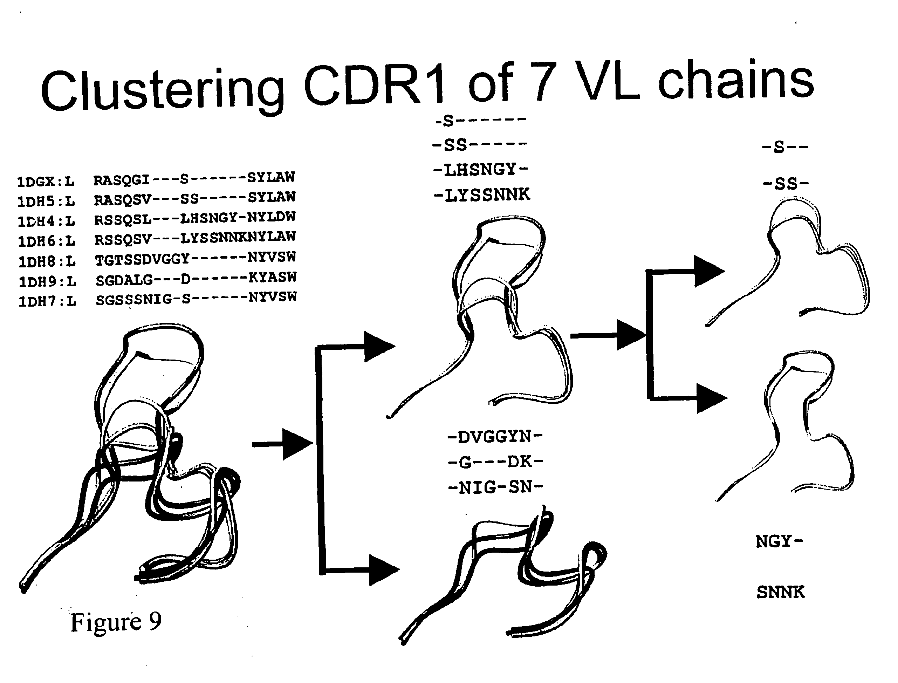

As a result of such clustering with a focus on a specific region of the VH or VL, regions, the number of antibody structure families might be clustered differently. **Figure 9** shows that clustering of the structures adopted by the seven consensus germline VL sequences based on the structural families in the CDR1 region led to two to three distinct families of antibody structures: (1DH7, 1DH8 and 1DH9), (1DH4 and 1DH6), and/or (1DGX and 1DH5). As shown in **Figure 9**, within each family, the members adopt similar conformations in its CDR 1 regions with varying length in amino acids.

Thus, by further clustering of antibody structures based on a more focused region of the global structure, i.e., CDR1, the seven consensus germline VL sequences of human antibody designated by Knappik et al. can also be represented by 2 to 3 distinctly different structural families. Combined with the clustering of the 7 consensus germlines VH sequences into 5 structural families, the total structural families for human antibody framwork sequences can be represented by 5x (2 to 3) = 10 to 15 distinct families, a much more reduced structural repertoire than the consensus framwork sequence repertoire of Knappik et al.: 7x7=49.

As illustrated by the above example, the method of the present invention enables one to reduce the size of the antibody sequence library by clustering them according to their 3D structural families. Since the structure of a protein or antibody determines its function in the biological system, the structural ensemble or ensemble average in each structure family of the present invention should represent the population of diverse antibody sequences sharing similar functions, e.g. in antigen recognition and affinity binding.

The above-described method of clustering structures of consensus human germline antibody sequences only serves as an example to illustrate the principal of the invention. It should be noted that such a clustering method is not limited to these structures. In a broader application, structures of both human and non-human vertebrate antibodies can be combined in a pool and clustered based their structural ensemble or ensemble averages or representative structure. This approach presumably reduces the risk of the biased library consisting only of structures of human antibodies generated by limited exposure to various antigens. By combining and clustering structures from both human and non-human vertebrate antibodies, this structural ensemble or ensemble average determined should better represent the functional epitope of the antibody family. In addition, compared to the approach based on consensus antibody sequences, the structural ensemble or ensemble average generated by using the methods of the present invention is based on some well-established structural principle instead of the ill-defined consensus sequences.

The following lists the principles followed in clustering structures:

a). Align structures based on the RMSD for C alfa carbon atoms in the backbone and Z-score and gaps in the length of amino acids.

b). Clustering structures into the same family progressively based on smaller RMSD values and smaller gaps in amino acids.

c). Clustering structure using globe or important motifs.

It is believed that because structural repertoire is better way to represent functional repertoire, starting from structure should provide an important and more rational basis for library construction. The antibody-antigen interaction occurs on the 3D structure space rather than 1D sequence space. The structure change in CDRs should be better represented in 3D space. Using the structure as the criteria without details into the exact interaction between Ag and Ab should be make it possible to score for human sequence better compatible with the representative structure motif or ensemble.

**3. Selection of Antibody Sequences that Fit onto the Targeted Structural Ensemble or Ensemble Average**

Once the structures of the antibodies are clustered using methods described above, either the structure ensemble within a cluster or its ensemble average or its representative member can serve as the target structural scaffold in the search for those human antibody sequences that adopt the same or similar 3D structure. For example, an ensemble average of the structures of a target antibody can be used as a structural template in the search in a protein database for antibody sequences with diverse sequences and yet retaining the same functionally relevant structure.

In a preferred embodiment, the human antibody sequences are selected from the human immunoglobulin germline sequences. The germline sequences have been clustered into different sequence families including the V-genes, D-genes and J-genes. The rearranged immunoglobin sequences are collected in the Kabat-Wu sequence databases (Johnson & Wu, Kabat Database and its applications: future directions (2001) 29, 205-206). These human immunoglobin sequences are retrieved from the Kabat-Wu sequence databases and stored in the human immunoglobulin (or antibody) sequence data of the present invention (**Figure 1**).

According to the present invention, a variety of methods can be used to search for those human antibody sequences that adopt the same or similar structure as the target structural scaffold. The following are examples of the methods that may be used for achieving this purpose.

1) Reverse threading

The conventional threading of protein sequence is used to predict the 3D structure scaffold of a protein. Typically, it is a process of assigning the folding of the protein by threading its sequence to a library of potential structural templates by using a scoring function that incorporates the sequence as well as the local parameters such as secondary structure and solvent exposure. Bowie et al. (1991) Science 253:164-170; Rost et al. (1997) 270:471-480; Xu and Xu (2000) Proteins: Structure, Function, and Genetics 40:343-354; and Panchenko et al. (2000) J. Mol. Biol. 296:1319-1331. For example, Rost et al. supra the threading process starts from prediction of the secondary structure of the amino acid sequence and solvent accessibility for each residue of the query sequence. The resulting one-dimensional (1D) profile of the predicted structure is threaded into each member of a library of known 3D structures. The optimal threading for each sequence-structure pair is obtained using dynamic programming. The overall best sequence-structure pair constitutes the predicted 3D structure for the query sequence.

In contrast, the reverse threading of the present invention is a process of finding the optimal sequence within a library of sequences to fit onto a target structure. Various scoring functions may be used to select for the optimal sequence(s) from the library comprising antibody sequences with various lengths. In a preferred embodiment, the scoring function is capable of discriminating the following interactions among different sequences with different lengths: (a) The interactions between the side chains and backbone template as well as between side chains; and (b) the gap penalties for sequences with varying lengths in CDR1, CDR2 and CD3 regions.

For example, amino acid sequences from a human germline immunoglobulin database can be threaded onto the 3D structure of the target structural template (or scaffold) and to search for the sequences with optimal acceptable scores.

2) Matching the target structure with the optimal sequence composition of multiple aligned sequence family

For this method, the optimal sequence that will fit onto the target structure is selected by matching the target structure with the optimal sequence composition of multiple aligned sequence family. The top hitting sequences found from human antibody sequence database can be optimized at each position with all possible composition to yield the best sequence composition that fits a target structure based on the scoring of the interactions between side chains and backbone and side chain and side chain.

3) Selecting the optimal sequence by homology alignment with the sequence of the target structure

Another method of selecting human antibody sequence that will fit onto the structural scaffold of each member of the structural family is through homologous alignment with the amino acid sequence of the representative structure within a family. Such a method of structure-based sequence alignment can be practiced by the following procedure.

The target structure may be a member of the structural family clustered by using the method described in Section 1. This target structure serves as a structural scaffold with which a library of human antibody sequences are matched. The matching process is performed through homologous alignment of the library of human antibody sequences with the amino acid sequence of the target structure (the sequence template). This method is a process of indirect structure-based sequence query, instead of directly searching for sequences that can be thread onto the structural scaffold in a reverse threading process described in Section 1) above. Through homologous alignment with the sequence template of the target structure, optimal human antibody sequences will be efficiently selected based on simple sequence alignment method such as BLAST.

The following is an example of selecting optimal human antibody sequence(s) by using the indirect structure-based sequence alignment according to the present invention.

This example demonstrates that fully human antibody sequences with extremely high sequence homology (100% sequence identity) could be found by matching the library of human antibody sequences against the sequence template of the target structure, i.e., the query sequence. It can be reasonably assumed that the antibody sequence having the highest sequence identity with the query sequence should adopt the same or a very similar structure as that of the query sequence. This sequence(s) is included in the library of selected human antibody to represent the same scaffold as the target structure. For each member of the structural family, human antibody sequences can be selected to match the sequence of the structural ensemble or ensemble average (there is only one member within each family). The selected human antibody sequences are combined to form a library relatively small in sequence space and yet functionally diverse.

In this example, the library of human antibody germline sequences (HuCal sequences) serves as the library of human antibody sequences **(Figure 1).** The HuCal sequences in fasta format as shown in **Figure 2** were divided into variable light chains and variable heavy chains. These sequences were then used to compare with human germline sequences using Blast (Basic Local Alignment Search Tool)

The amino acid sequences of the consensus human germline sequences that are clustered by using the method described in Section 1 serve as the query sequences. Each of the query sequences and the human germline sequences were aligned and ranked in decreasing identity. **Figure 10** shows the PDB IDs of the query sequence, name of the retrieved germline gene segment, sequence id no, residues aligned, high score, P(N) sum, smallest probability, % identity with the query sequence, the germline family to which the identified germline sequence belongs to (vhaagrp-fl.aa stands for the f1 subfamily of VH chain; vkallaa-fl stands for f1 subfamily of VL kappa chain; vlallaa-f1.aa stands for f1 subfamily of VL lamda chain).

**Figure 11** shows the homology alignment for each of the selected human antibody germline sequences with the query sequence.

As shown in **Figures 10** and **11,** human antibody germline sequences with up to 100% homology with the query sequence can be found from the library of human antibody germline sequences. For example, 1DHA, 1DHW and 1DHV have the identical sequence as the germline sequence segment, while close germiline homologues can be found for other sequences corresponding to the target structural models. These are trivial cases because there is only one query sequence for each structural template.

4) Selecting the optimal remote homologous sepuence(s) of structure-based multiple sequence alignment by using profile Hidden Markov Model.

Given one clustered structure family, how to search for optimal sequence(s) that match with their aligned multiple sequence profile corresponding to their structure alignment? The flow chart in **Figure 12A** illustrates an indirect approach to search for remote homologues consistent with multiple sequence alignment from clustered structures. The clustered structures within a structure family can give multiple sequence alignment based on their 3D structures. These aligned sequences might come from different species; they may be close or remote sequence homologues. The multiple sequence alignment can be used, however, to build a profile Hidden Markov Model (HMM); and this HMM can then be used to select the close and/or remote human homologues from human sequence database such as the human germline and/or rearranged sequence database.

**Figure 12B** shows the result generated by using the method diagramed in **Figure 12A** based on a sequence profile of a structure cluster of the FR regions of 3 $V_H$ sequences. The structure cluster of the framework regions of 3 $V_H$ sequences, 1dha, 1dho and 1dhw, is shown in **Figure 4A.** Sequences of the FR regions of these 3 $V_H$ in the structure cluster were obtained by removing CDR1-3 from $V_H$, which are designated as FR123 **(Figure 12B).** FR123 sequences were used to build HMM and search human germline antibody sequences or humanized sequences. Fifty-two human germline antibody sequences (i.e., hits for FR123) were found. Variants in each position of the amino acid residues were profiled. Variants that occur less than 5 times in the position were filtered (i.e., cutoff value = 5) and discarded. The rest of the variants were combined combinatorially to produce a library of recombinant FR sequences. The hits for FR123 and/or the recombinant FR sequences can be scored based on their structurally compatibility with the structure cluster of the framework regions.

**Figure 12C** shows the result generated by using the method diagramed in **Figure 12A** based on a sequence profile of a structure cluster of the CDR1 of 4 kappa $V_L$ sequences. The structure cluster of the 4 kappa $V_L$ sequences, 1dgx, 1dh5, 1dh4, and 1dh6, is shown in **Figure 8B.** Sequences of CDR1 of these 4 kappa $V_L$ sequences in the structure cluster were used to build HMM and search Kabat database. The regions in these 4 kappa $V_L$ sequences showing a greater variability, i.e., the supervariable regions, are highlighted in red **(Figure 12C).** Numerous hits were found with diverse sequences and variable lengths. The hits were grouped according to their lengths. The group having the same length as one of the 4 kappa $V_L$ CDR1 sequences were compared and profiled based on variability in each amino acid residue. Such a variant profile was built for each of the 4 kappa $V_L$ CDR1 sequences, 1dgx, 1dh5, 1dh4, and 1dh6. To demonstrate, hits with lengths different than these 4 target sequences were also selected by using the inventive method, three artificial sequences, 1dh5a, 1dh5b, and 1dh5c, were constructed by inserting more residues into the supervariable region of 1dh5 and used as references to group these hits. As shown in the right portion of **Figure 12C**, hits with lengths different from the 4 "real" target sequences, 1dgx, 1dh5, 1dh4, and 1dh6, were also be found, variant profiles of which were shown underneath each of the 3 artificial sequences.

The variant profiles shown in **Figure 12C** reveal that there is a much higher variability in the supervariable region than the rest of the CDR1. The amino acid residues in the supervariable region may make a greater contribution to the specific and high affinity binding of the antibody to its antigen. This region can be specifically targeted to generate a more focused library of recombinant antibodies for structural and functional screening in silico, in vitro or in vivo.

As also shown in **Figure 12C**, the CDR variants with less than 5% of occurrence frequency were filtered and discarded. The rest of the variants were combined combinatorially to produce a library of recombinant CDR1 sequences. The hits for CDR1 and/or the recombinant CDR1 sequences can be scored based on their structurally compatibility with the structure cluster of the 4 kappa $V_L$ CDR1 sequences.

For the reclustered structure family in the Hucal model of Knappik et al., three of the $V_H$ structures are re-clustered into one family based on the structure criteria (superimposition and gaps), these three sequences should be used as the profiled sequences to build their HMM and then search the corresponding human germline sequence that is closest to all of them. **Figure 13 A-C** show the results of the search using this method. The identified human germline sequences (labeled as "Top Hits") can then be used to represent the corresponding structure in our diversity library for the target structures.

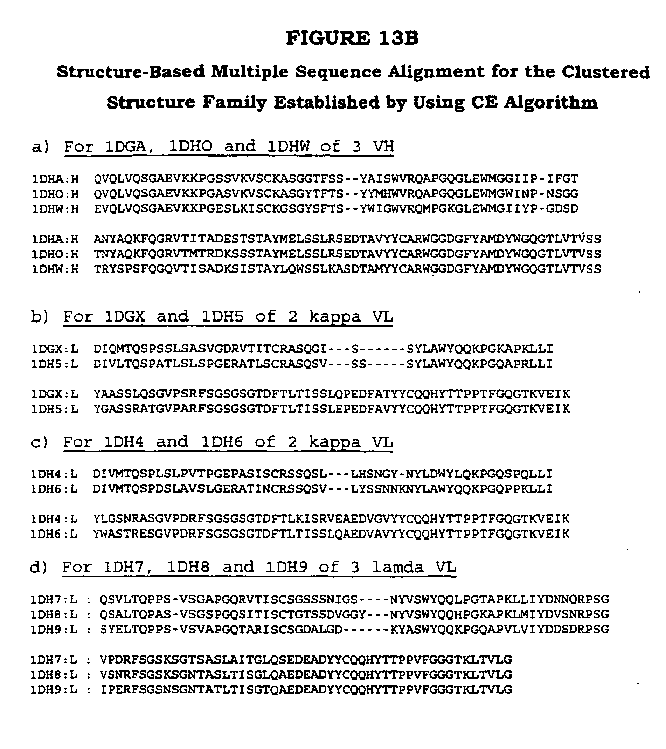

As shown in **Figure 13A**, the seven VHs of the Hucal models are clustered into 5 structure families: (1DGA, 1DHO, 1DHW), 1DHQ, 1DHU, 1DHV, and 1DHZ. The seven VLs of the Hucal models are clustered into 3 structure families: (1DGX, 1DH5) and (1DH4, 1DH6) for Kappa VL, and (1DH7, 1DH8, 1DH9) for Lamda VL. **Figure 13B** shows the alignment of the amino acid sequences based on the structures of the members within each structure family.

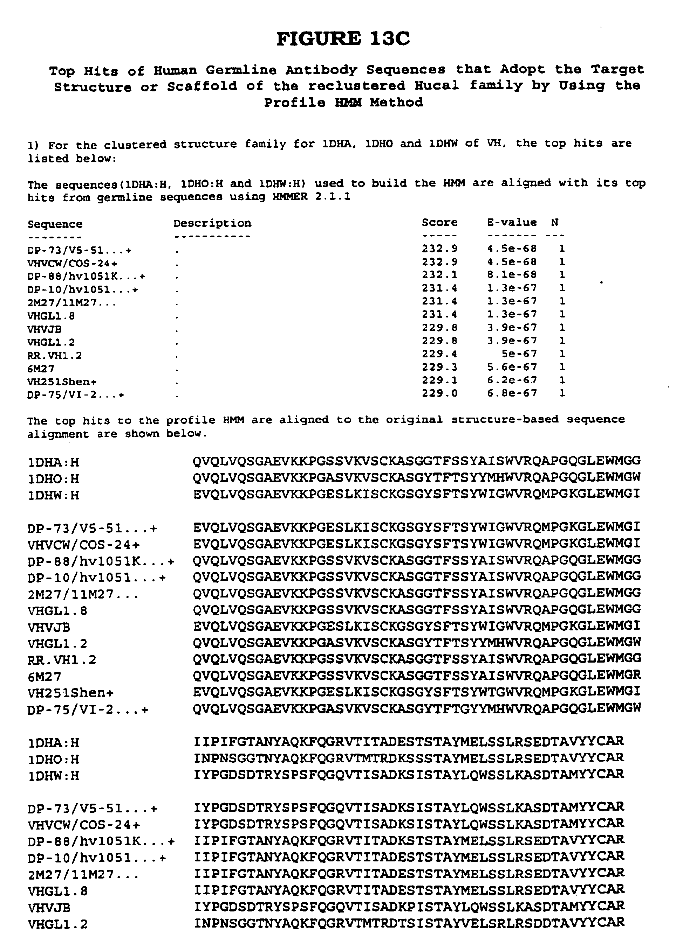

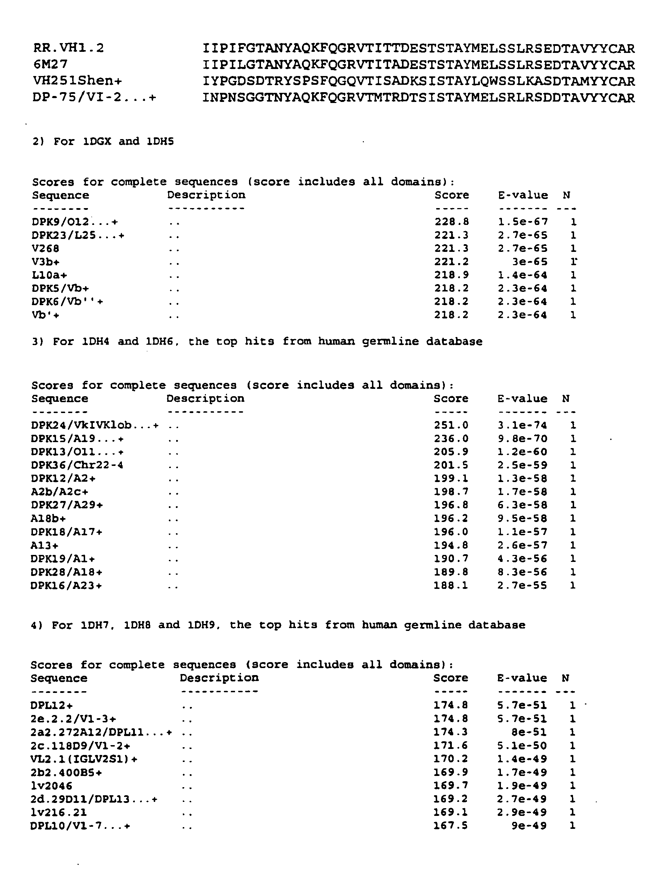

**Figure 13 C** lists the top hits of human germline antibodies identified by using the profile HMM method (HMMER2.1.1). The HMM has been calibrated; and E-values are empirical estimates. The top hits to the query sequence profile shows some important features which make it necessary to capture in order to make a comprehensive library for the clustered structure family of 1DHA, 1DHW and 1DHO. It is noted that 1DHW belongs to a different family of VH (f5 see **Figure 10**) where 1DHA and 1DHO belong to the same family of VH (f1 in **Figure 10**) based on the sequence homology classification. It is also apparent that comparison between hits and query sequence profile show that in some regions the sequence are highly conservative whereas in other regions sequence variability is large. The constant region should be good part for making master framework whereas the highly variable regions are some position for making sequence library.

It should be noted that the order of the top hits depends sensitively on the multiple sequence alignment derived from structure-based alignment. This demonstrates that the structure information is important for selecting the hit sequences. As shown in **Figure 13C**, some of the top hits are nontrivial from those obtained by BLAST.

5). <u>Matching the library of structural template with the library of sequence pools</u>

A powerful approach to compare the target structural template library with the sequence database is to match them in both directions. Using threading to find the optimal template for each sequence among the sequence database and then using reverse threading to match each template structure to sequence in the sequence database. The convergence of the both direction should give a reliable sequence to construct the sequence libraries for the desired target structures. This method can be also used in combination with other sequence searching method such as COBLATH that combines PSI-BLAST with Threading method.

**4. Examples of Structural Computational Engines**

Many programs are available for modeling structures or structural ensembles of antibodies. For example, a molecular mechanics software may be employed for these purposes, examples of which include, but are not limited to CONGEN, SCWRL, UHBD, and GENPOL.

CONGEN (**CON**formation **GEN**erator) is a program performing conformational searches on segments of proteins (R. E. Bruccoleri (1993) Molecular Simulations 10, 151-174 (1993); R. E. Bruccoleri, E. Haber, J. Novotny, (1988) Nature 335, 564-568 (1988); R. Bruccoleri, M. Karplus. (1987) Biopolymers 26, 137-168. It is most suited to problems where one needs to construct underdetermined loops or segments in a known structure, i.e. homology modeling. The program is a modification of CHARMM version 16, and has most of the capabilities of that version of CHARMM (Brooks BR, Bruccoleri BE, Olafson BD, States DJ, Swaminathan S, Karplus M. (1983) J. Comput. Chem. 4, 187-217). The energy functions of the total energy include bonds, angles, torsional angels, improper term, vdw and electrostatic interactions with distance dependent dielectric constant using Amber94 forcefield in CONGEN. It provides a simple yet fast way to scan sequence library for their compatibility with their template structure with decent correlation with the more refined scoring energy functions.

The CONGEN program is a modeling stratagem based on the theory that the lowest energy conformation should be close or correspond to the naturally occurring one. Bruccoleri and Karplus (1987) Biopolymers 26:137-168; and Bruccoleri and Novotny (1992) Immunomethods 96-106. Given an accurate Gibbs function and a short loop sequence, all of the stereochemically acceptable structures of the loop can be generated and their energies calculated. The one with the lower energy is selected.

The program can be used to perform both conformational searches and structural evaluation using standard scoring function. The program can calculate other properties of the molecules such as the solvent accessible surface area and conformational entropies, given steric constraints. Each one of these properties in combination with other properties described below can be used to score the digital libraries.

The defined canonical structures are available for five of the CDRs ($V_L$ CDR1, 2, and 3, and $V_H$ CDR1 and 2) except for $V_H$ CDR3. $V_H$ CDR3 is known to show large variation in its length and conformations, although progress has been made in modeling its conformation with increasing number of antibody structures becoming available in the PDB (protein data bank) database. CONGEN may be used to generate conformations of a loop region (e.g., $V_H$ CDR3) if no canonical structure is available, to replace the side chains of the template sequence with the corresponding side chain rotamers of the target amino acids. Third, the model will be further optimized by energy minimization or molecular dynamic simulation or other protocols to relieve the steric clash etc in the structure model.

SCWRL is a side chain placing program that can be used to generate side chain rotamers and combinations of rotamers using the backbone dependent rotamer library (Dunbrack RL Jr, Karplus M (1993) J Mol Biol 230:543-574). SCWRL is a program for adding sidechains to a protein backbone based on the backbone-dependent rotamer library (Bower, MJ, Cohen FE, Dunbrack RL (1997) J Mol Biol 267, 1268-1282). The library provides lists of chil-chi2-chi3-chi4 values and their relative probabilities for residues at given phi-psi values. The program can further explore these conformations to minimize sidechain-backbone clashes and sidechain-sidechain clashes. Once the steric clash is minimized, the side chains and the backbone of the substituted segment can be energy minimized to relieve local strain using CONGEN (Bruccoleri and Karplus (1987) Biopolymers 26:137-168). Each structure is scored using a custom energy function that measures the relative stability of the sequence in the lead structural template.

Several automatic programs that are developed specifically for building antibody structures may be used for structural modeling of antibody in the present invention. The ABGEN program is an automated antibody structure generation algorithm for obtaining structural models of antibody fragments. Mandal et al. (1996) Nature Biotech. 14:323-328. ABGEN utilizes a homology based scaffolding technique and includes the use of invariant and strictly conserved residues, structural motifs of known Fab, canonical features of hypervariable loops, torsional constraints for residue replacements and key inter-residue interactions. Specifically, the ABGEN algorithm consists of two principal modules, ABalign and ABbuild. ABalign is the program that provides the alignment of the antibody V-region sequence whose structure is desired with all the V-region sequences of antibodies whose structures are known and computes scores for the fitting. The highest scoring library sequence is considered to be the best fit to the test sequence. ABbuild then uses this best fit model output by Abalign to generate the three-dimensional structure and provides Cartesian coordinates for the desired antibody sequence.

WAM (Whitelegg NRJ and Rees, AR (2000) Protein Engineering 13, 819-824) is an improved version of ABM which is uses a combined algorithm by (Martin, ACR, Cheetham, JC, and Rees AR (1989) PNAS 86, 9268-9272) Rees etc- to model the CDR conformations using the canonical conformations of CDRs loops from x-ray PDB database and loop conformations generated using CONGEN (see reference by Rees 1995 (Ab antibody engineering). In short, the modular nature of antibody structure make it possible to model its structure using a combination of protein homology modeling and structure predictions.

In a preferred embodiment, the following procedure will be used to model antibody structure. Because antibody is one of the most conserved proteins in both sequence and structure, homology models of antibodies are relatively straightforward, except for certain CDR loops that are not yet determined within existing canonical structures or with insertion or deletions. These loop structures can be, however, modeled using a combined algorithms that combines homology modeling with conformational search (for example, CONGEN can be used for such purpose).

The defined canonical structures for five of the CDRs (L1,2,3 and VH1,2) except for H3 (i.e., $V_H$ CDR3) are used. $V_H$ H3 is known to show large variation in its length and conformations, although progress has been made in modeling its conformation with increasing number of antibody structures become available in the (protein data bank) PDB database using protein structure prediction methods, including threading and comparative modeling, which aligns the sequence of unknown structure with at least one known structure based on the similarity spanning modeled sequence. The de novo or ab initio methods also show increasing promising to predict the structure from sequence alone. The unknown loop conformations can be sampled using CONGEN if no canonical structure is available (Bruccoleri RE, Haber E, Novotny J (1988) Nature 355, 564-568). Alternatively, ab initio methods including but not limited to Rosetta ab initio method can be used to predict antibody CDR structures (Bonneau R, Tsai J, Ruczinski I, Chivian D, Rohl C, Strauss CE, Baker D (2001) Proteins Suppl 5, 119-126) without relying on similarity at the fold level between the modeled sequence and any of the known structures. The more accurate method that uses the state-of-the-art explicit solvent molecular dynamics and implicit solvent free energy calculations can be used to refine and select for native-like structures from models generated from either CONGEN or Rossetta *ab initio* method (Lee MR, Tsai J, Baker D, Kollman PA (2001) J Mol Biol 313, 417-430). The interactions between CDRs are first scored using the principles that determine the structure of β-sheet barrels in proteins.

## 5. Scoring functions for evaluating structural compatibility of tester sequence and structural template

In the implementation of the inventive methods described above, thermodynamic computational analysis can be used for evaluating structural compatibility of a tester sequence with a target structural template. The structural evaluation is based on an empirical and parameterized scoring function and is intended to reduce the number of subsequent *in vitro* screenings necessary. The scoring function consists of three energy terms: nonpolar solvation, sidechain entropy, and electrostatic energy (Sharp KA. (1998) Proteins 33, 39-48; Novotny J, Bruccoleri RE, Davis M, Sharp KA (1997) J Mol Biol 268, 401-411).

For energy functions, there are many that can be used to score compatibility of sequences with template structure or structure ensemble. The scoring function is composed of several terms including contribution from electrostatic and van der Waals interactions, $\Delta G_{MM}$ calculated using molecular mechanic forcefield, contribution from solvation including electrostatic solvation and solvent-accessible surface, $\Delta G_{sol}$, and contribution from the conformational entropy.

A simple fast way for computational screening is to calculate structural stability of a sequence using the total or combination of energy terms from molecular mechanic forcefield such as Amber94 implemented in CONGEN.

$$\Delta E_{total} = E_{vdw} + E_{bond} + E_{angel} + E_{electrostatics} + E_{solvation}$$

or alternatively, the binding free energy is calculated as

$$\Delta G_b = \Delta G_{MM} + \Delta G_{sol} (Ag\text{-}Ab) - \Delta G_{sol} (Ag) - \Delta G_{sol} (Ab) - T\Delta S$$

where:

$$\Delta G_{MM} = \Delta G_{ele} + \Delta G_{vdw} \ (1)$$

$$\Delta G_{sol} = \Delta G_{ele\text{-}sol} + \Delta G_{ASA} \ (2)$$

The $AG_{ele}$ and $\Delta G_{vdw}$ electrostatic and van der Waals interaction energy are calculated using Amber94 parameters implemented in CONGEN for $\Delta G_{MM}$, whereas the $AG_{ele\text{-}sol}$ is electrostatic solvation energy required to move a heterogeneously distributed charges from the gas phase into an aqueous phase. This is calculated by solving the Poisson-Boltzmann equation for the electrostatic potential for the reference and mutant structures. $\Delta G_{ASA}$, the nonpolar energy is the energetic cost of moving nonpolar solute groups into polar solvent, resulting in reorganization of the solvent molecules. This has been shown to correlate linearly with the solvent accessible surface area of the molecule (Sitkoff D, Sharp, KA, Honig B (1994) J Phys Chem 98, 1978-1988).

The change in sidechain entropy is a measure of the effect on the local sidechain conformational space particularly at the binding interface. This is calculated from the ratio of the number of allowed sidechain conformations in the reference and mutant structures, in the bound and unbound states. For general scoring purposes, the independent sidechain approximation is applied to the mutated sidechains in order to reduce computational time resulting from sampling the huge conformational space for individual side chains in various structural context.

## 6. Energy Functions

Many energy functions can be used to score the compatibility between sequences and structures. There are four kinds of energy functions can be used: (1) empirical physical chemistry-based forcefields based on simple model compounds such as standard molecular mechanic forcefields discussed below; (2) knowledge-basted statistical forcefields extracted from protein structures, the so called potential of mean force (PMF) or the threading score derived from the structure-based sequence profiling (3) parameterized forcefield by fitting the forcefield parameters using experimental model system; (4) combinations of one or several terms from (1) to (3) with various weighting factor for each term.

The following well-tested physical-chemistry forcefields can be used or incorporated into the scoring functions. For example, amber 94 fircefield was used in Congen to score the sequence-structure compatibility in the examples below. The forcefields include but are not limited to the following forcefields which are widely used for those skilled at the art. Amber 94 (Cornell, WD, Cieplak P, Bayly CI, Gould IR, Merz KM Jr, Ferguson DM, Spellmeyer DC, Fox T, Caldwell JW and Kollman PA. JACS (1995) 117, 5179-5197 (1995); Charmm forcefields (Brooks, B.R., Bruccoleri, R.E., Olafson, B.D., States, D.J., Swaminathan, S., Karplus, M. (1983) J. Comp. Chem. 4, 187-217.; MacKerell, A D ; Bashford, D; Bellott, M; Dunbrack, R L; Eva seck, J D; Field, M J; Fischer, S; Gao, J; Guo, H; Ha, S; JosephMcCarthy, D; Kuc nir, L; Kuczera, K; Lau, F T K; Mattos, C; Michnick, S; Ngo, T; Nguyen, D T; Pro hom, B; Reiher, W E; Roux, B; Schlenkrich, M; Smith, J C; Stote, R; Straub, J; W tanabe, M; WiorkiewiczKuczera, J; Yin, D; Karplus, M (1998) J. Phys. Chem., B 102, 3586-3617). The Discover cvff forcefields (Dauber-Osguthorpe, P.; Roberts, V. A.; Osguthorpe, D. J.; Wolff, J.; Genest, M.; Hagler, A. T. (1988) Proteins: Structure, Function and Genetics, 4, 31-47.) The ECEPP forcefields (Momany, F. A., McGuire, R. F., Burgess, A. W., & Scheraga, H. A., (1975) J. Phys. Chem. 79, 2361-2381.; Nemethy, G., Pottle, M. S., & Scheraga, H. A., (1983) J. Phys. Chem. 87, 1883-1887.). The GROMOS forcefields (Hermans, J., Berendsen, H. J. C., van Gunsteren, W. F., & Postma, J. P. M., (1984) Biopolymers 23, 1). The MMFF94 forcefields (Halgren, T. A. (1992) J. Am. Chem. Soc. 114, 7827-7843.; Halgren, T. A. (1996) J. Comp. Chem 17, 490-519.; Halgren, T. A. (1996) J. Comp. Chem. 17, 520-552.; Halgren, T. A. (1996) J. Comp. Chem. 17, 553-586.; Halgren, T. A., and Nachbar, R. B. (1996) J. Comp. Chem. 17, 587-615.; Halgren, T. A. (1996) J. Comp. Chem. 17, 616-641.). The OPLS forcefields (see Jorgensen, W. L., & Tirado-Rives, J.,(1988) J. Am. Chem. Soc. 110, 1657-1666.; Damm, W., A. Frontera, J. Tirado-Rives and W. L. Jorgensen (1997) J. Comp. Chem. 18, 1955-1970.). The Tripose forcefield (Clark, M., Cramer III, R. D., van Opdenhosch, N., (1989) Validation of the General Purpose Tripose 5.2 Force Field, J. Comp. Chem. 10, 982-1012.). The MM3 forcefield (Lii, J-H., & Allinger, N. L. (1991) J. Comp. Chem. 12, 186-199). Other generic forcefields such as Dreiding (Mayo SL, Olafson BD, Goddard (1990) J Phy Chem 94, 8897-8909) or specific forcefield used for protein folding or simulations like UNRES (United Residue Forcefield; Liwo et al., (1993) Protein Science 2, 1697-1714; Liwo et al., (1993) Protein Science 2, 1715-1731; Liwo et al., (1997) J. Comp. Chem. 18, 849-873; Liwo et al., (1997) J. Comp. Chem. 18:874-884; Liwo et al., (1998) J. Comp. Chem. 19:259-276.

The statistical forcefields derived from protein structures can be also used to assess the compatibility between sequences and protein structure. These potential include but not limited to residue pair potentials (Miyazawa S, Jernigan R (1985) Macromolecules 18, 534-552; Jernigan RL, Bahar, I (1996) Curr. Opin. Struc. Biol. 6, 195-209). The potentials of mean force (Hendlich et al., (1990) J. Mol. Biol. 216, 167-180) has been used to calculate the conformational ensembles of proteins (Sippl M (1990) J Mol Biol. 213, 859-883). However, some limitations of these forcefields are also discussed (Thomas PD, Dill KA (1996) J Mol Biol 257, 457-469; Ben-Naim A (1997) J Chem Phys 107, 3698-3706). Another methods to score the compatibility between sequences and structure is to use sequence profiling (Bowie JU, Luthy R, Eisenberg DA (1991) Science 253, 164-170) or threading scores (Jones DT, Taylor WR, Thornton JM (1992) Nature 358, 86-89; Bryant, SH, Lawrence, CE (1993) Proteins 16, 92-112; Rost B, Schneider R, Sander C (1997) J Mol Biol 270, 471-480; Xu Y, Xu D (2000) Proteins 40, 343-354). These statistical forcefields based on the quasichemical approximation or Boltzmann statistics or Bayes theorem (Simons KT, Kooperberg C, Huang E, Baker D (1997) J Mol Biol 268, 209-225) are evaluated to assess the goodness of the fit between a sequence and a structure or for protein design (Dima RI, Banavar J R, Maritan A (2000) Protein Science 9, 812-819).

The structure-based thermodynamic or parameters related to formation of the secondary structures of proteins can be also used to evaluate the fitness between a sequence and a structure. In the structure-based thermodynamic methods, the thermodynamic quantities such as heat capacity, enthalpy, entropy can be calculated based on the structure of a protein to explain the temperature-dependence of the thermal unfolding using the thermodynamic data from model compounds or protein calorimetry studies (Spolar RS, Livingstone JR, Record MT (1992) Biochemistry 31, 3947-3955; Spolar RS, Record MT (1994) Science 263, 777-784; Murphy KP, Freire E (1992) Adv Protein Chem 43, 313-361; Privalov PL, Makhatadze GI (1993) J Mol Biol 232, 660-679; Makhatadze GI, Privalov PL (1993) J Mol Biol 232, 639-659). The structure-based thermodynamic parameters can be used to calculate structural stability of mutant sequences and hydrogen exchange protection factors using ensemble-based statistical thermodynamic approach (Hilser VJ, Dowdy D, Oas TG, Freire E (1998) PNAS 95, 9903-9908). Thermodynamc parameters relating to statistical thermodynamic models of the formation of the protein secondary structures have been also determined using experimental model systems with excellent agreement between predictions and experimental data (Rohl CA, Baldwin RL (1998) Methods Enzymol 295, 1-26; Serrano L (2000) Adv Protein Chem 53, 49-85).

A combination of various terms from molecular mechanic forcefields plus some specific components has been used in most protein design programs. In a preferred embodiment, the forcefield is composed of one or several some terms such as the van der Waals, hydrogen bonding and electrostatic interactions from the standard molecular mechanics forcefields such as Amber, Charmm, OPLS, cvff, ECEPP, plus one or several terms that are believed to control the stability of proteins.

## 7. Examples of forcefields for protein design

It is understood that as a general solution to protein design problem, the energy surface describing the interactions among all elements of the system are sampled as a function of its atomic coordinates over all available sequences and their conformational space. Such a procedure may be implemented in following steps: i) providing a target scaffold with the backbone structure, e.g., a X-ray crystal structure retrieved from protein databank (PDB) or a structural model built by modeling; ii) building side chain models of amino acid variants onto a selected backbone by using a rotamer library derived from a protein structure database; iii) assigning forcefield parameters such as charge, radii, etc. to each atom to construct the target function; and iv) searching the energy surface of the target function using deterministic and/or stochastic algorithms to find optimal solution or solutions ranked in their scores.

Each individual protein design method is distinguished mainly from each other in terms of the forcefield and sampling algorithm. However, scoring functions and sampling algorithms in these protein design methods may optionally be used for a structure-based evaluation of the sequences from the hit and/or hit variant library.

For example, as an important interaction for scoring the correct packing interactions inside the core of proteins, van der Waals (vdw) interaction was used to design the protein core sequences by testing allowed rotamer sequences in enumeration (Ponder JW, Richards FM (1987) J Mol Biol 193, 775-791. A group of sequences can be selected under a potential function using simulated evolution with stochastic algorithm; the ranking order of the energies of selected sequences for residues in the hydrophobic cores of proteins correlates well with their biological activities (Hellinga HW, Richards FM (1994) PNAS 91, 5803-5807).

Similar approaches were also used to design proteins using stochastic algorithm (Desjarlais J, Handel T, (1995) Protein Science 4, 2006-2018; Kono H, Doi J (1994) Proteins, 19, 244-255). Effect of potential function on the designed sequences of a target scaffold has been evaluated by including van der Waals, electrostatics, and surface-dependent semiempirical environmental free energy or combinations of terms in an automatic protein design method that keeps the composition of amino acid sequence unchanged. It was shown that each additional term of the energy function increases progressively the performance of the designed sequences with vdw for packing, electrostatics for folding specificity and environmental solvation term for burial of the hydrophobic residues and for exposure of the hydrophilic residue (Koehl P, Levitt M (1999) J Mol Biol 293, 1161-1181).

The self-consistent mean field approach was used to sample the energy surface in order to find the optimal solution, (Delarue M, Koehl. (1997) Pac. Symp. Biocomput. 109-121; Koehl P, Delarue M, (1994) J. Mol. Biol. 239, 249-275; Koehl P, Delarue M (1995) Nat. Struct. Biol. 2,163-170; Koehl P, Delarue M (1996) Curr. Opin. Struct. Biol. 6: 222-226; Lee J. (1994) Mol. Biol. 236, 918-939; Vasquez (1995) Biopolymers 36, 53-70). Combination of terms from Molecular Mechanics or MM forcefield, knowledge-based statistical forcefield and other empirical correction has been also used to design protein sequences that are close to the native sequence of the target scaffold (Kuhlman B, Baker D (2000) PNAS 97, 10383-10388). The structure-based thermodynamic terms were included in addition to the steric repulsion in the protein core design (Jiang X, Farid H, Pistor E, Farid RS (2000) Protein Science 9, 403-416). Knowledge-based potentials have been used to design proteins (Rossi A, Micheletti C, Seno F, Maritan A (2001) Biophysical Journal 80, 480-490).

Forcefields have been also optimized specifically for protein design purpose. The energy function is decomposed into pairwise functional forms that combine molecular mechanic energy terms with specific solvation term is used for residues at the core, boundary and surface positions; dead end elimination algorithm is used to sip through huge number of combinatorial rotameric sequences (Dahiyat BI, Mayo SL (1996) Protein Science 5, 895-903). The stringency of force fields and rigid inverse folding protocol with fix backbone used in protein design has inevitably resulted a significant rate of false negative: rejection of many sequences that might be acceptable if soft energy function or flexible backbone is allowed. Moreover, the energy function used for protein design is so different from forcefields such as Amber or Charmm that are widely used and tested for studying protein folding or stability (Gordon DB, Marshall SA, Mayo SL (1999) Curr Opion Stru Biol 9, 509-513). Cautions should be excised to compare the sequences designed using specific protocol with others from alternative methods because a direct comparison among them may not be possible due to the false negative issues involved in protein design protocols.

The inventor believes that although a high false negative rate in protein design is not a problem for designing proteins with no restriction, this will pose serious problem for designing proteins for pharmaceutical application because only small restrictive region is allowed to have altered sequences to improve protein functions such as the CDRs in antibodies and a few positions in the framework regions. Therefore, it is accuracy rather than the speed of computational screening that matters the most for functional improvement in order to identify those few mutants in the targeted region.

These methods can be used to generate structure ensembles by molecular dynamics calculations or computational methods of proteins in the native or unfolded states which provide more accurate methods to score sequence and its variants based on the ensemble averages of the energy functions (Kollman PA, Massova I, Reyes C, Kuhn B, Huo SH, Chong LT, Lee M, Lee TS, Duan Y, Wang W, Donini O, Cieplak P, Srinivasan P, Case DA, and Cheatham TE (2000) Acc. Chem Res. 33, 889-897). The ensemble averages calculated from ensemble structures show better correlation with corresponding data from experimental measurement.

In a particular embodiment, standard terms from MM terms have been combined with the solvation terms including electrostatic solvation and solvent-accessible solvation term calculated with continuous solvent model for electrostatic solvation; these MM-PBSA or MM-GBSA method, together with contribution from the conformational entropy including backbone and side chains, have shown good correlation between experimental and calculated values in the free energy change (Wang W, Kollman P (2001) JMB). Compared to other scoring functions used in protein and drug design, MM-PBSA or MM-GBSA is better physical model for scoring and would handle various problems on an uniform basis, although it is computational expensive because multiple trajectories from molecular dynamic simulation in explicit water is required to calculate the ensemble averages for the system.

**Claims**

1. A method for constructing a library of recombinant antibodies, based on a target structural template of a region of one or more antibodies comprising the steps of:

   (a) clustering variable regions of a collection of antibodies wherein each antibody has a known 3D structure into families of structural ensembles, each family of structural ensembles comprising at least two different antibody sequences, the antibody sequences in a family of structural ensembles having variation in sequence and/or length but having substantially identical main chain conformations, wherein the variable regions are clustered based on cut off values of the root mean square difference of the a carbon atoms of the main chain conformations of the antibody sequences of less than 2Å and/or statistical significance having a Z-score of more than 4;
   (b) selecting a structural template from each family of structural ensembles, wherein the structural template is a member of the structural ensemble representative of the average of the characteristics of all other members within the family;
   (c) profiling a plurality of tester polypeptide sequences onto the structural template within each family of structural ensembles;
   (d) determining if each of the selected tester peptide sequences is structurally compatible with the structural template using an energy-based scoring function; and
   (e) constructing an *in vitro* library of recombinant antibodies based on these structurally compatible selected peptides by introducing DNA segments encoding the selected tester polypeptides into cells of a host organism; expressing the DNA segments in the host cells such that recombinant antibodies containing the selected polypeptide sequences are produced in the cells of the host organism; and selecting recombinant antibodies that binds to a target antigen with affinity higher than $10^6$ M$^{-1}$.

2. The method of claim 1, wherein the collection of antibodies include antibodies or immunoglobulins collected in a protein database.

3. The method of claim 2, wherein the protein database is selected from the group consisting of the protein data bank of Brookhaven National Laboratory, Genbank at the National Institute of Health, Swiss-PROT protein sequence database and the Kabat database for CDRs of antibodies.

4. The method of claim 1, wherein the collection of antibodies having known 3D structures include antibodies having resolved X-ray crystal structures, NMR structures or 3D structures based on structural modeling.

5. The method of claim 1, wherein the variable regions of the collection of antibodies are the full length heavy chain or light chain variable regions or specific portions of the heavy chain or light chain variable region selected from the group consisting of CDR, FR, and a combination thereof.

6. The method of claim 5, wherein the CDR is CDR1, CDR2, or CDR3 of an antibody.

7. The method of claim 5, wherein the FR is FR1, FR2, FR3, or FR4 of an antibody.

8. The method of claim 1, wherein the clustering step is implemented by an algorithm selected from the group consisting of CE, Monte Carlo and 3D clustering algorithms.

9. The method of claim 1, wherein the profiling step includes reverse threading the tester polypeptide sequence onto the representative structural template within each family of structural ensemble.

10. The method of claim 1, wherein the profiling step is implemented by a multiple sequence alignment algorithm.

11. The method of claim 10, wherein the multiple sequence alignment algorithm is profile HMM algorithm or PSI-BLAST.

12. The method of claim 1, wherein the structural template is adopted by a CDR region, and the profiling step includes profiling the tester polypeptide sequence that is a variable region of a human or non-human antibody onto the representative structural template within each family of structural ensemble.

13. The method of claim 1, wherein the structural template is adopted by a FR region, and the profiling step includes profiling the tester polypeptide sequence that is a variable region of a human antibody onto the representative structural template within each family of structural ensemble.

14. The method of claim 13, wherein the tester polypeptide sequence is a variable region of human germline antibody sequence.

15. The method of claim 1, wherein the tester polypeptide sequence is the sequence or a segment sequence of an expressed protein.

16. The method of claim 1, wherein the tester polypeptide sequence is a region of an antibody.

17. The method of claim 16, wherein the antibody is a human antibody.

18. The method of claim 1, wherein the tester polypeptide sequence is a region of a human germline antibody sequence.

19. The method of claim 1, wherein the energy scoring function is selected from the group consisting of electrostatic interactions, van der Waals interactions, electrostatic solvation energy, solvent- accessible surface solvation energy, and conformational entropy.

20. The method of claim 1, wherein the scoring function incorporates a forcefield selected from the group consisting of the Amber forcefield, Charmm forcefield, the Discover cvff forcefields, the ECEPP forcefields, the GROMOS forcefields, the OPLS forcefields, the MMFF94 forcefield, the Tripose forcefield, the MM3 forcefield, the Dreiding forcefield, and UNRES forcefield, and other knowledge-based statistical forcefield (mean field) and structure-based thermodynamic potential functions.

21. The method of claim 1, wherein the step of selecting the tester peptide sequences includes selecting sequences that have a lower or equal total energy than that of the representative sequence calculated based on a formula of

$$\Delta E_{total} = E_{vdw} + E_{bond} + E_{angle} + E_{electrostatics} + E_{solvation}$$

Where in $\Delta E_{total}$ is total energy; $E_{vdw}$ is energy of van der Waals interactions; $E_{bond}$ is bond energy; $E_{angle}$ is bond angle energy; $E_{electrostatics}$ is electrostatic energy; $E_{solvation}$ is solvent-accessible surface salvation energy calculated for the peptide or peptide segment.

22. The method of claim 1, wherein the step of selecting the tester peptide sequences includes selecting sequences that have a lower binding free energy than that of the representative sequence calculated as the difference between the bound and unbound states using a refined scoring function

$$\Delta G^b = \Delta G^{MM} + G^{sol} - T\Delta S$$

where

$$\Delta G_{MM} = \Delta G_{ele} + \Delta G_{vdw} \qquad (1)$$

$$\Delta G_{sol} = \Delta G_{ele\text{-}sol} + \Delta G_{ASA} \qquad (2)$$

23. The method of claim 1, further comprising the steps of: building an amino acid positional variant profile of the selected tester polypeptide sequences; filtering out the variants with occurrence frequency lower than 3; and combining the variants remained to produce a combinatorial library of antibody sequences.

24. The method of claim 23, wherein the filtering step includes filtering out the variants with occurrence frequency lower than 5.

25. The method of claim 1, wherein the recombinant antibody is a fully assembled antibody, a Fab fragment, an Fv fragment, or a single chain antibody.

26. The method of claim 1, wherein the host organism is selected from the group consisting of bacteria, yeast, plant, insect, and mammal.

27. The method of claim 1, wherein the target antigen is a small molecule, proteins, peptide, nucleic acid or polycarbohydate.

28. The method of claim 1, further comprising the steps of: building an amino acid 1 positional variant profile of the selected tester polypeptide sequences; filtering out the variants with occurrence frequency lower than 3; and combining the variants remained to produce a combinatorial library of antibody sequences.

29. The method of claim 28, wherein the filtering step includes filtering out the variants with occurrence frequency lower than 5.

**Patentansprüche**

1. Verfahren zum Erstellen einer Bibliothek von rekombinanten Antikörpern auf der Basis eines Ziel-Strukturtemplats eines Bereichs eines oder mehrerer Antikörper, umfassend die folgenden Schritte:

(a) Clustering von variablen Bereichen einer Sammlung von Antikörpern, wobei jeder Antikörper eine bekannte 3D-Struktur aufweist, zu Familien von Strukturensembles, wobei jede Familie von Strukturensembles wenigstens zwei verschiedene Antikörpersequenzen umfasst, wobei die Antikörpersequenzen in einer Familie von Strukturensembles Variationen von Sequenz und/oder Länge, aber im Wesentlichen identische Hauptkettenkonformationen aufweisen, wobei die variablen Bereiche auf der Basis von Schwellenwerten der Wurzel des mittleren quadratischen Unterschieds ("root mean square difference") der α-Kohlenstoffatome der Hauptkettenkonformationen der Antikörpersequenzen von weniger als 2 Å und/oder einer statistischen Signifikanz mit einem Z-Wert von mehr als 4 geclustert werden;
(b) Auswählen eines Strukturtemplats aus jeder Familie von Strukturensembles, wobei das Strukturtemplat ein Mitglied des Strukturensembles ist, das für das Mittel der Merkmale aller anderen Mitglieder der Familie repräsentativ ist;
(c) Profiling einer Vielzahl von Tester-Polypeptidsequenzen auf das Strukturtemplat innerhalb jeder Familie von Strukturensembles;
(d) unter Verwendung einer energiebasierten Scoring-Funktion Bestimmen, ob jede der ausgewählten Tester-Peptidsequenzen mit dem Strukturtemplat strukturell kompatibel ist; und

(e) Erstellen einer in-vitro-Bibliothek von rekombinanten Antikörpern auf der Basis dieser strukturell kompatiblen, ausgewählten Peptide durch Einführen von DNA-Segmenten, die die ausgewählten Tester-Polypeptide codieren, in Zellen eines Wirtsorganismus; Exprimieren der DNA-Segmente in den Wirtszellen, so dass rekombinante Antikörper, die die ausgewählten Polypeptidsequenzen enthalten, in den Zellen des Wirtsorganismus erzeugt werden; und Auswählen von rekombinanten Antikörpern, die mit einer Affinität von höher als $10^6$ $M^{-1}$ an ein Ziel-Antigen binden.

**2.** Verfahren gemäß Anspruch 1, wobei die Sammlung von Antikörpern Antikörper oder Immunglobuline umfasst, die in einer Protein-Datenbank gesammelt sind.

**3.** Verfahren gemäß Anspruch 2, wobei die Proteindatenbank ausgewählt ist aus der Gruppe bestehend aus der Protein Data Bank des Brookhaven National Laboratory, der Genbank am National Institute of Health, der Swiss-PROT Proteinsequenz-Datenbank und der Kabat-Datenbank für CDRs von Antikörpern.

**4.** Verfahren gemäß Anspruch 1, wobei die Sammlung von Antikörpern mit bekannter 3D-Struktur Antikörper mit aufgelösten Röntgen-Kristallstrukturen, NMR-Strukturen oder 3D-Strukturen auf der Basis von Strukturmodellen umfasst.

**5.** Verfahren gemäß Anspruch 1, wobei die variablen Bereiche der Sammlung von Antikörpern die volle Länge der variablen Bereiche der schweren Kette oder der leichten Kette oder spezifische Teile des variablen Bereichs der schweren Kette oder der leichten Kette, ausgewählt aus der Gruppe bestehend aus CDR, FR oder einer Kombination davon, sind.

**6.** Verfahren gemäß Anspruch 5, wobei die CDR CDR1, CDR2 oder CDR3 eines Antikörpers ist.

**7.** Verfahren gemäß Anspruch 5, wobei die FR FR1, FR2, FR3 oder FR4 eines Antikörpers ist.

**8.** Verfahren gemäß Anspruch 1, wobei der Schritt des Clusterings durch einen Algorithmus, ausgewählt aus der Gruppe bestehend aus CE-, Monte-Carlo- und 3D-Clustering-Algorithmen, implementiert ist.

**9.** Verfahren gemäß Anspruch 1, wobei der Schritt des Profilings das Reverse Threading der Tester-Polypeptidsequenz auf das repräsentative Strukturtemplat innerhalb jeder Familie von Strukturensembles umfasst.

**10.** Verfahren gemäß Anspruch 1, wobei der Schritt des Profilings durch einen Mehrsequenzalignment-Algorithmus implementiert ist.

**11.** Verfahren gemäß Anspruch 10, wobei der Mehrsequenzalignment-Algorithmus der Profil-HMM-Algorithmus oder PSI-BLAST ist.

**12.** Verfahren gemäß Anspruch 1, wobei das Strukturtemplat von einem CDR-Bereich angenommen wird und der Schritt des Profilings das Profiling der Tester-Polypeptidsequenz, die ein variabler Bereich eines humanen oder nicht-humanen Antikörpers ist, auf das repräsentative Strukturtemplat innerhalb jeder Familie von Strukturensembles umfasst.

**13.** Verfahren gemäß Anspruch 1, wobei das Strukturtemplat von einem FR-Bereich angenommen wird und der Schritt des Profilings das Profiling der Tester-Polypeptidsequenz, die ein variabler Bereich eines humanen oder nicht-humanen Antikörpers ist, auf das repräsentative Strukturtemplat innerhalb jeder Familie von Strukturensembles umfasst.

**14.** Verfahren gemäß Anspruch 13, wobei die Tester-Polypeptidsequenz ein variabler Bereich der humanen Keimbahn-Antikörper-Sequenz ist.

**15.** Verfahren gemäß Anspruch 1, wobei die Tester-Polypeptidsequenz die Sequenz oder eine Segmentsequenz eines exprimierten Proteins ist.

**16.** Verfahren gemäß Anspruch 1, wobei die Tester-Polypeptidsequenz ein Bereich eines Antikörpers ist.

**17.** Verfahren gemäß Anspruch 16, wobei der Antikörper ein humaner Antikörper ist.

**18.** Verfahren gemäß Anspruch 1, wobei die Tester-Polypeptidsequenz ein Bereich einer humanen Keimbahn-Antikörper-Sequenz ist.

**19.** Verfahren gemäß Anspruch 1, wobei die Energie-Scoring-Funktion ausgewählt ist aus der Gruppe bestehend aus elektrostatischen Wechselwirkungen, van-der-Waals-Wechselwirkungen, elektrostatischer Solvatisierungsenergie, Solvatisierungsenergie der lösungsmittelzugänglichen Oberfläche und Konformationsentropie.

**20.** Verfahren gemäß Anspruch 1, wobei die Scoring-Funktion ein Kraftfeld umfasst, ausgewählt aus der Gruppe bestehend aus dem Amber-Kraftfeld, dem Charmm-Kraftfeld, den Discover-cvff-Kraftfeldern, den ECEPP-Kraftfeldern, den GROMOS-Kraftfeldern, den OPLS-Kraftfeldern, dem MMFF94-Kraftfeld, dem Tripose-Kraftfeld, dem MM3-Kraftfeld, dem Dreiding-Kraftfeld und dem UNRES-Kraftfeld sowie einem anderen wissensbasierten statistischen Kraftfeld (mittleres Feld) und strukturbasierten thermodynamischen Potentialfunktionen.

**21.** Verfahren gemäß Anspruch 1, wobei der Schritt des Auswählens der Tester-Peptidsequenzen das Auswählen von Sequenzen umfasst, die eine kleinere oder die gleiche Gesamtenergie als bzw. wie jene der repräsentativen Sequenz aufweisen, berechnet auf der Basis der Formel

$$\Delta E_{gesamt} = E_{vdW} + E_{Bindung} + E_{Winkel} + E_{Elektrostatik} + E_{Solvatisierung}$$

wobei $\Delta E_{gesamt}$ die Gesamtenergie ist; $E_{vdw}$ die Energie der van-der-Waals-Wechselwirkungen ist; $E_{Bindung}$ die Bindungsenergie ist; $E_{Winkel}$ die Bindungswinkel-Energie ist; $E_{Elektrostatik}$ die elektrostatische Energie ist; $E_{Solvatisierung}$ die Solvatisierungsenergie der lösungsmittelzugänglichen Oberfläche ist, berechnet für das Peptid oder Peptidsegment.

**22.** Verfahren gemäß Anspruch 1, wobei der Schritt des Auswählens der Tester-Peptidsequenzen das Auswählen von Sequenzen umfasst, die eine kleinere freie Bindungsenergie als die repräsentative Sequenz aufweisen, berechnet als der Unterschied zwischen den gebundenen und nichtgebundenen Zuständen unter Verwendung einer verfeinerten Scoring-Funktion

$$\Delta G^h = \Delta G^{MM} + G^{sol} - T\Delta S_{SS}$$

wobei

$$\Delta G_{MM} = \Delta G_{ele} {}^+ \Delta G_{vdW} \qquad (1)$$

$$\Delta G_{sol} = \Delta G_{ele-sol} + \Delta G_{ASA} \qquad (2).$$

**23.** Verfahren gemäß Anspruch 1, ferner umfassend die folgenden Schritte: Erstellen eines Aminosäure-Positionsvariations-Profils der ausgewählten Tester-Polypeptidsequenzen; Ausfiltern der Varianten mit einer Häufigkeit des Auftretens kleiner als 3; und Kombinieren der verbleibenden Varianten, um eine kombinatorische Bibliothek von Antikörpersequenzen zu erstellen.

**24.** Verfahren gemäß Anspruch 23, wobei der Schritt des Filterns das Ausfiltern der Varianten mit einer Häufigkeit des Auftretens kleiner als 5 umfasst.

**25.** Verfahren gemäß Anspruch 1, wobei der rekombinante Antikörper ein vollständig aufgebauter Antikörper, ein Fab-Fragment, ein Fv-Fragment oder ein einzelkettiger Antikörper ist.

**26.** Verfahren gemäß Anspruch 1, wobei der Wirtsorganismus ausgewählt ist aus der Gruppe bestehend aus Bakterium, Hefe, Pflanze, Insekt und Säuger.

**27.** Verfahren gemäß Anspruch 1, wobei das Ziel-Antigen ein kleines Molekül, Proteine, ein Peptid, eine Nucleinsäure oder ein Polykohlenhydrat ist.

**28.** Verfahren gemäß Anspruch 1, ferner umfassend die folgenden Schritte: Erstellen eines Aminosäure-Positionsvariations-Profils der ausgewählten Tester-Polypeptidsequenzen; Ausfiltern der Varianten mit einer Häufigkeit des Auftretens kleiner als 3; und Kombinieren der verbleibenden Varianten, um eine kombinatorische Bibliothek von Antikörpersequenzen zu erstellen.

**29.** Verfahren gemäß Anspruch 28, wobei der Schritt des Filterns das Ausfiltern der Varianten mit einer Häufigkeit des Auftretens kleiner als 5 umfasst.

**Revendications**

**1.** Procédé pour la construction d'une bibliothèque d'anticorps recombinants, basé sur un motif structurel cible d'une région d'un ou de plusieurs anticorps comprenant les étapes de :

    (a) regroupement de régions variables d'une collection d'anticorps, chaque anticorps présentant une structure 3D connue, dans des familles d'ensembles structurels, chaque famille d'ensembles structurels comprenant au moins deux séquences d'anticorps différentes, les séquences d'anticorps dans une famille d'ensembles structurels présentant une variation de séquence et/ou de longueur mais présentant des conformations de chaîne principale substantiellement identiques, les régions variables étant regroupées sur base de valeurs limites de la différence de valeur moyenne quadratique des atomes de carbone $\alpha$ des conformations de la chaîne principale des séquences d'anticorps de moins de 2 Å et/ou d'une signification statistique présentant un score Z de plus de 4 ;
    (b) sélection d'un motif structurel à partir de chaque famille d'ensembles structurels, le motif structurel étant un élément de l'ensemble structurel représentatif de la moyenne des caractéristiques de tous les autres éléments dans la famille ;
    (c) profilage d'une pluralité de séquences de polypeptide de test sur le motif structurel dans chaque famille d'ensembles structurels ;
    (d) détermination de si chacune des séquences de peptide de test sélectionnées est structurellement compatible avec le motif structurel en utilisant une fonction de score basée sur l'énergie ; et
    (e) construction d'une bibliothèque in vitro d'anticorps recombinants basée sur ces peptides sélectionnés structurellement compatibles en introduisant des segments d'ADN codant pour les polypeptides de test sélectionnés dans des cellules d'un organisme hôte ; expression des segments d'ADN dans les cellules hôtes de manière telle que les anticorps recombinants contenant les séquences de polypeptide sélectionnées sont produits dans les cellules de l'organisme hôte ; et sélection des anticorps recombinants qui se lient à un antigène cible avec une affinité supérieure à $10^6$ M$^{-1}$.

**2.** Procédé selon la revendication 1, la collection d'anticorps incluant des anticorps ou des immunoglobulines recueillis dans une base de données de protéines.

**3.** Procédé selon la revendication 2, la base de données de protéines étant sélectionnée dans le groupe constitué par la banque de données de protéines du Brookhaven National Laboratory, la Genbank du National Institute of Health, la base de données de séquences de protéines Swiss-PROT et la base de données Kabat pour les CDR (Complementary Determining Region - région déterminant la complémentarité) d'anticorps.

**4.** Procédé selon la revendication 1, la collection d'anticorps présentant des structures 3D connues incluant des anticorps présentant des structures cristallines résolues aux rayons X, par RMN ou 3D basées sur la modélisation structurelle.

**5.** Procédé selon la revendication 1, les régions variables de la collection d'anticorps étant les régions variables de la chaîne lourde ou de la chaîne légère, de longueurs entières, ou des parties spécifiques de la région variable de la chaîne lourde ou de la chaîne légère sélectionnées dans le groupe constitué par la CDR, la FR (framework - charpente) et une combinaison de celles-ci.

**6.** Procédé selon la revendication 5, la CDR étant la CDR1, la CDR2 ou la CDR3 d'un anticorps.

**7.** Procédé selon la revendication 5, la FR étant la FR1, la FR2, la FR3 ou la FR4 d'un anticorps.

**8.** Procédé selon la revendication 1, l'étape de regroupement étant implémentée par un algorithme sélectionné dans le groupe constitué par les algorithmes de regroupement CE, Monte Carlo et 3D.

**9.** Procédé selon la revendication 1, l'étape de profilage incluant l'enfilage inverse de la séquence de polypeptide de test sur le motif structurel représentatif dans chaque famille d'ensembles structurels.

**10.** Procédé selon la revendication 1, l'étape de profilage étant implémentée par un algorithme d'alignement de séquences multiples.

**11.** Procédé selon la revendication 10, l'algorithme d'alignement de séquences multiples étant un algorithme HMM de profil ou PSI-BLAST.

**12.** Procédé selon la revendication 1, le motif structurel étant adopté par une région CDR et l'étape de profilage incluant le profilage de la séquence de polypeptide de test qui est une région variable d'un anticorps humain ou non humain sur le motif structurel représentatif dans chaque famille d'ensembles structurels.

**13.** Procédé selon la revendication 1, le motif structurel étant adopté par une région FR et l'étape de profilage incluant le profilage de la séquence de polypeptide de test qui est une région variable d'un anticorps humain sur le motif structurel représentatif dans chaque famille d'ensembles structurels.

**14.** Procédé selon la revendication 13, la séquence de polypeptide de test étant une région variable de séquence d'anticorps de lignée germinale humaine.

**15.** Procédé selon la revendication 1, la séquence de polypeptide de test étant la séquence ou une séquence de segment d'une protéine exprimée.

**16.** Procédé selon la revendication 1, la séquence de polypeptide de test étant une région d'un anticorps.

**17.** Procédé selon la revendication 16, l'anticorps étant un anticorps humain.

**18.** Procédé selon la revendication 1, la séquence de polypeptide de test étant une région d'une séquence d'anticorps de lignée germinale humaine.

**19.** Procédé selon la revendication 1, la fonction de score énergétique étant sélectionnée dans le groupe constitué par les interactions électrostatiques, les interactions de van der Waals, l'énergie de solvatation électrostatique, l'énergie de solvatation de surface accessible au solvant et l'entropie conformationnelle.

**20.** Procédé selon la revendication 1, la fonction de score incorporant un champ de force sélectionné dans le groupe constitué par le champ de force d'Amber, le champ de force de Charmm, les champs de force Discover CVFF, les champs de force ECEPP, les champs de force GROMOS, les champs de force OPLS, le champ de force MMFF94, le champ de force Tripose, le champ de force MM3, le champ de force Dreiding et le champ de force UNRES et d'autres fonctions de champs de force statistiques basés sur la connaissance (champ moyen) et fonctions potentielles thermodynamiques basées sur la structure.

**21.** Procédé selon la revendication 1, l'étape de sélection de séquences de peptide de test incluant la sélection des séquences qui présentent une énergie totale inférieure ou égale à celle de la séquence représentative calculée sur base d'une formule $\Delta E_{totale} = E_{vdw} + E_{liaison} + E_{angle} + E_{électrostatique} + E_{solvatation}$ $AE_{totale}$ représentant l'énergie totale ; $E_{vdw}$ représentant l'énergie des interactions de van der Waals ; $E_{liaison}$ représentant l'énergie de liaison ; $E_{angle}$ représentant l'énergie d'angle de liaison ; $E_{électrostatique}$ représentant l'énergie électrostatique; $E_{solvatation}$ représentant l'énergie de solvatation de surface accessible au solvant calculée pour le peptide ou un segment du peptide.

**22.** Procédé selon la revendication 1, l'étape de sélection des séquences de peptide de test incluant la sélection de séquences qui présentent une énergie libre de liaison inférieure à celle de la séquence représentative calculée comme la différence entre les états liés et non liés en utilisant une fonction de score affinée

$$\Delta G^h = \Delta G^{MM} + G^{sol} - T\Delta S_{ss}$$

où

$$\Delta G_{MM} = \Delta G_{ele} + \Delta G_{vdw} \qquad (1)$$

$$\Delta G_{sol} = \Delta G_{ele-sol} + \Delta G_{ASA} \qquad (2)$$

**23.** Procédé selon la revendication 1, comprenant en outre les étapes de : construction d'un profil variant en ce qui concerne la position des acides aminés des séquences de polypeptide de test sélectionnées ; exclusion par filtrage des variants présentant une fréquence d'occurrence inférieure à 3 ; et combinaison des variants restants pour produire une bibliothèque combinatoire de séquences d'anticorps.

**24.** Procédé selon la revendication 23, l'étape de filtrage incluant l'exclusion par filtrage des variants présentant une fréquence d'occurrence inférieure à 5.

**25.** Procédé selon la revendication 1, l'anticorps recombinant étant un anticorps complètement assemblé, un fragment Fab, un fragment Fv ou un anticorps à chaîne unique.

**26.** Procédé selon la revendication 1, l'organisme hôte étant sélectionné dans le groupe constitué par les bactéries, les levures, les plantes, les insectes et les mammifères.

**27.** Procédé selon la revendication 1, l'antigène cible étant une petite molécule, une protéine, un peptide, un acide nucléique ou un polycarbohydrate.

**28.** Procédé selon la revendication 1, comprenant en outre les étapes de : construction d'un profil variant en ce qui concerne la position des acides aminés des séquences de polypeptide de test sélectionnées ; exclusion par filtrage des variants présentant une fréquence d'occurrence inférieure à 3 ; et combinaison des variants restants pour produire une bibliothèque combinatoire de séquences d'anticorps.

**29.** Procédé selon la revendication 28, l'étape de filtrage incluant l'exclusion par filtrage des variants présentant une fréquence d'occurrence inférieure à 5.

## Structure-based construction of human antibody library

> Database of antibody structures and models in PDB etc
> Database of human germline and rearranged Ig sequences

⇓

> Clustering structures into structure families by structure alignment, gaps etc using the global structure or specific motifs such as CDRs

⇓       ⇓       ⇓

### direct selection     Dual selection     Indirect selection

| direct selection | Dual selection | Indirect selection |
|---|---|---|
| 1. Select sequence(s) fitting onto the target structure template from germline and rearranged sequence database using by reverse threading etc<br>2. Select the master framework sequences for each distinct structure family from the top hits<br>3. Pool the sequences and their combinatorial combinations at certain cut off for CDRs | 1. Select sequence(s) from germline and rearranged sequence database using dual selection such as coblath and double threading for each structure template<br>2. Select the master framework sequences for each distinct structure family using convergent top hits<br>3. Pool the sequences and their combinatorial combinations at certain cut off for CDRs | 1. Select sequence(s) from germline and rearranged sequence database using the sequence or sequence profile of each structure family<br>2. Select the master framework sequences for each distinct structure family using top hits given by the query sequence and sequence profile<br>3. Pool the sequences and their combinatorial combinations at certain cut off for CDRs |

⇓

> • Select the master frameworks for both VH and VL.
> • Combine the selected framework with designed CDR libraires
> • Synthesize the human antibody library

**Fig 1**

EP 1 390 741 B1

# FIGURE 2

>1DGX:L IMMUNOGLOBULIN VL DOMAIN
DIQMTQSPSSLSASVGDRVTITCRASQGISSYLAWYQQKPGKAPKLLIYAASSLQSGVPS
RFSGSGSGTDFTLTISSLQPEDFATYYCQQHYTTPPTFGQGTKVEIKR
>1DH4:L IMMUNOGLOBULIN VL KAPPA DOMAIN
DIVMTQSPLSLPVTPGEPASISCRSSQSLLHSNGYNYLDWYLQKPGQSPQLLIYLGSNRA
SGVPDRFSGSGSGTDFTLKISRVEAEDVGVYYCQQHYTTPPTFGQGTKVEIKR
>1DH5:L IMMUNOGLOBULIN VL KAPPA DOMAIN
DIVLTQSPATLSLSPGERATLSCRASQSVSSSYLAWYQQKPGQAPRLLIYGASSRATGVP
ARFSGSGSGTDFTLTISSLEPEDFAVYYCQQHYTTPPTFGQGTKVEIKR
>1DH6:L IMMUNOGLOBULIN VL KAPPA DOMAIN
DIVMTQSPDSLAVSLGERATINCRSSQSVLYSSNNKNYLAWYQQKPGQPPKLLIYWASTR
ESGVPDRFSGSGSGTDFTLTISSLQAEDVAVYYCQQHYTTPPTFGQGTKVEIKR
>1DH7:L IMMUNOGLOBULIN VL LAMBDA DOMAIN
QSVLTQPPSVSGAPGQRVTISCSGSSSNIGSNYVSWYQQLPGTAPKLLIYDNNQRPSGVP
DRFSGSKSGTSASLAITGLQSEDEADYYCQQHYTTPPVFGGGTKLTVLG
>1DH8:L IMMUNOGLOBULIN VL LAMBDA DOMAIN
QSALTQPASVSGSPGQSITISCTGTSSDVGGYNYVSWYQQHPGKAPKLMIYDVSNRPSGV
SNRFSGSKSGNTASLTISGLQAEDEADYYCQQHYTTPPVFGGGTKLTVLG
>1DH9:L IMMUNOGLOBULIN VL LAMBDA DOMAIN
SYELTQPPSVSVAPGQTARISCSGDALGDKYASWYQQKPGQAPVLVIYDDSDRPSGIPER
FSGSNSGNTATLTISGTQAEDEADYYCQQHYTTPPVFGGGTKLTVLG


>1DHA:H IMMUNOGLOBULIN HEAVY CHAIN VARIABLE DOMAIN
QVQLVQSGAEVKKPGSSVKVSCKASGGTFSSYAISWVRQAPGQGLEWMGGIIPIFGTANY
AQKFQGRVTITADESTSTAYMELSSLRSEDTAVYYCARWGGDGFYAMDYWGQGTLVTVSS
>1DHO:H IMMUNOGLOBULIN HEAVY CHAIN VARIABLE DOMAIN
QVQLVQSGAEVKKPGASVKVSCKASGYTFTSYYMHWVRQAPGQGLEWMGWINPNSGGTNY
AQKFQGRVTMTRDKSSSTAYMELSSLRSEDTAVYYCARWGGDGFYAMDYWGQGTLVTVSS
>1DHQ:H IMMUNOGLOBULIN HEAVY CHAIN VARIABLE DOMAIN
QVQLKESGPALVKPTQTLTLTCTFSGFSLSTSGVGVGWIRQPPGKALEWLALIDWDDDKY
YSTSLKTRLTISKDTSKNQVVLTMTNMDPVDTATYYCARWGGDGFYAMDYWGQGTLVTVS
S
>1DHU:H IMMUNOGLOBULIN HEAVY CHAIN VARIABLE DOMAIN
EVQLVESGGGLVQPGGSLRLSCAASGFTFSSYAMSWVRQAPGKGLEWVSAISGSGGSTYY
ADSVKGRFTISRDNSKNTLYLQMNSLRAEDTAVYYCARWGGDGFYAMDYWGQGTLVTVSS
>1DHV:H IMMUNOGLOBULIN HEAVY CHAIN VARIABLE DOMAIN
QVQLQESGPGLVKPSETLSLTCTVSGGSISSYYWSWIRQPPGKGLEWIGYIYYSGSTNYN
PSLKSRVTISVDTSKNQFSLKLSSVTAADTAVYYCARWGGDGFYAMDYWGQGTLVTVSS
>1DHW:H IMMUNOGLOBULIN HEAVY CHAIN VARIABLE DOMAIN
EVQLVQSGAEVKKPGESLKISCKGSGYSFTSYWIGWVRQMPGKGLEWMGIIYPGDSDTRY
SPSFQGQVTISADKSISTAYLQWSSLKASDTAMYYCARWGGDGFYAMDYWGQGTLVTVSS
>1DHZ:H IMMUNOGLOBULIN HEAVY CHAIN VARIABLE DOMAIN
QVQLQQSGPGLVKPSQTLSLTCAISGDSVSSNSAAWNWIRQSPGRGLEWLGRTYYRSKWY
NDYAVSVKSRITINPDTSKNQFSLQLNSVTPEDTAVYYCARWGGDGFYAMDYWGQGTLVT
VSS

# Structural clustering of seven variable heavy chains

Nterm

1dga: green
1dho: cyan
1dhw: yellow
1dhz: magenta
1dhv: grey
1dhq: blue
1dhu: red

Cterm

Figure 3

EP 1 390 741 B1

# Three VH (1dha, 1dho, 1dhw) are clustered into one structure family

Figure 4A

Figure 4B

1dga: green; 1dho: cyan; 1dhw: yellow

EP 1 390 741 B1

# Structural clustering of seven variable light chains

CDR1

CDR3

CDR2

N-term

1dgx: green
1dh4: yellow
1dh5: cyan
1dh6: magenta
1dh7: red
1dh8: grey
1dh9: blue

Figure 5

C-term

EP 1 390 741 B1

# Structural clustering of 4 VL kappa
# (1dgx, 1dh4,1dh5,1dh6)

**CDR1**

**CDR3**

N-term

**CDR2**

1dgx: green
1dh4: yellow
1dh5: cyan
1dh6: magenta

Figure 6

C-term

EP 1 390 741 B1

# Structural clustering of 3 VL lamda (1dh7,1dh8,1dh9)

**CDR3**

**CDR1**

N-term

**CDR2**

1dh7: red
1dh8: grey
1dh9: blue

C-term

Figure 7

# Clustering CDR1 of 3 VL lamda

```
1DH8:L   TGTSS-DVGGYN-YVSW
1DH9:L   SGDAL-G---DK-YASW
1DH7:L   SGSSS-NIG-SN-YVSW
```

Figure 8A

EP 1 390 741 B1

# Clustering CDR1 of 4 kappa VL

Figure 8B

```
1DGX:L   RASQGI S------ SYLAW
1DH5:L   RASQSV-SS----- SYLAW
1DH4:L   RSSQSL-LHSNGY- NYLDW
1DH6:L   RSSQSV-LYSSNNK NYLAW
```

# Clustering CDR1 of 2 kappa VL

Figure 8C

```
1DH4:L   RSSQSL-LHS NGY- NYLDW
1DH6:L   RSSQSV-LYS SNNK NYLAW
```

# Clustering CDR1 of 2 kappa VL

```
1DGX:L   RASQGI-S---SYLAW
1DH5:L   RASQSV-SS-SYLAW
```

Figure 8D

EP 1 390 741 B1

# Clustering CDR1 of 7 VL chains

```
                                     -S------
                                     -SS-----
                                                        -S--
1DGX:L   RASQGI---S------SYLAW       -LHSNGY-
1DH5:L   RASQSV---SS-----SYLAW       -LYSSNNK          -SS-
1DH4:L   RSSQSL---LHSNGY-NYLDW
1DH6:L   RSSQSV---LYSSNNKNYLAW
1DH8:L   TGTSSDVGGY------NYVSW
1DH9:L   SGDALG---D------KYASW
1DH7:L   SGSSSNIG-S------NYVSW
```

-DVGGYN-

-G---DK-

-NIG-SN-

NGY-

SNNK

Figure 9

EP 1 390 741 B1

# FIGURE 10

## SELECTED FULLY HUMAN ANTIBODY SEQUENCES WITH EXTREMELY HIGH HOMOLOGY TO THE SEQUENCES OF THE TARGET STRUCTURES

| PDB_ID | Germline Name | SEQ ID NO | Aligned Residue | High Score | P(N) smallest Sum probability | Identity% | Germline Family |
|--------|---------------|-----------|-----------------|------------|-------------------------------|-----------|-----------------|
| $V_H$ Chains: | | | | | | | |
| 1DHA | VHGL1.8 | 15 | 120:98 | 502 | 7.9e-52 | 100 | vhaagrp-f1.aa |
| 1DHW | DP-73/V5-51...+ | 16 | 120:98 | 523 | 8.3e-55 | 100 | vhaagrp-f5.aa |
| 1DHO | DP-75/VI-2...+ | 17 | 120:98 | 498 | 2.1e-51 | 94 | vhaagrp-f1.aa |
| 1DHQ | S12-9/DP-27...+ | 18 | 121:100 | 494 | 1.9e-51 | 94 | vhaagrp-f2.aa |
| 1DHU | DP-47/V3-23...+ | 19 | 120:98 | 500 | 3.4e-51 | 97 | vhaagrp-f3.aa |
| 1DHV | DP-71/3d197d...+ | 20 | 119:97 | 510 | 1.3e-52 | 100 | vhaagrp-f4.aa |
| 1DHZ | DP-74/VH-VI...+ | 21 | 123:101 | 530 | 1.4e-56 | 99 | vhaagrp-f6.aa |
| $V_L$ Kappa Chains: | | | | | | | |
| 1DGX | DPK9/O12...+ | 22 | 108:95 | 466 | 4.0e-48 | 95 | vkallaa-f1.aa |
| 1DH5 | 13K18 | 23 | 109:74 | 367 | 6.2e-38 | 97 | vkallaa-f3.aa |
| 1DH4 | DPK15/A19...+ | 24 | 113:100 | 502 | 4.5e-52 | 96 | vkallaa-f2.aa |
| 1DH6 | DPK24/VkIVKlob...+ | 25 | 114:101 | 519 | 2.0e-55 | 97 | vkallaa-f4.aa |
| $V_L$ Lamda Chains: | | | | | | | |
| 1DH7 | V1-17+ | 26 | 109:98 | 436 | 2.1e-45 | 93 | vlallaa-f1.aa |
| 1DH8 | 2b2.400B5+ | 27 | 110:99 | 448 | 1.6e-46 | 95 | vlallaa-f2.aa |
| 1DH9 | 3r.9C5/DPL23...+ | 28 | 107:95 | 421 | 2.0e-43 | 89 | vlallaa-f3.aa |

EP 1 390 741 B1

## FIGURE 11

### Sequence Alignment between the Sequence of the Target Structure and the Selected Human Antibody Germline Sequence

1DHA>VHGL1.8 .
          Length = 98

 Score = 502 (176.7 bits), Expect = 7.9e-52, P = 7.9e-52
 Identities = 98/98 (100%), Positives = 98/98 (100%)

Query:    1 QVQLVQSGAEVKKPGSSVKVSCKASGGTFSSYAISWVRQAPGQGLEWMGGIIPIFGTANY 60
            QVQLVQSGAEVKKPGSSVKVSCKASGGTFSSYAISWVRQAPGQGLEWMGGIIPIFGTANY
Sbjct:    1 QVQLVQSGAEVKKPGSSVKVSCKASGGTFSSYAISWVRQAPGQGLEWMGGIIPIFGTANY 60

Query:   61 AQKFQGRVTITADESTSTAYMELSSLRSEDTAVYYCAR 98
            AQKFQGRVTITADESTSTAYMELSSLRSEDTAVYYCAR
Sbjct:   61 AQKFQGRVTITADESTSTAYMELSSLRSEDTAVYYCAR 98


1DHW>DP-73/V5-51...+ .
          Length = 98

 Score = 523 (184.1 bits), Expect = 8.3e-55, P = 8.3e-55
 Identities = 98/98 (100%), Positives = 98/98 (100%)

Query:    1 EVQLVQSGAEVKKPGESLKISCKGSGYSFTSYWIGWVRQMPGKGLEWMGIIYPGDSDTRY 60
            EVQLVQSGAEVKKPGESLKISCKGSGYSFTSYWIGWVRQMPGKGLEWMGIIYPGDSDTRY
Sbjct:    1 EVQLVQSGAEVKKPGESLKISCKGSGYSFTSYWIGWVRQMPGKGLEWMGIIYPGDSDTRY 60

Query:   61 SPSFQGQVTISADKSISTAYLQWSSLKASDTAMYYCAR 98
            SPSFQGQVTISADKSISTAYLQWSSLKASDTAMYYCAR
Sbjct:   61 SPSFQGQVTISADKSISTAYLQWSSLKASDTAMYYCAR 98


1DHO>DP-75/VI-2...+ .
          Length = 98

 Score = 498 (175.3 bits), Expect = 2.1e-51, P = 2.1e-51
 Identities = 93/98 (94%), Positives = 94/98 (95%)

Query:    1 QVQLVQSGAEVKKPGASVKVSCKASGYTFTSYYMHWVRQAPGQGLEWMGWINPNSGGTNY 60
            QVQLVQSGAEVKKPGASVKVSCKASGYTFT YYMHWVRQAPGQGLEWMGWINPNSGGTNY
Sbjct:    1 QVQLVQSGAEVKKPGASVKVSCKASGYTFTGYYMHWVRQAPGQGLEWMGWINPNSGGTNY 60

Query:   61 AQKFQGRVTMTRDKSSSTAYMELSSLRSEDTAVYYCAR 98
            AQKFQGRVTMTRD S STAYMELS LRS+DTAVYYCAR
Sbjct:   61 AQKFQGRVTMTRDTSISTAYMELSRLRSDDTAVYYCAR 98

# FIGURE 11- continued

1DHQ>S12-9/DP-27...+ .
             Length = 100

 Score = 494 (173.9 bits), Expect = 1.9e-51, P = 1.9e-51
 Identities = 94/99 (94%), Positives = 96/99 (96%)

Query:       1 QVQLKESGPALVKPTQTLTLTCTFSGFSLSTSGVGVGWIRQPPGKALEWLALIDWDDDKY 60
               QV L+ESGPALVKPTQTLTLTCTFSGFSLSTSG+ V WIRQPPGKALEWLALIDWDDDKY
Sbjct:       1 QVTLRESGPALVKPTQTLTLTCTFSGFSLSTSGMCVSWIRQPPGKALEWLALIDWDDDKY 60

Query:      61 YSTSLKTRLTISKDTSKNQVVLTMTNMDPVDTATYYCAR 99
               YSTSLKTRLTISKDTSKNQVVLTMTNMDPVDTATYYCAR
Sbjct:      61 YSTSLKTRLTISKDTSKNQVVLTMTNMDPVDTATYYCAR 99


1DHU>DP-47/V3-23...+ .
             Length = 98

 Score = 500 (176.0 bits), Expect = 3.4e-51, P = 3.4e-51
 Identities = 96/98 (97%), Positives = 98/98 (100%)

Query:       1 EVQLVESGGGLVQPGGSLRLSCAASGFTFSSYAMSWVRQAPGKGLEWVSAISGSGGSTYY 60
               EVQL+ESGGGLVQPGGSLRLSCAASGFTFSSYAMSWVRQAPGKGLEWVSAISGSGGSTYY
Sbjct:       1 EVQLLESGGGLVQPGGSLRLSCAASGFTFSSYAMSWVRQAPGKGLEWVSAISGSGGSTYY 60

Query:      61 ADSVKGRFTISRDNSKNTLYLQMNSLRAEDTAVYYCAR 98
               ADSVKGRFTISRDNSKNTLYLQMNSLRAEDTAVYYCA+
Sbjct:      61 ADSVKGRFTISRDNSKNTLYLQMNSLRAEDTAVYYCAK 98


1DHV>DP-71/3d197d...+ .
             Length = 97

 Score = 510 (179.5 bits), Expect = 1.3e-52, P = 1.3e-52
 Identities = 97/97 (100%), Positives = 97/97 (100%)

Query:       1 QVQLQESGPGLVKPSETLSLTCTVSGGSISSYYWSWIRQPPGKGLEWIGYIYYSGSTNYN 60
               QVQLQESGPGLVKPSETLSLTCTVSGGSISSYYWSWIRQPPGKGLEWIGYIYYSGSTNYN
Sbjct:       1 QVQLQESGPGLVKPSETLSLTCTVSGGSISSYYWSWIRQPPGKGLEWIGYIYYSGSTNYN 60

Query:      61 PSLKSRVTISVDTSKNQFSLKLSSVTAADTAVYYCAR 97
               PSLKSRVTISVDTSKNQFSLKLSSVTAADTAVYYCAR
Sbjct:      61 PSLKSRVTISVDTSKNQFSLKLSSVTAADTAVYYCAR 97

## FIGURE 11- continued

**1DHZ>DP-74/VH-VI...+ .**
Length = 101

Score = 530 (186.6 bits), Expect = 1.4e-56, P = 1.4e-56
Identities = 100/101 (99%), Positives = 100/101 (99%)

```
Query:     1 QVQLQQSGPGLVKPSQTLSLTCAISGDSVSSNSAAWNWIRQSPGRGLEWLGRTYYRSKWY 60
             QVQLQQSGPGLVKPSQTLSLTCAISGDSVSSNSAAWNWIRQSP RGLEWLGRTYYRSKWY
Sbjct:     1 QVQLQQSGPGLVKPSQTLSLTCAISGDSVSSNSAAWNWIRQSPSRGLEWLGRTYYRSKWY 60

Query:    61 NDYAVSVKSRITINPDTSKNQFSLQLNSVTPEDTAVYYCAR 101
             NDYAVSVKSRITINPDTSKNQFSLQLNSVTPEDTAVYYCAR
Sbjct:    61 NDYAVSVKSRITINPDTSKNQFSLQLNSVTPEDTAVYYCAR 101
```

**1DGX>DPK9/O12...+ ..**
Length = 95

Score = 466 (164.0 bits), Expect = 4.0e-48, P = 4.0e-48
Identities = 91/95 (95%), Positives = 92/95 (96%)

```
Query:     1 DIQMTQSPSSLSASVGDRVTITCRASQGISSYLAWYQQKPGKAPKLLIYAASSLQSGVPS 60
             DIQMTQSPSSLSASVGDRVTITCRASQ ISSYL WYQQKPGKAPKLLIYAASSLQSGVPS
Sbjct:     1 DIQMTQSPSSLSASVGDRVTITCRASQSISSYLNWYQQKPGKAPKLLIYAASSLQSGVPS 60

Query:    61 RFSGSGSGTDFTLTISSLQPEDFATYYCQQHYTTP 95
             RFSGSGSGTDFTLTISSLQPEDFATYYCQQ Y+TP
Sbjct:    61 RFSGSGSGTDFTLTISSLQPEDFATYYCQQSYSTP 95
```

**1DH5>13K18 ..**
Length = 74

Score = 367 (129.2 bits), Expect = 6.2e-38, P = 6.2e-38
Identities = 72/74 (97%), Positives = 73/74 (98%)

```
Query:     8 PATLSLSPGERATLSCRASQSVSSSYLAWYQQKPGQAPRLLIYGASSRATGVPARFSGSG 67
             PATLSLSPGERATLSCRASQSVSSSYLAWYQQKPGQAPRLLIYGASSRATG+PARFSGSG
Sbjct:     1 PATLSLSPGERATLSCRASQSVSSSYLAWYQQKPGQAPRLLIYGASSRATGIPARFSGSG 60

Query:    68 SGTDFTLTISSLEP 81
             SGTDFTLTIS LEP
Sbjct:    61 SGTDFTLTISRLEP 74
```

# FIGURE 11- continued

```
1DH4/>DPK15/A19...+ ..
           Length = 100

 Score = 502 (176.7 bits), Expect = 4.5e-52, P = 4.5e-52
 Identities = 96/100 (96%), Positives = 96/100 (96%)

Query:     1 DIVMTQSPLSLPVTPGEPASISCRSSQSLLHSNGYNYLDWYLQKPGQSPQLLIYLGSNRA 60
             DIVMTQSPLSLPVTPGEPASISCRSSQSLLHSNGYNYLDWYLQKPGQSPQLLIYLGSNRA
Sbjct:     1 DIVMTQSPLSLPVTPGEPASISCRSSQSLLHSNGYNYLDWYLQKPGQSPQLLIYLGSNRA 60

Query:    61 SGVPDRFSGSGSGTDFTLKISRVEAEDVGVYYCQQHYTTP 100
             SGVPDRFSGSGSGTDFTLKISRVEAEDVGVYYC Q   TP
Sbjct:    61 SGVPDRFSGSGSGTDFTLKISRVEAEDVGVYYCMQALQTP 100


1DH6>DPK24/VkIVKlob...+ ..
           Length = 101

 Score = 519 (182.7 bits), Expect = 2.0e-55, P = 2.0e-55
 Identities = 98/101 (97%), Positives = 101/101 (100%)

Query:     1 DIVMTQSPDSLAVSLGERATINCRSSQSVLYSSNNKNYLAWYQQKPGQPPKLLIYWASTR 60
             DIVMTQSPDSLAVSLGERATINC+SSQSVLYSSNNKNYLAWYQQKPGQPPKLLIYWASTR
Sbjct:     1 DIVMTQSPDSLAVSLGERATINCKSSQSVLYSSNNKNYLAWYQQKPGQPPKLLIYWASTR 60

Query:    61 ESGVPDRFSGSGSGTDFTLTISSLQAEDVAVYYCQQHYTTP 101
             ESGVPDRFSGSGSGTDFTLTISSLQAEDVAVYYCQQ+Y+TP
Sbjct:    61 ESGVPDRFSGSGSGTDFTLTISSLQAEDVAVYYCQQYYSTP 101


1DH7>V1-17+ ..
        Length = 98

 Score = 436 (153.5 bits), Expect = 2.1e-45, P = 2.1e-45
 Identities = 83/89 (93%), Positives = 85/89 (95%)

Query:     1 QSVLTQPPSVSGAPGQRVTISCSGSSSNIGSNYVSWYQQLPGTAPKLLIYDNNQRPSGVP 60
             QSVLTQPPS SG PGQRVTISCSGSSSNIGSNYV WYQQLPGTAPKLLIY NNQRPSGVP
Sbjct:     1 QSVLTQPPSASGTPGQRVTISCSGSSSNIGSNYVYWYQQLPGTAPKLLIYSNNQRPSGVP 60

Query:    61 DRFSGSKSGTSASLAITGLQSEDEADYYC 89
             DRFSGSKSGTSASLAI+GL+SEDEADYYC
Sbjct:    61 DRFSGSKSGTSASLAISGLRSEDEADYYC 89
```

## FIGURE 11- continued

1DH8>2b2.400B5+ ..
           Length = 99

 Score = 448 (157.7 bits), Expect = 1.6e-46, P = 1.6e-46
·Identities = 86/90 (95%), Positives = 87/90 (96%)

Query:     1 QSALTQPASVSGSPGQSITISCTGTSSDVGGYNYVSWYQQHPGKAPKLMIYDVSNRPSGV 60
            QSALTQPASVSGSPGQSITISCTGTSSDVG YN VSWYQQHPGKAPKLMIY+VS RPSGV
Sbjct:     1 QSALTQPASVSGSPGQSITISCTGTSSDVGSYNLVSWYQQHPGKAPKLMIYEVSKRPSGV 60

Query:    61 SNRFSGSKSGNTASLTISGLQAEDEADYYC 90
            SNRFSGSKSGNTASLTISGLQAEDEADYYC
Sbjct:    61 SNRFSGSKSGNTASLTISGLQAEDEADYYC 90


1DH9>3r.9C5/DPL23...+ ..
           Length = 95

 Score = 421 (148.2 bits), Expect = 2.0e-43, P = 2.0e-43
 Identities = 79/88 (89%), Positives = 82/88 (93%)

Query:     1 SYELTQPPSVSVAPGQTARISCSGDALGDKYASWYQQKPGQAPVLVIYDDSDRPSGIPER 60
            SYELTQPPSVSV+PGQTA I+CSGD LGDKYA WYQQKPGQ+PVLVIY DS RPSGIPER
Sbjct:     1 SYELTQPPSVSVSPGQTASITCSGDKLGDKYACWYQQKPGQSPVLVIYQDSKRPSGIPER 60

Query:    61 FSGSNSGNTATLTISGTQAEDEADYYCQ 88
            FSGSNSGNTATLTISGTQA DEADYYCQ
Sbjct:    61 FSGSNSGNTATLTISGTQAMDEADYYCQ 88

## Selecting sequence(s) using profile Hidden Markov Model derived from a structure family

**Figure 12A**

```
┌─────────────────────────────────────┐
│      A clustered Structure family     │
└─────────────────────────────────────┘
                  ⇓
┌───────────────────────────────────────────────┐
│  A structure-based multiple sequence alignment  │
└───────────────────────────────────────────────┘
                  ⇓
┌───────────────────────────────────────────────────┐
│  Build the HMM model and calibrate the model        │
│  Search the germline and rearranged sequence database │
└───────────────────────────────────────────────────┘
                  ⇓
┌───────────────────────────────────────────────────┐
│      The top hits are ranked, analyzed, pooled       │
└───────────────────────────────────────────────────┘
```

# Sequence profile of VH framework region for one structurally clustered family of (1dha, 1dho, 1dhw)

Framework (fr123) alignment after removing CDR123

1DHA:H QVQLVQSGAEVKKPGSSVKVSCKASWVRQAPGQGLEWMGRVTITADESTSTAYMELSSLRSEDTAVYYCAR
1DHO:H QVQLVQSGAEVKKPGASVKVSCKASWVRQAPGQGLEWMGRVTITADESTSTAYMELSSLRSEDTAVYYCAR
1DHW:H EVQLVQSGAEVKKPGESLKISCKGSWIGWVRQMPGKGLEQVTISADKSISTAYLQWSSLKASDTAMYYCAR

Build HMM and search human germlines/humanized sequences

1dha: green; 1dho: cyan; 1dhw: yellow

VH1-1-2-1-69   QVQLVQSGAEVKKPGSSVKVSCKASWVRQAPGQGLEWMGRVTITADESTSTAYMELSSLRSEDTAVYYCAR
VH1-1-2-1-e    QVQLVQSGAEVKKPGSSVKVSCKASWVRQAPGQGLEWMGRVTITADKSTSTAYMELSSLRSEDTAVYYCAR
VH5-1-2-5-51   EVQLVQSGAEVKKPGESLKISCKGSWVRQMPGKGLEWMGQVTISADKSISTAYLQWSSLKASDTAMYYCAR
VH1-1-3-1-03   QVQLVQSGAEVKKPGASVKVSCKASWVRQAPGQRLEWMGRVTITRDTSASTAYMELSSLRSEDTAVYYCAR
VH1-1-3-1-46   QVQLVQSGAEVKKPGASVKVSCKASWVRQAPGQGLEWMGRVTMTRDTSTSTVYMELSSLRSEDTAVYYCAR
VH1-1-2-1-18   QVQLVQSGAEVKKPGASVKVSCKASWVRQAPGQGLEWMGRVTMTTDTSTSTAYMELRSLRSDDTAVYYCAR
VH1-1-3-1-08   QVQLVQSGAEVKKPGASVKVSCKASWVRQATGQGLEWMGRVTMTRNTSISTAYMELSSLRSEDTAVYYCAR
VH5-1-2-5-a    -VQLVQSGAEVKKPGESLRISCKGSGVRQMPGKGLEWMGHVTISADKSISTAYLQWSSLKASDTAMYYCAR
etc .......

Profile variant of hits

```
qvqlvesgggivkpggslrlscaaswvrqapgkglewvgrvtisrdtskntlylqmsslraedtavyycar
elt qqtrpevkqtsatvkvt kvy iqwprqqagkymsqfvmtvenatsqasmelnnvtsa m t h tk
m  k w av ir te  si  tf  fg htsrr vgia l fnank idsfv kwt mktv    l    kt
i  l   sa l   q  t    g      ss mp  le i    k d a ivh tir  dps    m    h
       t      r       i      m            e e m          c   dd        a
              s               v           p r v          g   g
              t                           t m
              d                           l g
```

Cutoff at 5 of occurrence frequency

```
qvqlvesgggivkpggslrlscaaswvrqapgkglewvgrvtisrdtskntlylqmsslraedtavyycar
e   qq  pevkq satvkvt kv  i  p  q    ms f  tv natsqasmeln vtsa        k
        a     e  s   t                ia     a     sf  k     k
              q
              r
```

Build HMM and search human germlines/humanized sequences

Score hit and/or combinatorial sequences for structural compatibility

## Figure 12B

# Sequence/variant profile of CDR1 of 4 kappa VL

Structure-based
multiple sequence alignment
→

| 1DGX:L | RASQGIS------SYLAW |
| 1DH5:L | RASQSVSS-----SYLAW |
| 1DH4:L | RSSQSLLHSNGY-NYLDW |
| 1DH6:L | RSSQSVLYSSNNKNYLAW |

Build HMM and search Kabat database
Hit sequences and variant profile

Score hit and/or combinatorial sequences for structural compatibility

5% cutoff of ocurrence frequency

Figure 12C

# FIGURE 13A

### Stucture-Based Clustering of the Hucal Library

a) The structure-based clustering of the VH chains

(1DGA, 1DHO, 1DHW), 1DHQ, 1DHU, 1DHV, 1DHZ

b) The structure-based clustering of 7 VL chains

----------Kappa------------    -----Lamda-------
((1DGX, 1DH5), (1DH4, 1DH6))  (1DH7, 1DH8, 1DH9)

# FIGURE 13B

## Structure-Based Multiple Sequence Alignment for the Clustered Structure Family Established by Using CE Algorithm

a) For 1DGA, 1DHO and 1DHW of 3 VH

```
1DHA:H   QVQLVQSGAEVKKPGSSVKVSCKASGGTFSS--YAISWVRQAPGQGLEWMGGIIP-IFGT
1DHO:H   QVQLVQSGAEVKKPGASVKVSCKASGYTFTS--YYMHWVRQAPGQGLEWMGWINP-NSGG
1DHW:H   EVQLVQSGAEVKKPGESLKISCKGSGYSFTS--YWIGWVRQMPGKGLEWMGIIYP-GDSD

1DHA:H   ANYAQKFQGRVTITADESTSTAYMELSSLRSEDTAVYYCARWGGDGFYAMDYWGQGTLVTVSS
1DHO:H   TNYAQKFQGRVTMTRDKSSSTAYMELSSLRSEDTAVYYCARWGGDGFYAMDYWGQGTLVTVSS
1DHW:H   TRYSPSFQGQVTISADKSISTAYLQWSSLKASDTAMYYCARWGGDGFYAMDYWGQGTLVTVSS
```

b) For 1DGX and 1DH5 of 2 kappa VL

```
1DGX:L   DIQMTQSPSSLSASVGDRVTITCRASQGI---S------SYLAWYQQKPGKAPKLLI
1DH5:L   DIVLTQSPATLSLSPGERATLSCRASQSV---SS-----SYLAWYQQKPGQAPRLLI

1DGX:L   YAASSLQSGVPSRFSGSGSGTDFTLTISSLQPEDFATYYCQQHYTTPPTFGQGTKVEIK
1DH5:L   YGASSRATGVPARFSGSGSGTDFTLTISSLEPEDFAVYYCQQHYTTPPTFGQGTKVEIK
```

c) For 1DH4 and 1DH6 of 2 kappa VL

```
1DH4:L   DIVMTQSPLSLPVTPGEPASISCRSSQSL---LHSNGY-NYLDWYLQKPGQSPQLLI
1DH6:L   DIVMTQSPDSLAVSLGERATINCRSSQSV---LYSSNNKNYLAWYQQKPGQPPKLLI

1DH4:L   YLGSNRASGVPDRFSGSGSGTDFTLKISRVEAEDVGVYYCQQHYTTPPTFGQGTKVEIK
1DH6:L   YWASTRESGVPDRFSGSGSGTDFTLTISSLQAEDVAVYYCQQHYTTPPTFGQGTKVEIK
```

d) For 1DH7, 1DH8 and 1DH9 of 3 lamda VL

```
1DH7:L : QSVLTQPPS-VSGAPGQRVTISCSGSSSNIGS----NYVSWYQQLPGTAPKLLIYDNNQRPSG
1DH8:L : QSALTQPAS-VSGSPGQSITISCTGTSSDVGGY---NYVSWYQQHPGKAPKLMIYDVSNRPSG
1DH9:L : SYELTQPPS-VSVAPGQTARISCSGDALGD------KYASWYQQKPGQAPVLVIYDDSDRPSG

1DH7:L : VPDRFSGSKSGTSASLAITGLQSEDEADYYCQQHYTTPPVFGGGTKLTVLG
1DH8:L : VSNRFSGSKSGNTASLTISGLQAEDEADYYCQQHYTTPPVFGGGTKLTVLG
1DH9:L : IPERFSGSNSGNTATLTISGTQAEDEADYYCQQHYTTPPVFGGGTKLTVLG
```

# FIGURE 13C

## Top Hits of Human Germline Antibody Sequences that Adopt the Target Structure or Scaffold of the reclustered Hucal family by Using the Profile HMM Method

1) For the clustered structure family for 1DHA, 1DHO and 1DHW of VH, the top hits are listed below:

The sequences(1DHA:H, 1DHO:H and 1DHW:H) used to build the HMM are aligned with its top hits from germline sequences using HMMER 2.1.1

| Sequence | Description | Score | E-value | N |
|----------|-------------|-------|---------|---|
| DP-73/V5-51...+ | . | 232.9 | 4.5e-68 | 1 |
| VHVCW/COS-24+ | . | 232.9 | 4.5e-68 | 1 |
| DP-88/hv1051K...+ | . | 232.1 | 8.1e-68 | 1 |
| DP-10/hv1051...+ | . | 231.4 | 1.3e-67 | 1 |
| 2M27/11M27... | . | 231.4 | 1.3e-67 | 1 |
| VHGL1.8 | . | 231.4 | 1.3e-67 | 1 |
| VHVJB | . | 229.8 | 3.9e-67 | 1 |
| VHGL1.2 | . | 229.8 | 3.9e-67 | 1 |
| RR.VH1.2 | . | 229.4 | 5e-67 | 1 |
| 6M27 | . | 229.3 | 5.6e-67 | 1 |
| VH251Shen+ | . | 229.1 | 6.2e-67 | 1 |
| DP-75/VI-2...+ | . | 229.0 | 6.8e-67 | 1 |

The top hits to the profile HMM are aligned to the original structure-based sequence alignment are shown below.

```
1DHA:H           QVQLVQSGAEVKKPGSSVKVSCKASGGTFSSYAISWVRQAPGQGLEWMGG
1DHO:H           QVQLVQSGAEVKKPGASVKVSCKASGYTFTSYYMHWVRQAPGQGLEWMGW
1DHW:H           EVQLVQSGAEVKKPGESLKISCKGSGYSFTSYWIGWVRQMPGKGLEWMGI


DP-73/V5-51...+  EVQLVQSGAEVKKPGESLKISCKGSGYSFTSYWIGWVRQMPGKGLEWMGI
VHVCW/COS-24+    EVQLVQSGAEVKKPGESLKISCKGSGYSFTSYWIGWVRQMPGKGLEWMGI
DP-88/hv1051K...+ QVQLVQSGAEVKKPGSSVKVSCKASGGTFSSYAISWVRQAPGQGLEWMGG
DP-10/hv1051...+ QVQLVQSGAEVKKPGSSVKVSCKASGGTFSSYAISWVRQAPGQGLEWMGG
2M27/11M27...    QVQLVQSGAEVKKPGSSVKVSCKASGGTFSSYAISWVRQAPGQGLEWMGG
VHGL1.8          QVQLVQSGAEVKKPGSSVKVSCKASGGTFSSYAISWVRQAPGQGLEWMGG
VHVJB            EVQLVQSGAEVKKPGESLKISCKGSGYSFTSYWIGWVRQMPGKGLEWMGI
VHGL1.2          QVQLVQSGAEVKKPGASVKVSCKASGYTFTSYYMHWVRQAPGQGLEWMGW
RR.VH1.2         QVQLVQSGAEVKKPGSSVKVSCKASGGTFSSYAISWVRQAPGQGLEWMGG
6M27             QVQLVQSGAEVKKPGSSVKVSCKASGGTFSSYAISWVRQAPGQGLEWMGR
VH251Shen+       EVQLVQSGAEVKKPGESLKISCKGSGYSFTSYWTGWVRQMPGKGLEWMGI
DP-75/VI-2...+   QVQLVQSGAEVKKPGASVKVSCKASGYTFTGYYMHWVRQAPGQGLEWMGW


1DHA:H           IIPIFGTANYAQKFQGRVTITADESTSTAYMELSSLRSEDTAVYYCAR
1DHO:H           INPNSGGTNYAQKFQGRVTMTRDKSSSTAYMELSSLRSEDTAVYYCAR
1DHW:H           IYPGDSDTRYSPSFQGQVTISADKSISTAYLQWSSLKASDTAMYYCAR


DP-73/V5-51...+  IYPGDSDTRYSPSFQGQVTISADKSISTAYLQWSSLKASDTAMYYCAR
VHVCW/COS-24+    IYPGDSDTRYSPSFQGQVTISADKSISTAYLQWSSLKASDTAMYYCAR
DP-88/hv1051K...+ IIPIFGTANYAQKFQGRVTITADKSTSTAYMELSSLRSEDTAVYYCAR
DP-10/hv1051...+ IIPIFGTANYAQKFQGRVTITADESTSTAYMELSSLRSEDTAVYYCAR
2M27/11M27...    IIPIFGTANYAQKFQGRVTITADESTSTAYMELSSLRSEDTAVYYCAR
VHGL1.8          IIPIFGTANYAQKFQGRVTITADESTSTAYMELSSLRSEDTAVYYCAR
VHVJB            IYPGDSDTRYSPSFQGQVTISADKPISTAYLQWSSLKASDTAMYYCAR
VHGL1.2          INPNSGGTNYAQKFQGRVTMTRDTSISTAYVELSRLRSDDTAVYYCAR
```

```
RR.VH1.2       IIPIFGTANYAQKFQGRVTITTDESTSTAYMELSSLRSEDTAVYYCAR
6M27           IIPILGTANYAQKFQGRVTITADESTSTAYMELSSLRSEDTAVYYCAR
VH251Shen+     IYPGDSDTRYSPSFQGQVTISADKSISTAYLQWSSLKASDTAMYYCAR
DP-75/VI-2...+ INPNSGGTNYAQKFQGRVTMTRDTSISTAYMELSRLRSDDTAVYYCAR
```

2) For 1DGX and 1DH5

Scores for complete sequences (score includes all domains):

| Sequence | Description | Score | E-value | N |
|----------|-------------|-------|---------|---|
| DPK9/O12...+ | .. | 228.8 | 1.5e-67 | 1 |
| DPK23/L25...+ | .. | 221.3 | 2.7e-65 | 1 |
| V268 | .. | 221.3 | 2.7e-65 | 1 |
| V3b+ | .. | 221.2 | 3e-65 | 1 |
| L10a+ | .. | 218.9 | 1.4e-64 | 1 |
| DPK5/Vb+ | .. | 218.2 | 2.3e-64 | 1 |
| DPK6/Vb''+ | .. | 218.2 | 2.3e-64 | 1 |
| Vb'+ | .. | 218.2 | 2.3e-64 | 1 |

3) For 1DH4 and 1DH6, the top hits from human germline database

Scores for complete sequences (score includes all domains):

| Sequence | Description | Score | E-value | N |
|----------|-------------|-------|---------|---|
| DPK24/VkIVKlob...+ | .. | 251.0 | 3.1e-74 | 1 |
| DPK15/A19...+ | .. | 236.0 | 9.8e-70 | 1 |
| DPK13/O11...+ | .. | 205.9 | 1.2e-60 | 1 |
| DPK36/Chr22-4 | .. | 201.5 | 2.5e-59 | 1 |
| DPK12/A2+ | .. | 199.1 | 1.3e-58 | 1 |
| A2b/A2c+ | .. | 198.7 | 1.7e-58 | 1 |
| DPK27/A29+ | .. | 196.8 | 6.3e-58 | 1 |
| A18b+ | .. | 196.2 | 9.5e-58 | 1 |
| DPK18/A17+ | .. | 196.0 | 1.1e-57 | 1 |
| A13+ | .. | 194.8 | 2.6e-57 | 1 |
| DPK19/A1+ | .. | 190.7 | 4.3e-56 | 1 |
| DPK28/A18+ | .. | 189.8 | 8.3e-56 | 1 |
| DPK16/A23+ | .. | 188.1 | 2.7e-55 | 1 |

4) For 1DH7, 1DH8 and 1DH9, the top hits from human germline database

Scores for complete sequences (score includes all domains):

| Sequence | Description | Score | E-value | N |
|----------|-------------|-------|---------|---|
| DPL12+ | .. | 174.8 | 5.7e-51 | 1 |
| 2e.2.2/V1-3+ | .. | 174.8 | 5.7e-51 | 1 |
| 2a2.272A12/DPL11...+ | .. | 174.3 | 8e-51 | 1 |
| 2c.118D9/V1-2+ | .. | 171.6 | 5.1e-50 | 1 |
| VL2.1(IGLV2S1)+ | .. | 170.2 | 1.4e-49 | 1 |
| 2b2.400B5+ | .. | 169.9 | 1.7e-49 | 1 |
| 1v2046 | .. | 169.7 | 1.9e-49 | 1 |
| 2d.29D11/DPL13...+ | .. | 169.2 | 2.7e-49 | 1 |
| 1v216.21 | .. | 169.1 | 2.9e-49 | 1 |
| DPL10/V1-7...+ | .. | 167.5 | 9e-49 | 1 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9708320 A

- US 60284407 B

### Non-patent literature cited in the description

- **CHOTHIA et al.** *J. Mol. Biol.,* 1985, vol. 186, 651-663
- **NOVOTNY ; HABER.** *Proc. Natl. Acad. Sci. USA,* 1985, vol. 82, 4592-4596
- **CHOTHIA ; LESK.** *J. Mol. Biol.,* vol. 196, 901-917
- **CHOTHIA et al.** *Nature (London,* vol. 342, 877
- **CHOTHIA et al.** *J. Mol. Biol.,* 1998, vol. 278, 457-479
- **DWEK et al.** *Eur. J. Biochem.,* 1975, vol. 53, 25-39
- **ANGLISTER et al.** *Biochem.,* 1987, vol. 26, 6958-6064
- **MCMANUS ; RIECHMANN.** *Biochem.,* 1991, vol. 30, 5851-5857
- **KABAT ; WU.** *Proc. Natl. Acad. Sci. USA,* 1972, vol. 69, 960-964
- **MARTIN, A.C.R.** *PNAS,* 1989, vol. 86, 9268-72
- **IBA et al.** *Protein Eng.,* 1998, vol. 11, 361-370
- **HUSE et al.** *Science,* vol. 246, 1275
- **CATON ; KOPROWSKI.** *Proc. Natl. Acad. Sci. (U.S.A.,* 1990, vol. 87, 6450
- **MULLINAX et al.** *Proc. Natl. Acad. Sci. (U.S.A.,* 1990, vol. 87, 8095
- **PERSSON et al.** *Proc. Natl. Acad. Sci. (U.S.A.,* 1991, vol. 88, 2432
- **KANG et al.** *Proc. Natl. Acad. Sci. (U.S.A.,* 1991, vol. 88, 4363
- **CLACKSON et al.** *Nature,* 1991, vol. 352, 624
- **MCCAFFERTY et al.** *Nature,* 1990, vol. 348, 552
- **BURTON et al.** *Proc. Natl. Acad. Sci. (U.S.A.,* 1991, vol. 88, 10134
- **HOOGENBOOM et al.** *Nucleic Acids Res.,* 1991, vol. 19, 4133
- **CHANG et al.** *J. Immunol.,* 1991, vol. 147, 3610
- **BREITLING et al.** *Gene,* 1991, vol. 104, 147
- **MARKS et al.** *J. Mol. Biol.,* 1991, vol. 222, 581
- **BARBAS et al.** *Proc. Natl. Acad. Sci. (U.S.A.,* vol. 89, 4457
- **HAWKINS ; WINTER.** *J. Immunol.,* 1992, vol. 22, 867

- **MARKS et al.** *Biotechnology,* 1992, vol. 10, 779
- **MARKS et al.** *J. Biol. Chem.,* 1992, vol. 267, 16007
- **LOWMAN et al.** *Biochemistry,* vol. 30, 10832
- **LERNER et al.** *Science,* 1992, vol. 258, 1313
- **RADER, C. ; BARBAS, C. F.** Phage display of combinatorial antibody libraries. *Curr. Opin. Biotechnol.,* 1997, vol. 8, 503-508
- **BARBAS ; BURTON.** *Trends Biotech.,* 1996, vol. 14, 230-234
- **HAARD et al.** *J. Biol. Chem.,* 1999, vol. 274, 18218-18230
- **MARKS et al.** *Eur. J. Immunol.,* 1991, vol. 21, 985-991
- **GRIFFITHS et al.** *EMBO J.,* vol. 13, 3245-3260
- **KNAPPIK et al.** *J. Mol. Biol.,* 2000, vol. 296, 57-86
- **GRIFFITHS et al.** *EMBO J.,* 15 July 1994, vol. 13 (14), 3245-3260
- **MARTI-RENOM et al.** *Annu. Rev. BioPhys. Biomol. Struct.,* 2000, vol. 29, 291-325
- **MACCULLUM et al.** Computational Analysis of Protein Sequence and Structure - Thesis. 1997, 1-16236-53133-136
- **MIRNY et al.** *J. Mol. Biol.,* vol. 283, 507-526
- **DILL.** *Biochem.,* 1985, vol. 24, 1501-1509
- **BARRE et al.** Structural conservation of hypervariable regions in immunoglobins evolution. *Nature Struct. Biol.,* 1994, vol. 1, 915-920
- **ORENGO et al.** *Proteins,* 1992, vol. 14, 139-167
- **HOLM ; SANDER.** *J. Mol. Biol.,* 1993, vol. 233, 123-138
- **FISCHER et al.** *J. Biomol. Struct. Dyn.,* vol. 9, 769-789
- **VRIEND ; SANDER.** *Proteins,* 1991, vol. 11, 52-58
- **ALEXANDROV.** *Protein Eng.,* 1996, vol. 9, 727-732
- **SHINDYALOV ; BOURNE.** *Protein Eng.,* 1998, vol. 9, 739-747

- **SHINDYALOV ; BOURNE.** *Nucleic Acid Res.,* 2001, vol. 29, 228-229
- **JOHNSON ; WU.** *Kabat Database and its applications: future directions,* 2001, vol. 29, 205-206
- **BOWIE et al.** *Science,* 1991, vol. 253, 164-170
- **XU ; XU.** *Proteins: Structure, Function, and Genetics,* 2000, vol. 40, 343-354
- **PANCHENKO et al.** *J. Mol. Biol.,* 2000, vol. 296, 1319-1331
- **R. E. BRUCCOLERI.** *Molecular Simulations,* 1993, vol. 10, 151-174
- **R. E. BRUCCOLERI ; E. HABER ; J. NOVOTNY.** *Nature,* 1988, vol. 335, 564-568
- **R. BRUCCOLERI ; M. KARPLUS.** *Biopolymers,* 1987, vol. 26, 137-168
- **BROOKS BR ; BRUCCOLERI BE ; OLAFSON BD ; STATES DJ ; SWAMINATHAN S ; KARPLUS M.** *J. Comput. Chem.,* 1983, vol. 4, 187-217
- **BRUCCOLERI ; KARPLUS.** *Biopolymers,* 1987, vol. 26, 137-168
- **BRUCCOLERI ; NOVOTNY.** *Immunomethods,* 1992, 96-106
- **DUNBRACK RL JR ; KARPLUS M.** *J Mol Biol,* 1993, vol. 230, 543-574
- **BOWER, MJ ; COHEN FE ; DUNBRACK RL.** *J Mol Biol,* 1997, vol. 267, 1268-1282
- **MANDAL et al.** *Nature Biotech.,* 1996, vol. 14, 323-328
- **WHITELEGG NRJ ; REES, AR.** *Protein Engineering,* 2000, vol. 13, 819-824
- **MARTIN, ACR ; CHEETHAM, JC ; REES AR.** *PNAS,* 1989, vol. 86, 9268-9272
- **BRUCCOLERI RE ; HABER E ; NOVOTNY J.** *Nature,* 1988, vol. 355, 564-568
- **BONNEAU R ; TSAI J ; RUCZINSKI I ; CHIVIAN D ; ROHL C ; STRAUSS CE ; BAKER D.** *Proteins,* 2001, vol. 5, 119-126
- **LEE MR ; TSAI J ; BAKER D ; KOLLMAN PA.** *J Mol Biol,* 2001, vol. 313, 417-430
- **SHARP KA.** *Proteins,* 1998, vol. 33, 39-48
- **NOVOTNY J ; BRUCCOLERI RE ; DAVIS M ; SHARP KA.** *J Mol Biol,* 1997, vol. 268, 401-411
- **SITKOFF D ; SHARP, KA ; HONIG B.** *J Phys Chem,* 1994, vol. 98, 1978-1988
- **CORNELL, WD ; CIEPLAK P ; BAYLY CI ; GOULD IR ; MERZ KM JR ; FERGUSON DM ; SPELLMEYER DC ; FOX T ; CALDWELL JW ; KOLLMAN PA.** *JACS,* 1995, vol. 117, 5179-5197
- **BROOKS, B.R. ; BRUCCOLERI, R.E. ; OLAFSON, B.D. ; STATES, D.J. ; SWAMINATHAN, S. ; KARPLUS, M.** *J. Comp. Chem.,* 1983, vol. 4, 187-217
- **MACKERELL, A D ; BASHFORD, D ; BELLOTT, M ; DUNBRACK, R L ; EVA SECK, J D ; FIELD, M J ; FISCHER, S ; GAO, J ; GUO, H ; HA, S.** *J. Phys. Chem., B,* 1998, vol. 102, 3586-3617
- **DAUBER-OSGUTHORPE, P. ; ROBERTS, V. A. ; OSGUTHORPE, D. J. ; WOLFF, J. ; GENEST, M. ; HAGLER, A. T.** *Proteins: Structure, Function and Genetics,* 1988, vol. 4, 31-47
- **MOMANY, F. A. ; MCGUIRE, R. F. ; BURGESS, A. W. ; SCHERAGA, H. A.** *J. Phys. Chem.,* 1975, vol. 79, 2361-2381
- **NEMETHY, G. ; POTTLE, M. S. ; SCHERAGA, H. A.** *J. Phys. Chem.,* 1983, vol. 87, 1883-1887
- **HERMANS, J. ; BERENDSEN, H. J. C. ; GUNSTEREN, W. F. ; POSTMA, J. P. M.** *Biopolymers,* 1984, vol. 23, 1
- **HALGREN, T. A.** *J. Am. Chem. Soc.,* 1992, vol. 114, 7827-7843
- **HALGREN, T. A.** *J. Comp. Chem,* 1996, vol. 17, 490-519
- **HALGREN, T. A.** *J. Comp. Chem.,* vol. 17, 520-552
- **HALGREN, T. A.** *J. Comp. Chem.,* 1996, vol. 17, 553-586
- **HALGREN, T. A. ; NACHBAR, R. B.** *J. Comp. Chem.,* 1996, vol. 17, 587-615
- **HALGREN, T. A.** *J. Comp. Chem.,* 1996, vol. 17, 616-641
- **JORGENSEN, W. L. ; TIRADO-RIVES, J.** *J. Am. Chem. Soc.,* 1997, vol. 110, 1657-1666
- **DAMM, W. ; A. FRONTERA ; J. TIRADO-RIVES ; W. L. JORGENSEN.** *J. Comp. Chem.,* vol. 18, 1955-1970
- **CLARK, M. ; CRAMER III, R. D. ; OPDENHOSCH, N.** Validation of the General Purpose Tripose 5.2 Force Field. *J. Comp. Chem.,* 1989, vol. 10, 982-1012
- **LII, J-H. ; ALLINGER, N. L.** *J. Comp. Chem.,* 1991, vol. 12, 186-199
- **MAYO SL ; OLAFSON BD ; GODDARD.** *J Phy Chem,* 1990, vol. 94, 8897-8909
- **LIWO et al.** *Protein Science,* 1993, vol. 2, 1697-1714
- **LIWO et al.** *Protein Science,* vol. 2, 1715-1731
- **LIWO et al.** *J. Comp. Chem.,* 1997, vol. 18, 849-873
- **LIWO et al.** *J. Comp. Chem.,* 1997, vol. 18, 874-884
- **LIWO et al.** *J. Comp. Chem.,* 1998, vol. 19, 259-276
- **MIYAZAWA S ; JERNIGAN R.** *Macromolecules,* 1985, vol. 18, 534-552
- **JERNIGAN RL ; BAHAR, I.** *Curr. Opin. Struc. Biol.,* 1996, vol. 6, 195-209
- **HENDLICH et al.** *J. Mol. Biol.,* 1990, vol. 216, 167-180
- **SIPPL M.** *J Mol Biol,* 1990, vol. 213, 859-883
- **THOMAS PD ; DILL KA.** *J Mol Biol,* 1996, vol. 257, 457-469
- **BEN-NAIM A.** *J Chem Phys,* 1997, vol. 107, 3698-3706

- **BOWIE JU ; LUTHY R ; EISENBERG DA.** *Science,* 1991, vol. 253, 164-170
- **JONES DT ; TAYLOR WR ; THORNTON JM.** *Nature,* 1992, vol. 358, 86-89
- **BRYANT, SH ; LAWRENCE, CE.** *Proteins,* 1993, vol. 16, 92-112
- **ROST B ; SCHNEIDER R ; SANDER C.** *J Mol Biol,* 1997, vol. 270, 471-480
- **XU Y ; XU D.** *Proteins,* 2000, vol. 40, 343-354
- **SIMONS KT ; KOOPERBERG C ; HUANG E ; BAKER D.** *J Mol Biol,* 1997, vol. 268, 209-225
- **DIMA RI ; BANAVAR J R ; MARITAN A.** *Protein Science,* 2000, vol. 9, 812-819
- **SPOLAR RS ; LIVINGSTONE JR ; RECORD MT.** *Biochemistry,* 1992, vol. 31, 3947-3955
- **SPOLAR RS ; RECORD MT.** *Science,* vol. 263, 777-784
- **MURPHY KP ; FREIRE E.** *Adv Protein Chem,* 1992, vol. 43, 313-361
- **PRIVALOV PL ; MAKHATADZE GI.** *J Mol Biol,* 1993, vol. 232, 660-679
- **MAKHATADZE GI ; PRIVALOV PL.** *J Mol Biol,* 1993, vol. 232, 639-659
- **HILSER VJ ; DOWDY D ; OAS TG ; FREIRE E.** *PNAS,* 1998, vol. 95, 9903-9908
- **ROHL CA ; BALDWIN RL.** *Methods Enzymol,* 1998, vol. 295, 1-26
- **SERRANO L.** *Adv Protein Chem,* 2000, vol. 53, 49-85
- **PONDER JW ; RICHARDS FM.** *J Mol Biol,* 1987, vol. 193, 775-791
- **HELLINGA HW ; RICHARDS FM.** *PNAS,* 1994, vol. 91, 5803-5807
- **DESJARLAIS J ; HANDEL T.** *Protein Science,* 1995, vol. 4, 2006-2018
- **KONO H ; DOI J.** *Proteins,* 1994, vol. 19, 244-255
- **KOEHL P ; LEVITT M.** *J Mol Biol,* 1999, vol. 293, 1161-1181
- **DELARUE M ; KOEHL.** *Pac. Symp. Biocomput.,* 1997, 109-121
- **KOEHL P ; DELARUE M.** *J. Mol. Biol.,* 1994, vol. 239, 249-275
- **KOEHL P ; DELARUE M.** *Nat. Struct. Biol.,* vol. 2, 163-170
- **KOEHL P ; DELARUE M.** *Curr. Opin. Struct. Biol.,* vol. 6, 222-226
- **LEE J.** *Mol. Biol.,* 1994, vol. 236, 918-939
- **VASQUEZ.** *Biopolymers,* 1995, vol. 36, 53-70
- **KUHLMAN B ; BAKER D.** *PNAS,* 2000, vol. 97, 10383-10388
- **JIANG X ; FARID H ; PISTOR E ; FARID RS.** *Protein Science,* 2000, vol. 9, 403-416
- **ROSSI A ; MICHELETTI C ; SENO F ; MARITAN A.** *Biophysical Journal,* 2001, vol. 80, 480-490
- **DAHIYAT BI ; MAYO SL.** *Protein Science,* 1996, vol. 5, 895-903
- **GORDON DB ; MARSHALL SA ; MAYO SL.** *Curr Opion Stru Biol,* 1999, vol. 9, 509-513
- **KOLLMAN PA ; MASSOVA I ; REYES C ; KUHN B ; HUO SH ; CHONG LT ; LEE M ; LEE TS ; DUAN Y ; WANG W.** *Acc. Chem. Res.,* 2000, vol. 33, 889-897
- **WANG W ; KOLLMAN P.** *JMB,* 2001

SEQUENCE LISTING

    <110> Abmaxis, Inc.
    Luo, Peizhi

    <120> STRUCTURE-BASED CONSTRUCTION OF HUMAN ANTIBODY LIBRARY

    <130> 26050-706

    <150> US 60/284,407
    <151> 2001-04-17

    <160> 28

    <170> PatentIn version 3.1

    <210> 1
    <211> 120
    <212> PRT
    <213> Artificial sequence

    <220>

<223> Human consensus antibody heavy chain variable region

<400> 1

```
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
            35                  40                  45

Gly Gly Ile Ile Pro Ile Phe Gly Thr Ala Asn Tyr Ala Gln Lys Phe
        50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Trp Gly Gly Asp Gly Phe Tyr Ala Met Asp Tyr Trp Gly Gln
            100                 105                 110

Gly Thr Leu Val Thr Val Ser Ser
            115                 120
```

<210> 2
<211> 120
<212> PRT
<213> Artificial Sequence

<220>
<223> Human consensus antibody heavy chain variable region

<400> 2

Gln Val Gln Leu Val Gln Ser Gly Ala Glu Val Lys Lys Pro Gly Ala
1                   5                   10                  15

Ser Val Lys Val Ser Cys Lys Ala Ser Gly Tyr Thr Phe Thr Ser Tyr
              20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Lys Ser Ser Ser Thr Ala Tyr
65                  70                  75                  80

Met Glu Leu Ser Ser Leu Arg Ser Glu Asp Thr Ala Val Tyr Tyr Cys
              85                  90                  95

Ala Arg Trp Gly Gly Asp Gly Phe Tyr Ala Met Asp Tyr Trp Gly Gln
            100                 105                 110

Gly Thr Leu Val Thr Val Ser Ser
            115                 120

<210> 3
<211> 120
<212> PRT
<213> Artificial sequence

<220>
<223> Human consensus antibody heavy chain variable region

<400> 3

```
Gln Val Gln Leu Lys Glu Ser Gly Pro Ala Leu Val Lys Pro Thr Gln
1               5               10              15

Thr Leu Thr Leu Thr Cys Thr Phe Ser Gly Phe Ser Leu Ser Thr Ser
            20              25              30

Gly Val Gly Val Gly Trp Ile Arg Gln Pro Pro Gly Lys Ala Leu Glu
        35              40              45

Trp Leu Ala Leu Ile Asp Trp Asp Asp Asp Lys Tyr Tyr Ser Thr Ser
    50              55              60

Leu Lys Thr Arg Leu Thr Ile Ser Lys Asp Thr Ser Lys Asn Gln Val
65              70              75              80

Val Leu Thr Met Thr Asn Met Asp Pro Val Asp Thr Ala Thr Tyr Tyr
            85              90              95

Cys Ala Arg Trp Gly Gly Asp Gly Phe Tyr Ala Met Asp Tyr Trp Gly
            100             105             110

Gln Gly Thr Leu Val Thr Val Ser
            115             120
```

<210> 4
<211> 120
<212> PRT
<213> Artificial sequence

<220>
<223> Human consensus antibody heavy chain variable region

<400> 4

```
Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Ser Ser Tyr
            20              25              30

Ala Met Ser Trp Val Arg Gln Ala Pro Gly Lys Gly Leu Glu Trp Val
        35              40              45

Ser Ala Ile Ser Gly Ser Gly Gly Ser Thr Tyr Tyr Ala Asp Ser Val
    50              55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
            85              90              95

Ala Arg Trp Gly Gly Asp Gly Phe Tyr Ala Met Asp Tyr Trp Gly Gln
        100             105             110

Gly Thr Leu Val Thr Val Ser Ser
        115             120
```

<210> 5
<211> 119
<212> PRT
<213> Artificial Sequence

<220>
<223> Human consensus antibody heavy chain variable region

<400> 5

```
Gln Val Gln Leu Gln Glu Ser Gly Pro Gly Leu Val Lys Pro Ser Glu
1               5               10              15

Thr Leu Ser Leu Thr Cys Thr Val Ser Gly Gly Ser Ile Ser Ser Tyr
            20              25              30

Tyr Trp Ser Trp Ile Arg Gln Pro Pro Gly Lys Gly Leu Glu Trp Ile
        35              40              45
```

EP 1 390 741 B1

Gly Tyr Ile Tyr Tyr Ser Gly Ser Thr Asn Tyr Asn Pro Ser Leu Lys
50 55 60

Ser Arg Val Thr Ile Ser Val Asp Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Trp Gly Gly Asp Gly Phe Tyr Ala Met Asp Tyr Trp Gly Gln Gly
100 105 110

Thr Leu Val Thr Val Ser Ser
115

<210> 6
<211> 120
<212> PRT
<213> Artificial Sequence

<220>
<223> Human consensus antibody heavy chain variable region

<400> 6

Glu Val Gln Leu Val Gln Ser Gly Ala Glu Val Lys Lys Pro Gly Glu
1 5 10 15

Ser Leu Lys Ile Ser Cys Lys Gly Ser Gly Tyr Ser Phe Thr Ser Tyr
20 25 30

Trp Ile Gly Trp Val Arg Gln Met Pro Gly Lys Gly Leu Glu Trp Met
35 40 45

Gly Ile Ile Tyr Pro Gly Asp Ser Asp Thr Arg Tyr Ser Pro Ser Phe
50 55 60

Gln Gly Gln Val Thr Ile Ser Ala Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg Trp Gly Gly Asp Gly Phe Tyr Ala Met Asp Tyr Trp Gly Gln
100 105 110

Gly Thr Leu Val Thr Val Ser Ser
115 120

<210> 7
<211> 123
<212> PRT
<213> Artificial Sequence

<220>
<223> Human consensus antibody heavy chain variable region

<400> 7

```
Gln Val Gln Leu Gln Gln Ser Gly Pro Gly Leu Val Lys Pro Ser Gln
1                   5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Gly Arg Gly Leu Glu
            35                  40                  45

Trp Leu Gly Arg Thr Tyr Tyr Arg Ser Lys Trp Tyr Asn Asp Tyr Ala
        50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Trp Gly Gly Asp Gly Phe Tyr Ala Met Asp Tyr
            100                 105                 110

Trp Gly Gln Gly Thr Leu Val Thr Val Ser Ser
            115                 120
```

<210> 8
<211> 108
<212> PRT
<213> Artificial Sequence

<220>
<223> Human consensus antibody light chain variable region

<400> 8

```
Asp Ile Gln Met Thr Gln Ser Pro Ser Ser Leu Ser Ala Ser Val Gly
1               5               10              15

Asp Arg Val Thr Ile Thr Cys Arg Ala Ser Gln Gly Ile Ser Ser Tyr
            20              25              30

Leu Ala Trp Tyr Gln Gln Lys Pro Gly Lys Ala Pro Lys Leu Leu Ile
            35              40              45

Tyr Ala Ala Ser Ser Leu Gln Ser Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln His Tyr Thr Thr Pro Pro
            85              90              95

Thr Phe Gly Gln Gly Thr Lys Val Glu Ile Lys Arg
            100             105
```

<210> 9
<211> 113
<212> PRT
<213> Artificial Sequence

<220>
<223> Human consensus antibody light chain variable region

<400> 9

```
Asp Ile Val Met Thr Gln Ser Pro Leu Ser Leu Pro Val Thr Pro Gly
1               5               10              15

Glu Pro Ala Ser Ile Ser Cys Arg Ser Ser Gln Ser Leu Leu His Ser
            20              25              30

Asn Gly Tyr Asn Tyr Leu Asp Trp Tyr Leu Gln Lys Pro Gly Gln Ser
            35              40              45

Pro Gln Leu Leu Ile Tyr Leu Gly Ser Asn Arg Ala Ser Gly Val Pro
```

EP 1 390 741 B1

|  | | 50 | | | | 55 | | | | 60 | | | |

Asp Arg Phe Ser Gly Ser Gly Ser Gly Thr Asp Phe Thr Leu Lys Ile
65           70            75          80

Ser Arg Val Glu Ala Glu Asp Val Gly Val Tyr Tyr Cys Gln Gln His
       85            90          95

Tyr Thr Thr Pro Pro Thr Phe Gly Gln Gly Thr Lys Val Glu Ile Lys
       100           105         110

Arg

<210> 10
<211> 109
<212> PRT
<213> Artificial sequence

<220>
<223> Human consensus antibody light chain variable region

<400> 10

Asp Ile Val Leu Thr Gln Ser Pro Ala Thr Leu Ser Leu Ser Pro Gly
1           5            10          15

Glu Arg Ala Thr Leu Ser Cys Arg Ala Ser Gln Ser Val Ser Ser Ser
       20            25          30

Tyr Leu Ala Trp Tyr Gln Gln Lys Pro Gly Gln Ala Pro Arg Leu Leu
       35           40          45

Ile Tyr Gly Ala Ser Ser Arg Ala Thr Gly Val Pro Ala Arg Phe Ser
       50           55          60

Gly Ser Gly Ser Gly Thr Asp Phe Thr Leu Thr Ile Ser Ser Leu Glu
65           70           75         80

Pro Glu Asp Phe Ala Val Tyr Tyr Cys Gln Gln His Tyr Thr Thr Pro
       85            90          95

Pro Thr Phe Gly Gln Gly Thr Lys Val Glu Ile Lys Arg
       100           105

<210> 11
<211> 114
<212> PRT
<213> Artificial Sequence

<220>
<223> Human consensus antibody light chain variable region

<400> 11

```
Asp Ile Val Met Thr Gln Ser Pro Asp Ser Leu Ala Val Ser Leu Gly
1               5               10                  15

Glu Arg Ala Thr Ile Asn Cys Arg Ser Ser Gln Ser Val Leu Tyr Ser
            20              25                  30

Ser Asn Asn Lys Asn Tyr Leu Ala Trp Tyr Gln Gln Lys Pro Gly Gln
        35              40                  45

Pro Pro Lys Leu Leu Ile Tyr Trp Ala Ser Thr Arg Glu Ser Gly Val
        50              55              60

Pro Asp Arg Phe Ser Gly Ser Gly Ser Gly Thr Asp Phe Thr Leu Thr
65              70              75                  80

Ile Ser Ser Leu Gln Ala Glu Asp Val Ala Val Tyr Tyr Cys Gln Gln
            85              90                  95

His Tyr Thr Thr Pro Pro Thr Phe Gly Gln Gly Thr Lys Val Glu Ile
            100             105                 110

Lys Arg
```

<210> 12
<211> 109
<212> PRT
<213> Artificial Sequence

<220>
<223> Human consensus antibody light chain variable region

<400> 12

```
Gln Ser Val Leu Thr Gln Pro Pro Ser Val Ser Gly Ala Pro Gly Gln
1               5               10              15

Arg Val Thr Ile Ser Cys Ser Gly Ser Ser Ser Asn Ile Gly Ser Asn
            20              25              30

Tyr Val Ser Trp Tyr Gln Gln Leu Pro Gly Thr Ala Pro Lys Leu Leu
        35              40              45

Ile Tyr Asp Asn Asn Gln Arg Pro Ser Gly Val Pro Asp Arg Phe Ser
    50              55              60

Gly Ser Lys Ser Gly Thr Ser Ala Ser Leu Ala Ile Thr Gly Leu Gln
65              70              75              80

Ser Glu Asp Glu Ala Asp Tyr Tyr Cys Gln Gln His Tyr Thr Thr Pro
            85              90              95

Pro Val Phe Gly Gly Gly Thr Lys Leu Thr Val Leu Gly
            100             105
```

<210> 13
<211> 110
<212> PRT
<213> Artificial Sequence

<220>
<223> Human consensus antibody light chain variable region

<400> 13

```
Gln Ser Ala Leu Thr Gln Pro Ala Ser Val Ser Gly Ser Pro Gly Gln
1               5               10              15

Ser Ile Thr Ile Ser Cys Thr Gly Thr Ser Ser Asp Val Gly Gly Tyr
            20              25              30

Asn Tyr Val Ser Trp Tyr Gln Gln His Pro Gly Lys Ala Pro Lys Leu
        35              40              45

Met Ile Tyr Asp Val Ser Asn Arg Pro Ser Gly Val Ser Asn Arg Phe
    50              55              60

Ser Gly Ser Lys Ser Gly Asn Thr Ala Ser Leu Thr Ile Ser Gly Leu
65              70              75              80

Gln Ala Glu Asp Glu Ala Asp Tyr Tyr Cys Gln Gln His Tyr Thr Thr
            85              90              95

Pro Pro Val Phe Gly Gly Gly Thr Lys Leu Thr Val Leu Gly
            100             105             110
```

<210> 14
<211> 107
<212> PRT
<213> Artificial Sequence

<220>
<223> Human consensus antibody light chain variable region

<400> 14

```
Ser Tyr Glu Leu Thr Gln Pro Pro Ser Val Ser Val Ala Pro Gly Gln
1               5               10              15

Thr Ala Arg Ile Ser Cys Ser Gly Asp Ala Leu Gly Asp Lys Tyr Ala
            20              25              30

Ser Trp Tyr Gln Gln Lys Pro Gly Gln Ala Pro Val Leu Val Ile Tyr
        35              40              45

Asp Asp Ser Asp Arg Pro Ser Gly Ile Pro Glu Arg Phe Ser Gly Ser
    50              55              60

Asn Ser Gly Asn Thr Ala Thr Leu Thr Ile Ser Gly Thr Gln Ala Glu
65              70              75              80

Asp Glu Ala Asp Tyr Tyr Cys Gln Gln His Tyr Thr Thr Pro Pro Val
            85              90              95

Phe Gly Gly Gly Thr Lys Leu Thr Val Leu Gly
            100             105
```

<210> 15
<211> 98
<212> PRT
<213> Homo sapiens

<400> 15

Gln Val Gln Leu Val Gln Ser Gly Ala Glu Val Lys Lys Pro Gly Ser
1                5                     10                 15

Ser Val Lys Val Ser Cys Lys Ala Ser Gly Gly Thr Phe Ser Ser Tyr
              20                  25                     30

Ala Ile Ser Trp Val Arg Gln Ala Pro Gly Gln Gly Leu Glu Trp Met
          35                  40                  45

Gly Gly Ile Ile Pro Ile Phe Gly Thr Ala Asn Tyr Ala Gln Lys Phe
        50                  55                  60

Gln Gly Arg Val Thr Ile Thr Ala Asp Glu Ser Thr Ser Thr Ala Tyr
65                   70                  75                   80

Met Glu Leu Ser Ser Leu Arg Ser Glu Asp Thr Ala Val Tyr Tyr Cys
              85                  90                  95

Ala Arg

<210> 16
<211> 98
<212> PRT
<213> Homo sapiens

<400> 16

Glu Val Gln Leu Val Gln Ser Gly Ala Glu Val Lys Lys Pro Gly Glu
1                5                     10                 15

Ser Leu Lys Ile Ser Cys Lys Gly Ser Gly Tyr Ser Phe Thr Ser Tyr
              20                  25                     30

Trp Ile Gly Trp Val Arg Gln Met Pro Gly Lys Gly Leu Glu Trp Met
          35                  40                  45

Gly Ile Ile Tyr Pro Gly Asp Ser Asp Thr Arg Tyr Ser Pro Ser Phe
        50                  55                  60

Gln Gly Gln Val Thr Ile Ser Ala Asp Lys Ser Ile Ser Thr Ala Tyr
65                   70                  75                   80

Leu Gln Trp Ser Ser Leu Lys Ala Ser Asp Thr Ala Met Tyr Tyr Cys
              85                  90                  95

Ala Arg

<210> 17
<211> 98
<212> PRT
<213> Homo sapiens

<400> 17

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Thr Gly Tyr
            20              25              30

Tyr Met His Trp Val Arg Gln Ala Pro Gly Gln Gly Leu Glu Trp Met
        35              40              45

Gly Trp Ile Asn Pro Asn Ser Gly Gly Thr Asn Tyr Ala Gln Lys Phe
        50              55              60

Gln Gly Arg Val Thr Met Thr Arg Asp Thr Ser Ile Ser Thr Ala Tyr
65              70              75              80

Met Glu Leu Ser Arg Leu Arg Ser Asp Asp Thr Ala Val Tyr Tyr Cys
                85              90              95

Ala Arg
```

<210> 18
<211> 99
<212> PRT
<213> Homo sapiens

<400> 18

```
Gln Val Thr Leu Arg Glu Ser Gly Pro Ala Leu Val Lys Pro Thr Gln
1               5               10              15

Thr Leu Thr Leu Thr Cys Thr Phe Ser Gly Phe Ser Leu Ser Thr Ser
            20              25              30

Gly Met Cys Val Ser Trp Ile Arg Gln Pro Pro Gly Lys Ala Leu Glu
        35              40              45

Trp Leu Ala Leu Ile Asp Trp Asp Asp Asp Lys Tyr Tyr Ser Thr Ser
        50              55              60

Leu Lys Thr Arg Leu Thr Ile Ser Lys Asp Thr Ser Lys Asn Gln Val
65              70              75              80

Val Leu Thr Met Thr Asn Met Asp Pro Val Asp Thr Ala Thr Tyr Tyr
                85              90              95

Cys Ala Arg
```

<210> 19
<211> 98

<212> PRT
<213> Homo sapiens

<400> 19

Glu Val Gln Leu Leu Glu Ser Gly Gly Gly Leu Val Gln Pro Gly Gly
1               5               10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys

<210> 20
<211> 97
<212> PRT
<213> Homo sapiens

<400> 20

Gln Val Gln Leu Gln Glu Ser Gly Pro Gly Leu Val Lys Pro Ser Glu
1               5               10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg

<210> 21
<211> 101
<212> PRT
<213> Homo sapiens

<400> 21


Gln Val Gln Leu Gln Gln Ser Gly Pro Gly Leu Val Lys Pro Ser Gln
1               5               10              15

Thr Leu Ser Leu Thr Cys Ala Ile Ser Gly Asp Ser Val Ser Ser Asn
                20              25              30

Ser Ala Ala Trp Asn Trp Ile Arg Gln Ser Pro Ser Arg Gly Leu Glu


        35              40              45

Trp Leu Gly Arg Thr Tyr Tyr Arg Ser Lys Trp Tyr Asn Asp Tyr Ala
    50              55              60

Val Ser Val Lys Ser Arg Ile Thr Ile Asn Pro Asp Thr Ser Lys Asn
65              70              75              80

Gln Phe Ser Leu Gln Leu Asn Ser Val Thr Pro Glu Asp Thr Ala Val
                85              90              95

Tyr Tyr Cys Ala Arg
            100

<210> 22
<211> 95
<212> PRT
<213> Homo sapiens

<400> 22

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20              25              30

Leu Asn Trp Tyr Gln Gln Lys Pro Gly Lys Ala Pro Lys Leu Leu Ile
        35              40              45

Tyr Ala Ala Ser Ser Leu Gln Ser Gly Val Pro Ser Arg Phe Ser Gly
    50              55              60

Ser Gly Ser Gly Thr Asp Phe Thr Leu Thr Ile Ser Ser Leu Gln Pro
65              70              75                      80

Glu Asp Phe Ala Thr Tyr Tyr Cys Gln Gln Ser Tyr Ser Thr Pro
            85              90              95
```

<210> 23
<211> 74
<212> PRT
<213> Homo sapiens

<400> 23

```
Pro Ala Thr Leu Ser Leu Ser Pro Gly Glu Arg Ala Thr Leu Ser Cys
1               5                   10                  15

Arg Ala Ser Gln Ser Val Ser Ser Ser Tyr Leu Ala Trp Tyr Gln Gln
            20              25              30

Lys Pro Gly Gln Ala Pro Arg Leu Leu Ile Tyr Gly Ala Ser Ser Arg
        35              40              45

Ala Thr Gly Ile Pro Ala Arg Phe Ser Gly Ser Gly Ser Gly Thr Asp
    50              55              60

Phe Thr Leu Thr Ile Ser Arg Leu Glu Pro
65              70
```

<210> 24
<211> 100
<212> PRT
<213> Homo sapiens

<400> 24

```
Asp Ile Val Met Thr Gln Ser Pro Leu Ser Leu Pro Val Thr Pro Gly
1               5               10              15

Glu Pro Ala Ser Ile Ser Cys Arg Ser Ser Gln Ser Leu Leu His Ser
            20              25              30

Asn Gly Tyr Asn Tyr Leu Asp Trp Tyr Leu Gln Lys Pro Gly Gln Ser
            35              40              45

Pro Gln Leu Leu Ile Tyr Leu Gly Ser Asn Arg Ala Ser Gly Val Pro
        50              55              60

Asp Arg Phe Ser Gly Ser Gly Ser Gly Thr Asp Phe Thr Leu Lys Ile
65              70              75              80

Ser Arg Val Glu Ala Glu Asp Val Gly Val Tyr Tyr Cys Met Gln Ala
            85              90              95

Leu Gln Thr Pro
            100
```

<210> 25
<211> 101
<212> PRT
<213> Homo sapiens

<400> 25

```
Asp Ile Val Met Thr Gln Ser Pro Asp Ser Leu Ala Val Ser Leu Gly
1               5               10              15

Glu Arg Ala Thr Ile Asn Cys Lys Ser Ser Gln Ser Val Leu Tyr Ser
            20              25              30

Ser Asn Asn Lys Asn Tyr Leu Ala Trp Tyr Gln Gln Lys Pro Gly Gln
        35              40              45

Pro Pro Lys Leu Leu Ile Tyr Trp Ala Ser Thr Arg Glu Ser Gly Val
        50              55              60

Pro Asp Arg Phe Ser Gly Ser Gly Ser Gly Thr Asp Phe Thr Leu Thr
65              70              75              80

Ile Ser Ser Leu Gln Ala Glu Asp Val Ala Val Tyr Tyr Cys Gln Gln
            85              90              95

Tyr Tyr Ser Thr Pro
            100
```

<210> 26
<211> 89

<212> PRT
<213> Homo sapiens

<400> 26

```
Gln Ser Val Leu Thr Gln Pro Pro Ser Ala Ser Gly Thr Pro Gly Gln
1               5               10              15

Arg Val Thr Ile Ser Cys Ser Gly Ser Ser Ser Asn Ile Gly Ser Asn
            20              25              30

Tyr Val Tyr Trp Tyr Gln Gln Leu Pro Gly Thr Ala Pro Lys Leu Leu
        35              40              45

Ile Tyr Ser Asn Asn Gln Arg Pro Ser Gly Val Pro Asp Arg Phe Ser
    50              55              60

Gly Ser Lys Ser Gly Thr Ser Ala Ser Leu Ala Ile Ser Gly Leu Arg
65              70              75              80

Ser Glu Asp Glu Ala Asp Tyr Tyr Cys
                85
```

<210> 27
<211> 90
<212> PRT
<213> Homo sapiens

<400> 27

```
Gln Ser Ala Leu Thr Gln Pro Ala Ser Val Ser Gly Ser Pro Gly Gln
1               5               10              15

Ser Ile Thr Ile Ser Cys Thr Gly Thr Ser Ser Asp Val Gly Ser Tyr
            20              25              30

Asn Leu Val Ser Trp Tyr Gln Gln His Pro Gly Lys Ala Pro Lys Leu
        35              40              45

Met Ile Tyr Glu Val Ser Lys Arg Pro Ser Gly Val Ser Asn Arg Phe
    50              55              60

Ser Gly Ser Lys Ser Gly Asn Thr Ala Ser Leu Thr Ile Ser Gly Leu
65              70              75              80

Gln Ala Glu Asp Glu Ala Asp Tyr Tyr Cys
                85              90
```

<210> 28
<211> 88
<212> PRT
<213> Homo sapiens

<400> 28

```
Ser Tyr Glu Leu Thr Gln Pro Pro Ser Val Ser Val Ser Pro Gly Gln

 1            5               10                  15


Thr Ala Ser Ile Thr Cys Ser Gly Asp Lys Leu Gly Asp Lys Tyr Ala
            20            25                  30


Cys Trp Tyr Gln Gln Lys Pro Gly Gln Ser Pro Val Leu Val Ile Tyr
            35            40                  45


Gln Asp Ser Lys Arg Pro Ser Gly Ile Pro Glu Arg Phe Ser Gly Ser
            50            55                  60


Asn Ser Gly Asn Thr Ala Thr Leu Thr Ile Ser Gly Thr Gln Ala Met
65                70                  75                  80


Asp Glu Ala Asp Tyr Tyr Cys Gln
                 85
```